(19) Europäisches Patentamt
European Patent Office
Office européen des brevets

(11) EP 4 383 988 A1

(12) EUROPEAN PATENT APPLICATION

(43) Date of publication:
**12.06.2024 Bulletin 2024/24**

(21) Application number: **22211740.0**

(22) Date of filing: **06.12.2022**

(51) International Patent Classification (IPC):
***H10K 85/60*** *(2023.01)* *H10K 50/16* *(2023.01)*

(52) Cooperative Patent Classification (CPC):
**H10K 85/654; H10K 85/615;** H10K 50/16;
H10K 85/6572

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Novaled GmbH**
**01099 Dresden (DE)**

(72) Inventors:
- **SCHOLZ, Johannes**
**01099 Dresden (DE)**
- **GANIER, Jerome**
**01099 Dresden (DE)**
- **GALÁN GARCÍA, Elena**
**01099 Dresden (DE)**
- **STENNETT, Thomas**
**01099 Dresden (DE)**

(74) Representative: **Bittner, Thomas L.**
**Boehmert & Boehmert**
**Anwaltspartnerschaft mbB**
**Pettenkoferstrasse 22**
**80336 München (DE)**

(54) **ORGANIC LIGHT EMITTING DIODE, DEVICE COMPRISING THE SAME AND COMPOUND**

(57) The present invention relates to an organic light emitting diode and a device comprising the same. The invention further relates to a compound which can be used in the organic light emitting diode.

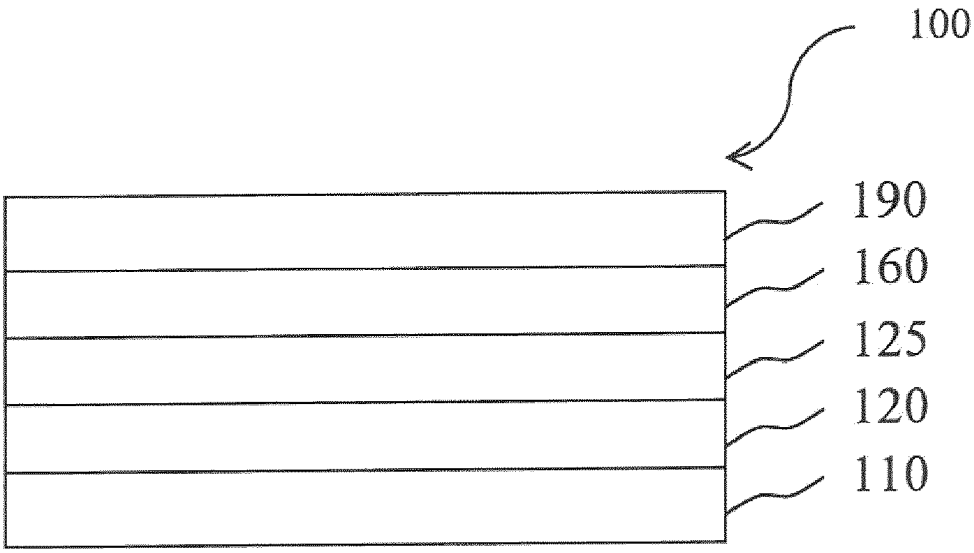


Fig.1

EP 4 383 988 A1

**Description**

TECHNICAL FIELD

The present invention relates to an organic light emitting diode and a device comprising the same. The invention further relates to a compound which can be used in the organic light emitting diode.

BACKROUND OF THE INVENTION

Organic semiconducting devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of an organic material of the organic semiconductor layer.

Particularly, development of an organic semiconductor layer being capable of increasing electron mobility and simultaneously increasing electrochemical stability is needed so that the organic semiconducting device, such as an organic light emitting diode, may be applied to a large-size flat panel display.

It is, therefore, the object of the present invention to provide organic light emitting diodes and compounds for preparing the same overcoming drawbacks of the prior art, in particular providing compounds for use in organic light emitting diodes comprising the same helpful to improve the performance thereof, especially with respect to efficiency.

DISCLOSURE

The object is achieved by an organic light emitting diode, comprising an anode, a cathode, an emission layer, and an electron transport layer,
wherein

- the electron transport layer is arranged between the emission layer and the cathode;
- the electron transport layer is free of an electrical dopant;
- the electron transport layer comprises a compound of formula (I)

$$(Ar^2)_m\text{-}(Z_k\text{-}G)_n \qquad (I);$$

wherein in formula (I)

- m and n are independently 1 or 2;
- k is independently 0, 1 or 2;
- $Ar^2$ is independently selected from the group consisting of $C_2$ to $C_{42}$ heteroaryl and $C_6$ to $C_{60}$, aryl,
  - wherein each Ar2 may be substituted with one or two substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- wherein each $C_6$ to $C_{12}$ aryl substituent on $Ar^2$ and each $C_3$ to $C_{11}$ heteroaryl substituent on $Ar^2$ may be substituted with $C_1$ to $C_4$ alkyl or halogen;

- Z is independently selected from $C_6$ to $C_{30}$ aryl,

  - wherein each Z may be substituted with one or two substituents independently selected from the group consisting of $C_6$ to $C_{12}$ aryl and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from 0, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

  - wherein each $C_6$ to $C_{12}$ aryl substituent on Z may be substituted with $C_1$ to $C_4$ alkyl or halogen;

- G is chosen so that the dipole moment of a compound G-phenyl is ≥ 1 D and ≤ 7 D;

- the electron transport layer further comprises a compound of formula (II) and/or a compound of formula (III)

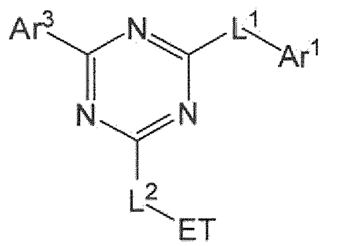
(II);
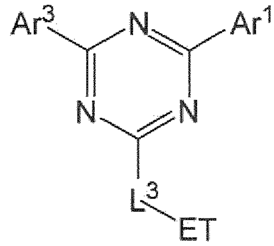
(III)

wherein in formula (II) and in formula (III) respectively

- $Ar^1$ is selected independently from the group consisting of $C_6$ to $C_{19}$ aryl and $C_2$ to $C_{19}$ heteroaryl;

  - wherein $Ar^1$ may be substituted with one or two substituents independently selected from the group consisting of $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- $Ar^3$ is selected independently from the group consisting of $C_6$ to $C_{19}$ aryl;

  - wherein $Ar^3$ may be substituted with one or two substituents independently selected from the group consisting of $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- ET is selected independently from the group consisting of $C_6$ to $C_{60}$ aryl and $C_2$ to $C_{60}$ heteroaryl;

  - wherein ET may be substituted with one or two substituents independently selected from the group consisting of $C_6$ to $C_{20}$ aryl, $C_3$ to $C_{20}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$

alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- wherein each $C_6$ to $C_{20}$ aryl substituent on ET and each $C_3$ to $C_{20}$ heteroaryl substituent on ET may be substituted with $C_1$ to $C_4$ alkyl or halogen;

- $L^1$ has the formula (IIa)

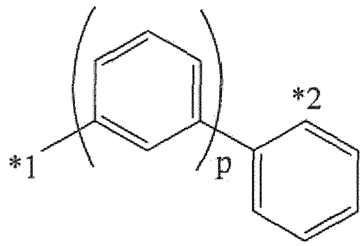

(IIa)

wherein $L^1$ is bonded at *1 to the triazine moiety in formula (II); and $L^1$ is bonded at *2 to $Ar^1$; and p is o or 1;

- $L^2$ has the formula (IIb)

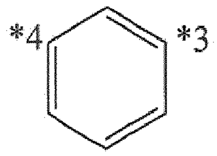

(IIb)

wherein $L^2$ is bonded at *3 to the triazine moiety in formula (II); and $L^2$ is bonded at *4 to ET; and

- $L^3$ has the formula (IIIa.1) or (IIIa.2)

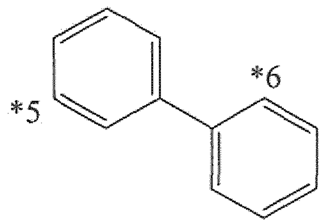

(IIIa.1)

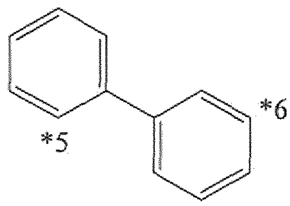

(IIIa.2)

wherein $L^3$ is bonded at *5 to the triazine moiety in formula (III); and $L^3$ is bonded at *6 to ET.

The object is further achieved by a device comprising the organic light emitting diode according to the present invention, wherein the device is a display device or a lighting device.

The object is further achieved by a compound of the formula (IV) or of the formula (V)

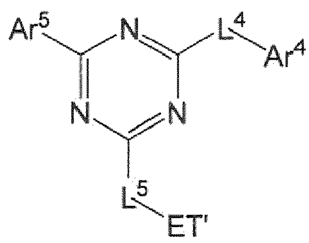
(IV)

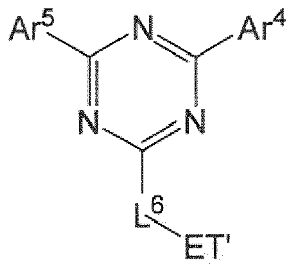
(V)

wherein in formula (IV) and in formula (V) respectively

- $Ar^4$ is selected from the group consisting of $C_6$ to $C_{19}$ aryl and $C_2$ to $C_{19}$ heteroaryl;

  - wherein $Ar^4$ may be substituted with one or two substituents independently selected from the group consisting of $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- $Ar^5$ is selected from the group consisting of $C_6$ to $C_{19}$ aryl;

  - wherein $Ar^5$ may be substituted with one or two substituents independently selected from the group consisting of $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})2$, wherein Y is selected from O, S or Se, preferably 0, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$, to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- ET' is selected from the group consisting of pyrazinyl; triphenylpyrazinyl; pyridine-anthracenyl; acridine, benzoacridine, dibenzoacridine, phenylanthracenyl and a compound of the formula ET'-i

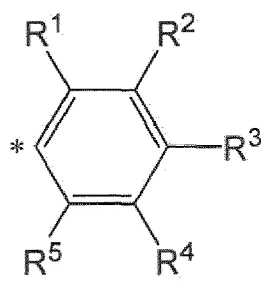
ET'-i

wherein $R^1$ to $R^5$ are independently H or phenyl, provided that at least two, preferably at least three, most preferred four of $R^1$ to $R^5$ are phenyl with the remaining $R^1$ to $R^5$ being H; and ET'-i is bonded at * to $L^5$, respectively to $R^6$;

- $L^4$ has the formula (IVa)

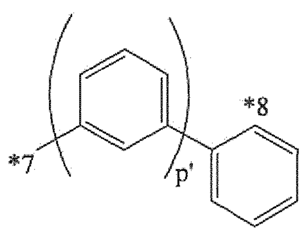
(IVa)

wherein $L^4$ is bonded at *7 to the triazine moiety in formula (IV); and $L^4$ is bonded at *8 to $Ar^4$; and p' is o or 1;

- $L^5$ has the formula (IVb)

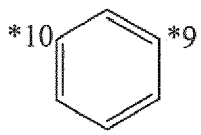

(IVb)

wherein $L^5$ is bonded at *9 to the triazine moiety in formula (IV); and $L^5$ is bonded at *10 to ET'; and

- $L^6$ has the formula (Va.1) or (Va.2)

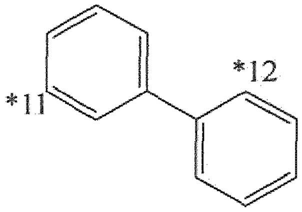

(Va.1)

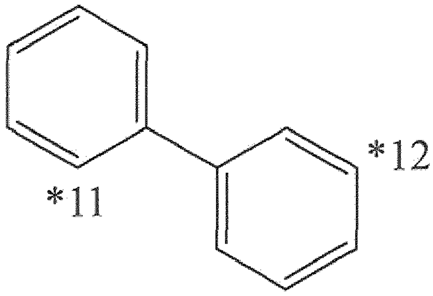

(Va.2)

wherein $L^6$ is bonded at *11 to the triazine moiety in formula (V); and $L^6$ is bonded at *12 to ET'.

Compound of formula (I)

The electron transport layer comprises a compound of formula (I)

$$(Ar^2)_m\text{-}(Z_k\text{-}G)_n \qquad (I).$$

The electron transport layer may consist of a mixture of the compound of formula (I), the compound of formula (II) and/or (III) and one or more further compounds, provided that none of the further compounds is an electrical dopant. The electron transport layer may comprise more than one compound of formula (I). The electron transport layer may consist of a mixture of the compound of formula (I) and the compound of formula (II). Exemplary further electron transport matrix compounds which may be contained are disclosed below.

In the compound of formula (I), the group "Z" is a spacer moiety connecting (if present, that is in case that k > 1) the groups $Ar^2$ and G. In case that the compound of formula (I) comprises more than one groups ($Z_k$-G) the groups may or may not independently comprise the spacer Z.

In formula (I), m and n are independently 1 or 2. In formula (I), m and n may be 1.

In formula (I), k is independently 0, 1 or 2. In formula (I), k may be independently 1 or 2.

$Ar^2$ may be independently selected from the group consisting of $C_2$ to $C_{39}$ heteroaryl and $C_6$ to $C_{54}$ aryl, optionally $C_2$ to $C_{36}$ heteroaryl and $C_6$ to $C_{48}$ aryl, optionally $C_3$ to $C_{30}$ heteroaryl and $C_6$ to $C_{42}$ aryl, optionally $C_3$ to $C_{27}$ heteroaryl and $C_6$ to $C_{36}$ aryl, optionally $C_3$ to $C_{24}$ heteroaryl and $C_6$ to $C_{30}$ aryl, and optionally $C_3$ to $C_{21}$ heteroaryl and $C_6$ to $C_{24}$ aryl.

$Ar^2$ may be independently selected from the group consisting of $C_2$ to $C_{39}$ N-containing heteroaryl and $C_6$ to $C_{54}$ aryl, optionally $C_2$ to $C_{36}$ N-containing heteroaryl and $C_6$ to $C_{48}$ aryl, optionally $C_3$ to $C_{30}$ N-containing heteroaryl and $C_6$ to $C_{42}$ aryl, optionally $C_3$ to $C_{27}$ N-containing heteroaryl and $C_6$ to $C_{36}$ aryl, optionally $C_3$ to $C_{24}$ N-containing heteroaryl and $C_6$ to $C_{30}$ aryl, and optionally $C_3$ to $C_{21}$ N-containing heteroaryl and $C_6$ to $C_{24}$ aryl. In this regard, it may be provided that a respective N-containing heteroaryl comprises one or more N-atoms as the only heteroatom(s).

$Ar^2$ may comprise at least two annelated 5- or 6-membered rings.

$Ar^2$ may be independently selected from the group consisting of pyridinyl, triazinyl, 1,2-diazinyl, 1,3-diazinyl, 1,4-diazinyl, quinazolinyl, benzoquinazolinyl, benzimidazolyl, quinolinyl, benzoquinolinyl benzoacridinyl, dibenzoacridinyl, fluoranthenyl, anthracenyl, naphthyl, triphenylenyl, phenathrolinyl, and dinaphthofuranyl which may be substituted or unsubstituted, respectively.

$Ar^2$ may be independently selected from the group consisting of dibenzoacridinyl, 1,3-diazinyl, 1,4-diazinyl, anthracenyl, triazinyl, phenathrolinyl, triphenylenyl, pyridinyl, dinaphthofuranyl.

$Ar^2$ may be selected independently from one of the following groups

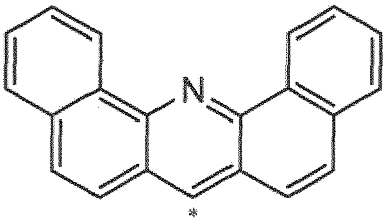 ; 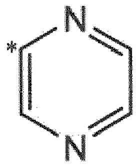 ;

; 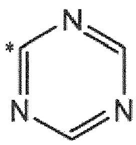 ; 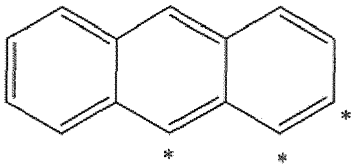 ; 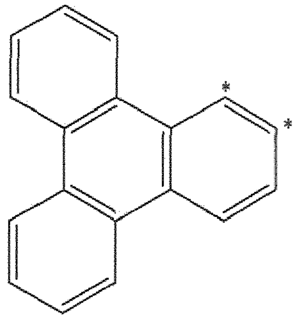 ;

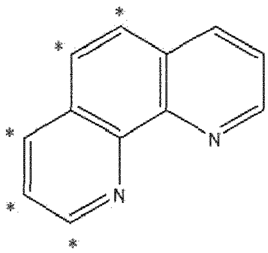 ; 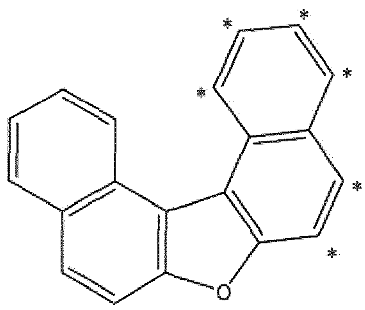 ; 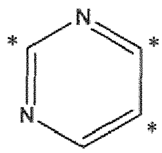

wherein the asterisk symbol "*" represents the binding position for binding the to Z, respectively.

In case that $Ar^2$ is substituted, each substituent on $Ar^2$ may be independently selected from the group consisting of phenyl, naphthyl, optionally β-naphthyl, pyridinyl and biphenyl-yl which may be substituted or unsubstituted, respectively.

In case that $Ar^2$ is substituted, each substituent on $Ar^2$ may be independently selected from the group consisting of phenyl, pyridinyl and biphenyl-yl, optionally para-biphenyl-yl.

Z may be independently selected from $C_6$ to $C_{24}$ aryl, alternatively $C_6$ to $C_{18}$ aryl, alternatively $C_6$ to $C_{12}$ aryl, which may be substituted or unsubstituted.

Z may be selected from the group consisting of phenylene, naphthylene, phenylene-naphthylene, biphenylene and terphenylene which may be substituted or unsubstituted, respectively.

Z may be selected independently from one of the following groups

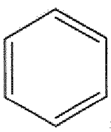 ; 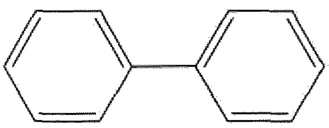 ; 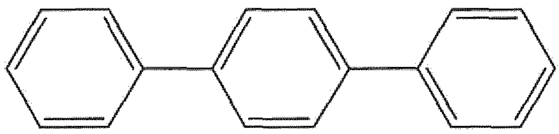 ;

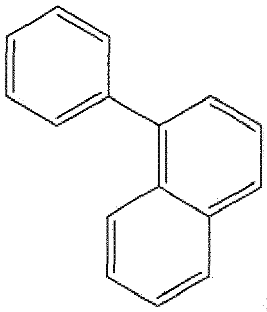 ; 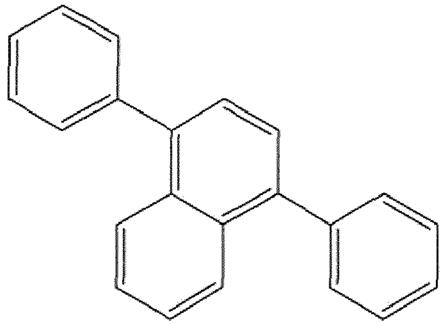

wherein the binding positions for binding to $Ar^2$ and G can be freely selected.

In case that Z is substituted, each substituent on Z may be independently selected from the group consisting of phenyl and $C_1$ to $C_4$ alkyl.

G is chosen so that the dipole moment, computed by the TURBOMOLE V6.5 program package using hybrid functional B3LYP and Gaussian 6-31G* basis set, of a compound G-phenyl is ≥ 1 D and ≤ 7 D. The unit for the dipole moment "Debye" is abbreviated with the symbol "D". The inventors have found that it is advantageous if the compound of formula (I) comprises a group having a certain polarity, that is a specific dipole moment within the above range or the ranges mentioned below. It was further found that it is still advantageous that the compound of formula (I) comprises such a polar group (first polar group) if the compound of formula (I) comprises, in addition, a further polar group (second polar group) which is suitable to balance the dipole moment of the first polar group in a way that the total dipole moment of the compound of formula (I) is low, for example, in case that the compound is a symmetrical molecule comprising a first polar group and a second polar group which are the same, the dipole moment could be 0 Debye. Therefore, the compound of formula (I) cannot be characterized be referring to the total dipole moment of the compound. As a consequence, reference is made instead to an artificial compound comprising the polar group "G" and an unpolar group "phenyl". In this regards, the dipole moment $|\vec{\mu}|$ of a compound containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule. The dipole moment is determined by a semi-empirical molecular orbital method. The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules. In this regard, the entire moiety G encompasses all possible substituents, which may be comprised.

G may be selected so that the dipole moment of a compound G-phenyl is > 1 D; optionally ≥ 2 D; optionally ≥ 2.5 D, optionally ≥ 3 D, and optionally ≥ 3.5 D. G may be chosen so that the dipole moment of a compound G-phenyl is ≤ 7 D, optionally ≤ 6.5 D, optionally ≤ 6 D, optionally ≤ 5.5 D, optionally ≤ 5 D. If more than one conformational isomer of the compound G-phenyl is viable then the average value of the dipole moments of the conformational isomers of G-phenyl is selected to be in this range. Conformational isomerism is a form of stereoisomerism in which the isomers can be interconverted just by rotations about formally single bonds.

By selecting the G such that the dipole moment of a compound G-phenyl lies in the above range it is provided that the electron injection from the adjacent, distinct electron injection layer (EIL) is improved and voltage of the OLED device is decreased and the cd/A efficiency of the OLED device is increased.

Exemplary compounds "G-phenyl" are listed in the following Table 1, wherein the moiety

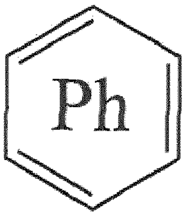

in the respective compound specifies the "phenyl" part in "G-phenyl"

Table 1:

| | Structure of G-phenyl | HOMO [eV] | LUMO [eV] | Dipole *momen* t [D] |
|---|---|---|---|---|
| G-phenyl-1 | 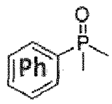 | -6.88 | -0.62 | **4.16** |
| G-phenyl-2 | 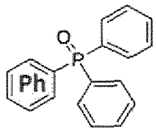 | -6.74 | -0.86 | **4.19** |
| G-phenyl-3 | 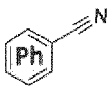 | -8.97 | 1.00 | **4.56** |
| G-phenyl-4 | 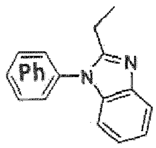 | -5.82 | -0.62 | **3.97** |
| G-phenyl-5 | 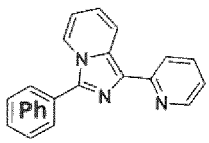 | -5.04 | -1.18 | **3.86** |
| G-phenyl-6 | 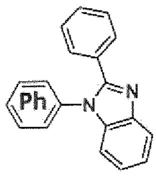 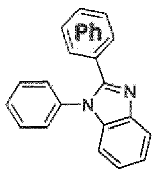 | -5.70 | -1.02 | **3.70** |
| G-phenyl-7 | 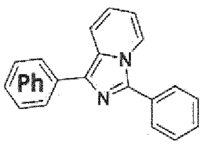 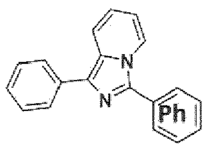 | -4.92 | -1.11 | **3.11** |
| G-phenyl-8 | 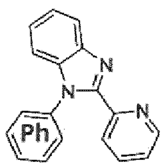 | -5.86 | -1.19 | **5.14** |

(continued)

| | Structure of G-phenyl | HOMO [eV] | LUMO [eV] | Dipole *momen* t [D] |
|---|---|---|---|---|
| G-phenyl-9 | 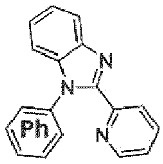 | -5.76 | -1.33 | **2.61** |
| G-phenyl-10 | 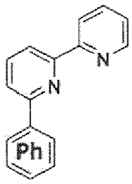 | -5.96 | -1.35 | **2.69** |
| G-phenyl-11 | 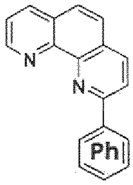 | -5.83 | -1.59 | **2.67** |
| G-phenyl-12 | 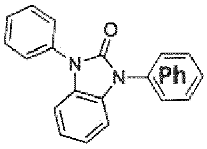 | -5.54 | -0.48 | **2.12** |
| G-phenyl-13 | 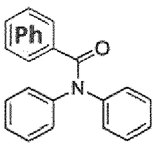 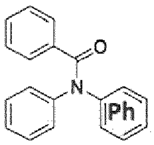 | -5.79 | -1.06 | **3.33** |
| G-phenyl-14 | 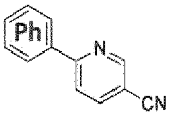 | -6.59 | -2.08 | **4.79** |
| G-phenyl-15 | 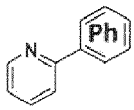 | -6.12 | -1.13 | **1.71** |
| G-phenyl-16 | 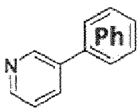 | -6.32 | -0.98 | **2.31** |
| G-phenyl-17 | 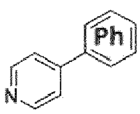 | -6.57 | -1.19 | **2.75** |
| G-phenyl-18 | 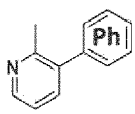 | -6.28 | -0.77 | **2.00** |

(continued)

| | Structure of G-phenyl | HOMO [eV] | LUMO [eV] | Dipole *momen* t [D] |
|---|---|---|---|---|
| G-phenyl-19 | 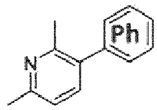 | -6.12 | -0.69 | **1.50** |
| G-phenyl-20 | 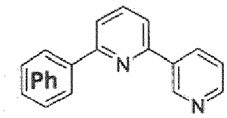 | -6.10 | -1.41 | **3.51** |
| G-phenyl-21 | 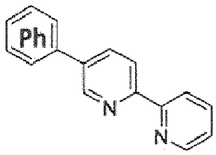 | -6.10 | -1.38 | **2.98** |
| G-phenyl-22 | 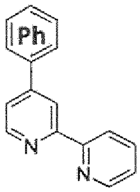 | -6.47 | -1.31 | **3.46** |
| G-phenyl-23 | 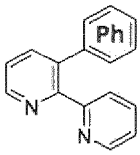 | -6.19 | -1.03 | **3.02** |
| G-phenyl-24 | 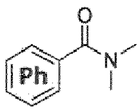 | -6.35 | -0.17 | **3.62** |
| G-phenyl-25 | 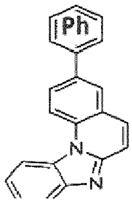 | -5.54 | -1.58 | **3.49** |
| G-phenyl-26 | 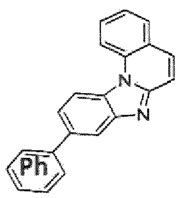 | -5.60 | -1.61 | **3.39** |
| G-phenyl-27 | 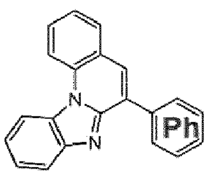 | -5.48 | -1.67 | **2.76** |

(continued)

| | Structure of G-phenyl | HOMO [eV] | LUMO [eV] | Dipole *momen* t [D] |
|---|---|---|---|---|
| G-phenyl-28 | 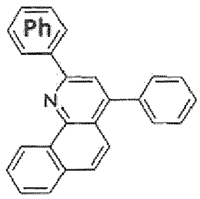 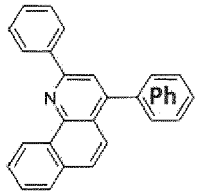 | -5.63 | -1.56 | **1.84** |
| G-phenyl-29 | 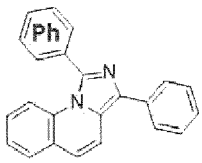 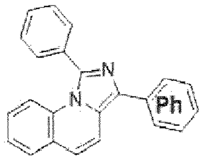 | -5.02 | -1.39 | **2.96** |
| G-phenyl-30 | 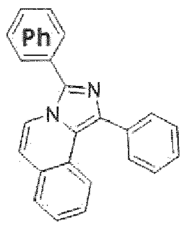 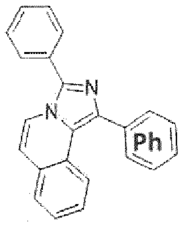 | -5.08 | -1.13 | **2.70** |
| G-phenyl-31 | 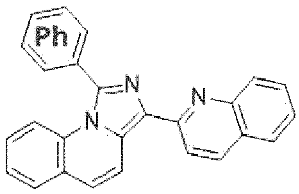 | -5.07 | -1.58 | **2.29** |
| G-phenyl-32 | 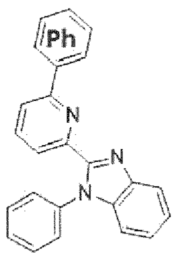 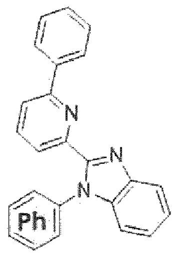 | -5.81 | -1.19 | **4.61** |
| G-phenyl-33 | 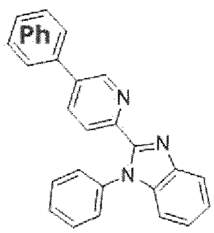 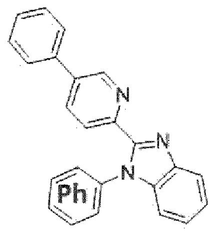 | -5.78 | -1.42 | **5.20** |
| G-phenyl-34 | 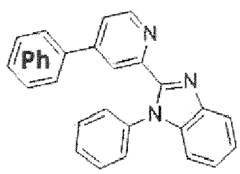 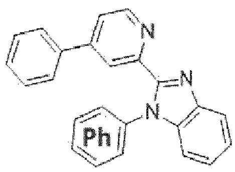 | -5.84 | -1.38 | **5.63** |

(continued)

| | Structure of G-phenyl | HOMO [eV] | LUMO [eV] | Dipole *momen* t [D] |
|---|---|---|---|---|
| G-phenyl-35 | 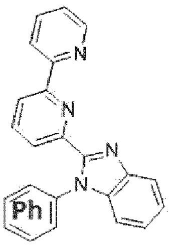 | -5.83 | -1.35 | **3.37** |
| G-phenyl-36 | 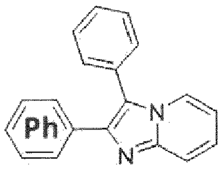 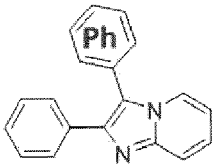 | -5.37 | -0.98 | **3.32** |
| G-phenyl-37 | 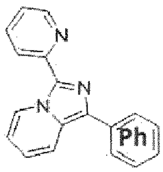 | -4.94 | -1.15 | **1.81** |
| G-phenyl-38 | 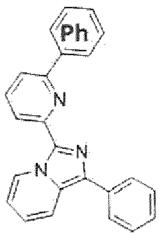 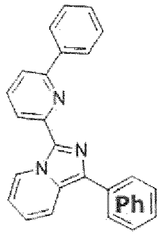 | -4.94 | -1.16 | **2.12** |
| G-phenyl-39 | 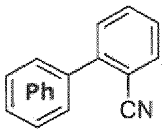 | -6.52 | -1.47 | **4.17** |
| G-phenyl-40 | 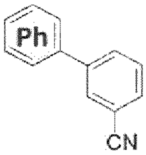 | -6.56 | -1.46 | **4.85** |
| G-phenyl-41 | 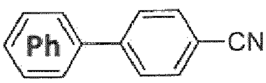 | -6.53 | -1.67 | **5.27** |
| G-phenyl-42 | 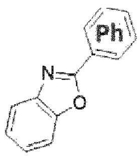 | -6.00 | -1.43 | **1.14** |

(continued)

| | Structure of G-phenyl | HOMO [eV] | LUMO [eV] | Dipole *momen* t [D] |
|---|---|---|---|---|
| G-phenyl-43 | 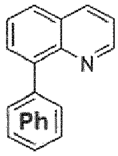 | -5.84 | -1.47 | **1.94** |
| G-phenyl-44 | 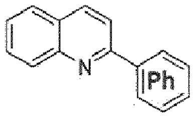 | -5.97 | -1.56 | **1.53** |
| G-phenyl-45 | 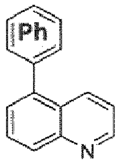 | -6.01 | -1.42 | **2.31** |
| G-phenyl-46 | 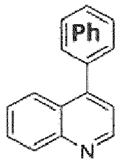 | -6.09 | -1.47 | **2.57** |
| G-phenyl-47 | 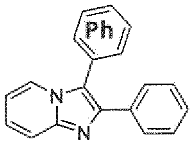 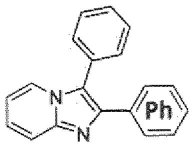 | -5.37 | -0.98 | **3.32** |
| G-phenyl-48 | 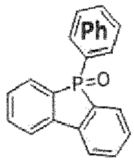 | -6.22 | -1.47 | **4.49** |
| G-phenyl-49 | 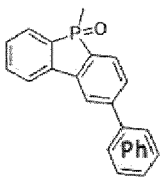 | -6.15 | -1.55 | **4.79** |
| G-phenyl-50 | 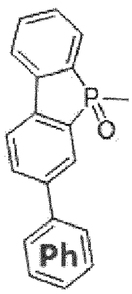 | -5.95 | -1.58 | **4.45** |

(continued)

| | Structure of G-phenyl | HOMO [eV] | LUMO [eV] | Dipole *momen* t [D] |
|---|---|---|---|---|
| G-phenyl-51 | 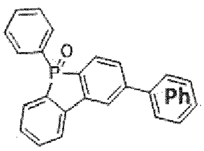 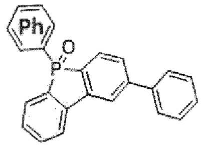 | -6.10 | -1.57 | **4.65** |
| G-phenyl-52 | 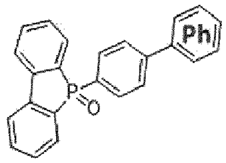 | -6.14 | -1.47 | **4.59** |
| G-phenyl-53 | 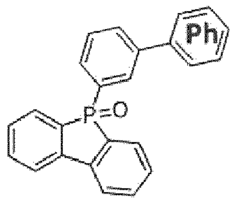 | -6.12 | -1.48 | **4.26** |
| G-phenyl-54 | 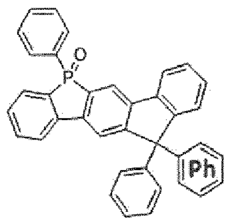 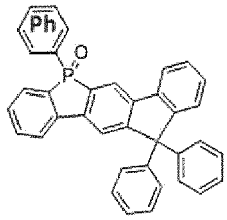 | -5.71 | -1.60 | **4.55** |
| G-phenyl-55 | 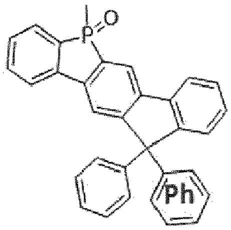 | -5.74 | -1.60 | **4.73** |
| G-phenyl-56 | 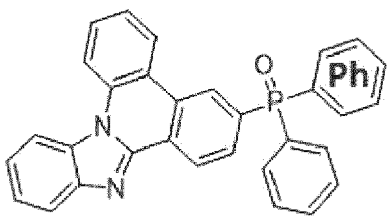 | -5.75 | -1.62 | **-6.70** |
| G-phenyl-57 | 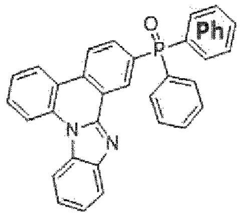 | -5.68 | -1.61 | **6.58** |

(continued)

| | Structure of G-phenyl | HOMO [eV] | LUMO [eV] | Dipole *momen* t [D] |
|---|---|---|---|---|
| G-phenyl-58 | 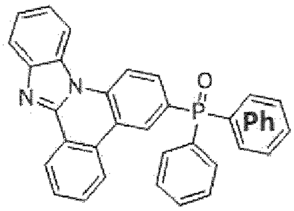 | -5.71 | -1.45 | **4.34** |
| G-phenyl-59 | 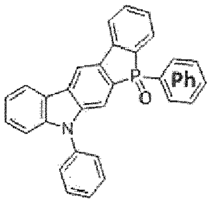 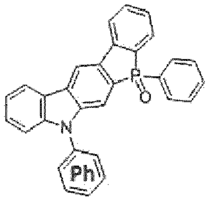 | -5.42 | -1.34 | **4.33** |
| G-phenyl-60 | 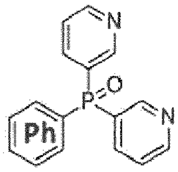 | -6.89 | -1.25 | **3.44** |
| G-phenyl-61 | 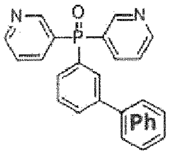 | -6.32 | -1.28 | **3.20** |
| G-phenyl-62 | 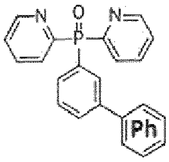 | -5.96 | -1.24 | **4.42** |
| G-phenyl-63 | 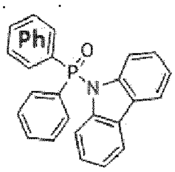 | -5.63 | -0.96 | **4.10** |
| G-phenyl-64 | 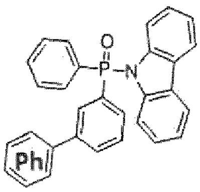 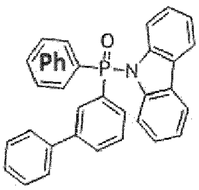 | -5.64 | -1.05 | **4.23** |
| G-phenyl-65 | 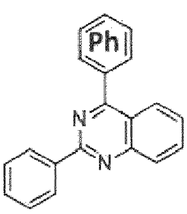 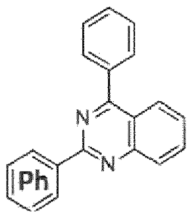 | -6.00 | -1.88 | **2.46** |

(continued)

| | Structure of G-phenyl | HOMO [eV] | LUMO [eV] | Dipole *momen* t [D] |
|---|---|---|---|---|
| G-phenyl-66 | 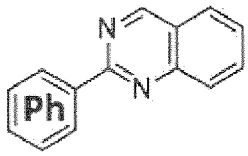 | -6.12 | -1.82 | **2.22** |
| G-phenyl-67 | 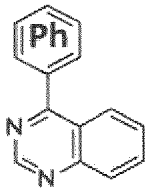 | -6.36 | -1.87 | **3.04** |
| G-phenyl-68 | 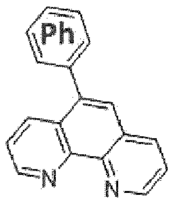 | -6.03 | -1.46 | **3.58** |
| G-phenyl-69 | 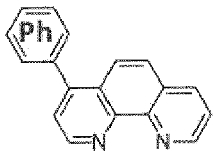 | -6.09 | -1.46 | **3.67** |
| G-phenyl-70 | 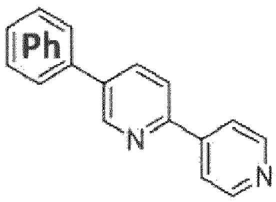 | -6.17 | -1.85 | **4.56** |
| G-phenyl-71 | 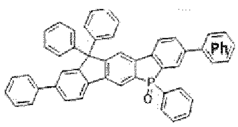 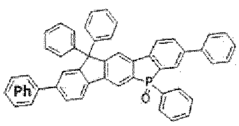 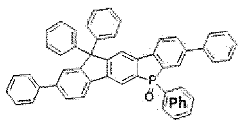 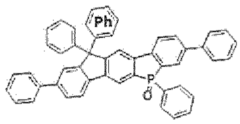 | -5.50 | -1.74 | **4.45** |
| G-phenyl-72 | 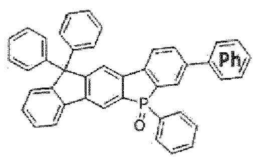 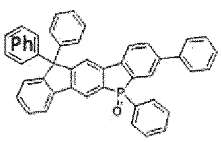 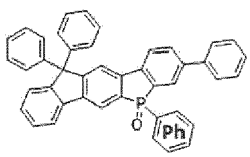 | -5.55 | -1.76 | **4.44** |

(continued)

| | Structure of G-phenyl | HOMO [eV] | LUMO [eV] | Dipole *momen* t [D] |
|---|---|---|---|---|
| G-phenyl-73 | 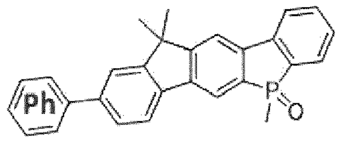 | -5.58 | -1.64 | **4.98** |
| G-phenyl-74 | 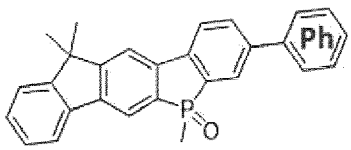 | -5.60 | -1.66 | **4.81** |
| G-phenyl-75 | 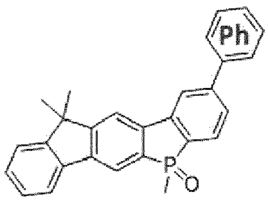 | -5.70 | -1.60 | **5.11** |
| G-phenyl-76 | 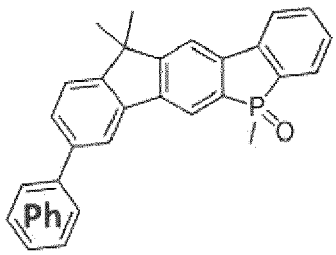 | -5.66 | -1.58 | **4.90** |
| G-phenyl-77 | 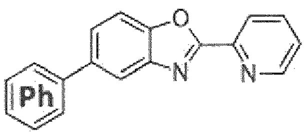 | -5.95 | -1.70 | **1.25** |
| G-phenyl-78 | 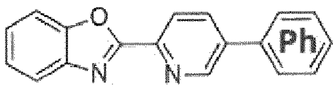 | -5.92 | -1.91 | **1.83** |
| G-phenyl-79 | 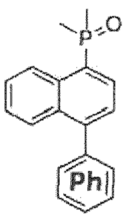 | -5.84 | -1.41 | **4.28** |
| G-phenyl-80 | 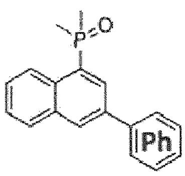 | -5.81 | -1.40 | **3.98** |

(continued)

| | Structure of G-phenyl | HOMO [eV] | LUMO [eV] | Dipole *momen* t [D] |
|---|---|---|---|---|
| G-phenyl-81 | 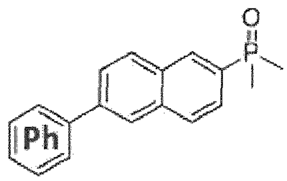 | -5.84 | -1.61 | **4.50** |
| G-phenyl-82 | 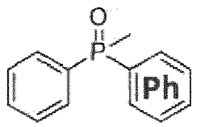 | -6.85 | -0.85 | **4.00** |
| G-phenyl-83 | 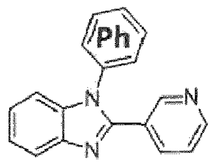 | -5.88 | -1.27 | **2.59** |
| G-phenyl-84 | 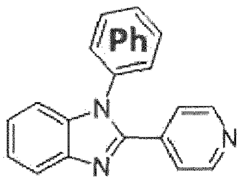 | -6.02 | -1.48 | **4.35** |

G may be selected from the group consisting of dialkylphosphinyl, diarylphosphinyl, alkylarylphosphinyl, diheteroarylphosphinyl, arylheteroarylphosphinyl, cyclic diarylphosphinyl, phosphine oxide, aryl-containing phosphine oxide, heteroaryl-containing phosphine oxide, cyclic arylheteroarylphosphinyl, cyclic heteroaryl-containing phosphine oxide, nitrile, benzonitrile, nicotinonitrile, amide, carbamide, and $C_2$ to $C_{42}$ heteroaryl; wherein G may include one or more substituents attached to the group, wherein the one or more substituents are selected from the group consisting of $C_6$ to $C_{18}$ aryl, $C_1$ to $C_{10}$ alkyl, $C_2$ to $C_{14}$ heteroaryl. In this regard, "cyclic" means that the phosphorus atom of the phosphinyl, respectively of the phosphine oxide, respectively of the "P=O" group, is part of a ring formed with further moieties of the group G.

G may be selected from the group consisting of di-$C_1$ to $d_{10}$-alkylphosphinyl, di-$C_6$ to $C_{10}$-arylphosphinyl, $C_{10}$-$C_4$, diheteroarylphosphinyl, $C_7$-$C_{42}$ arylheteroarylphosphinyl, $C_8$-$C_{42}$ phosphine oxide, $C_8$-$C_{42}$ aryl-containing phosphine oxide, $C_8$-$C_{63}$ heteroaryl-containing phosphine oxide, $C_{12}$-$C_{63}$ cyclic arylphosphinyl, $C_7$-$C_{42}$ cyclic arylheteroarylphosphinyl, $C_7$-$C_{42}$ cyclic heteroaryl-containing phosphine oxide, and $C_2$ to $C_{39}$ heteroaryl, optionally $C_2$ to $C_{35}$ heteroaryl, optionally $C_2$ to $C_{32}$ heteroaryl, optionally $C_2$ to $C_{29}$ heteroaryl, optionally $C_2$ to $C_{25}$ heteroaryl; G may include one or more substituents attached to the group, wherein the one or more substituents are selected from the group consisting of $C_6$ to $C_{12}$ aryl, $C_1$ to $C_6$ alkyl, $C_2$ to $C_{11}$, heteroaryl.

G may be selected from the group consisting of di-$C_1$ to $C_4$-alkylphosphinyl, di-$C_6$ to $C_{10}$-arylphosphinyl, $C_{10}$ diheteroarylphosphinyl, $C_7$-$C_{25}$ arylheteroarylphosphinyl, $C_8$-$C_{42}$ phosphine oxide, $C_8$-$C_{42}$ aryl-containing phosphine oxide, $C_8$-$C_{24}$ heteroaryl-containing phosphine oxide, $C_{12}$-$C_{42}$ cyclic arylphosphinyl, $C_7$-$C_{25}$ cyclic arylheteroarylphosphinyl, $C_7$-$C_{25}$ cyclic heteroaryl-containing phosphine oxide, and $C_2$ to $C_{25}$ heteroaryl; wherein the respective G may include one or more substituents attached to the group, wherein the one or more substituents are selected from the group consisting of $C_6$ to $C_{10}$ aryl, $C_1$ to $C_4$ alkyl, $C_2$ to $C_5$ heteroaryl.

G is selected from the group consisting of dialkylphosphinyl, diarylphosphinyl, alkylarylphosphinyl, diheteroarylphosphinyl, arylheteroarylphosphinyl, cyclic diarylphosphinyl, phosphine oxide, aryl-containing phosphine oxide, heteroaryl-containing phosphine oxide, cyclic arylheteroarylphosphinyl, cyclic heteroaryl-containing phosphine oxide, nitrile, benzonitrile, nicotinonitrile, amide-yl, carbamide-yl and $C_2$ to $C_{17}$ heteroaryl; wherein the respective G may include one or more substituents attached to the group, wherein the one or more substituents are selected from the group consisting of phenyl, methyl, ethyl, and pyridyl.

G may be selected independently from the group consisting of dimethylphosphinyl, diphenylphosphinyl, nitrile, benzonitrile, nicotinonitrile, di-hydro-benzoimidazolone-yl, diphenyl-propane-yl, *N*,*N*-dimethylacetamid, amide, carbamide, imidazolyl, phenylbenzoimidazolyl, ethylbenzoimidazolyl phenylbenzoquinolinyl, phenylbenzoimidazoquinolinyl, pyridinyl, bipyridinyl, picolinyl, lutidenyl, pyridazinyl, pyrimidinyl, pyrazinyl, triphenyl-pyrazinyl, benzoquinolinyl, phenanthrolinyl, phenylphenanthrolinyl, quinazolinyl, benzoxazolyl, benzimidazolyl, pyridinyl-imidazopyridinyl;

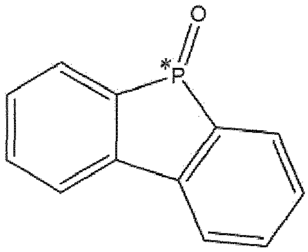 ; 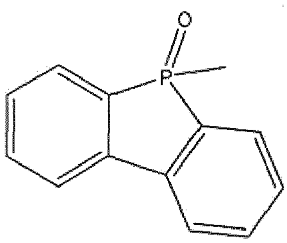 ; 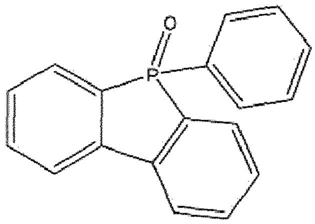 ;

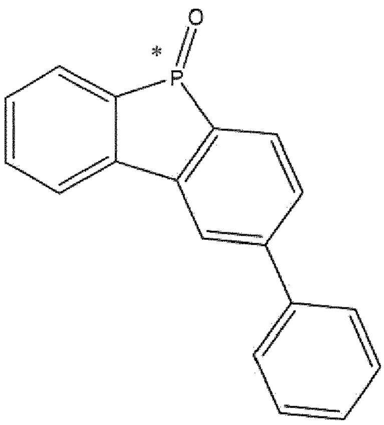 ; 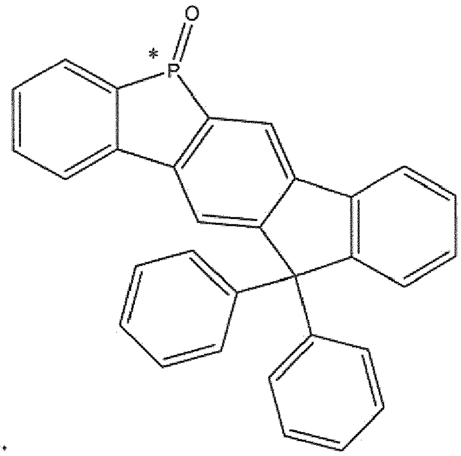 ;

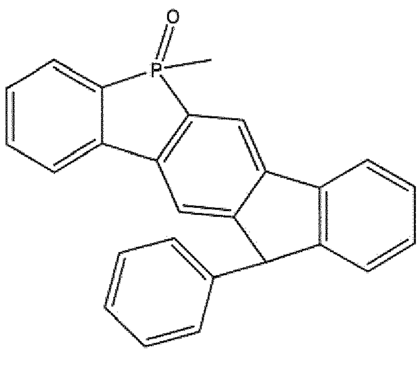 ; 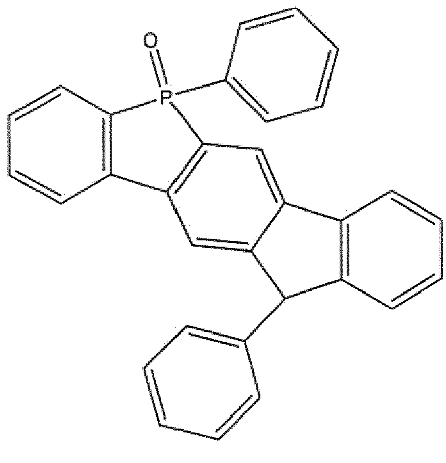 ; 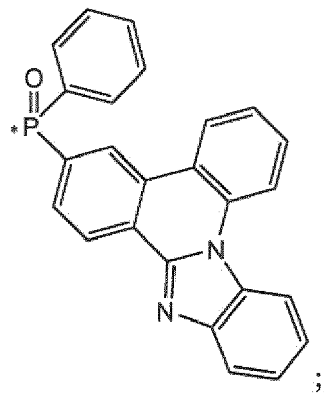 ;

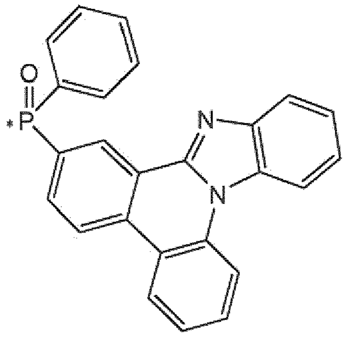 ; 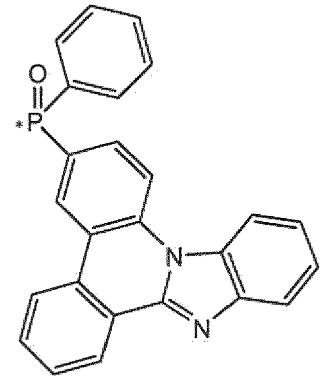 ; 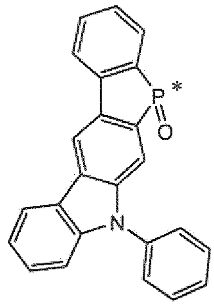 ;

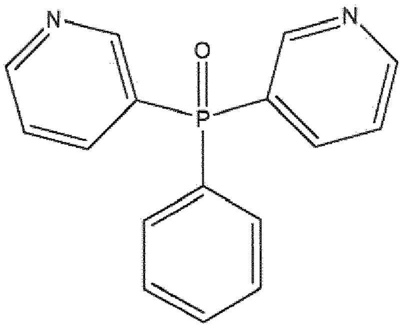 ; 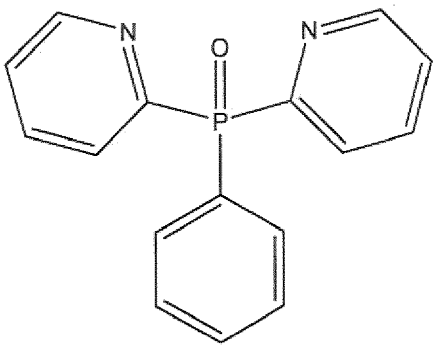 ; 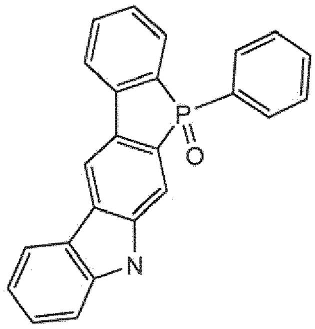 ;

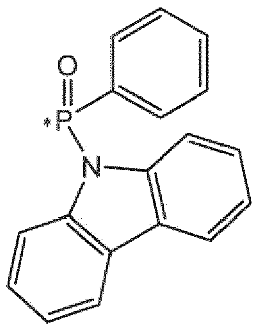 ; 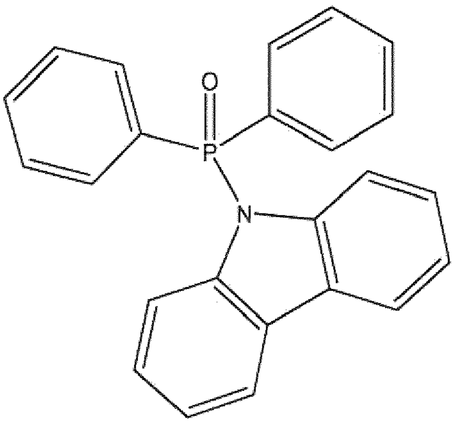 ; 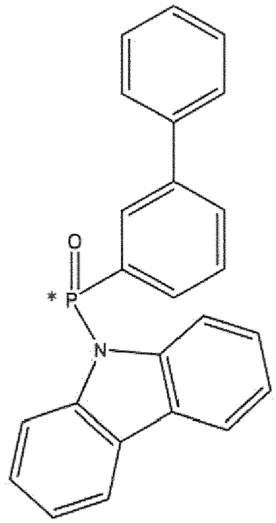 ;

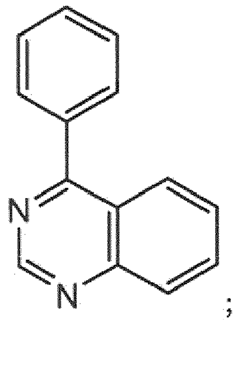 ; 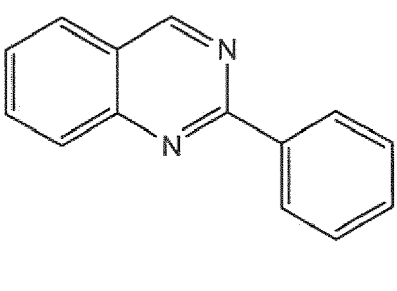 ;

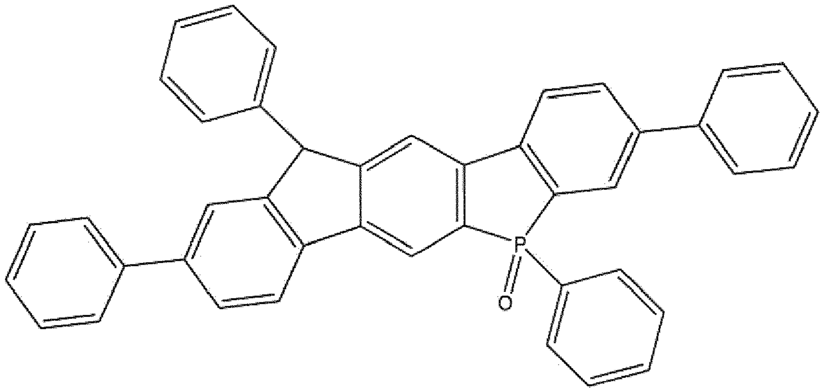

;

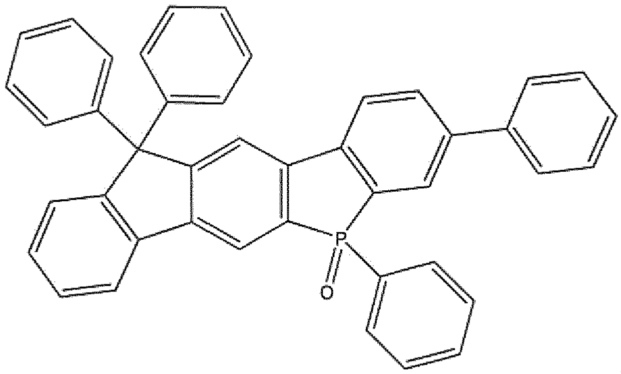

;

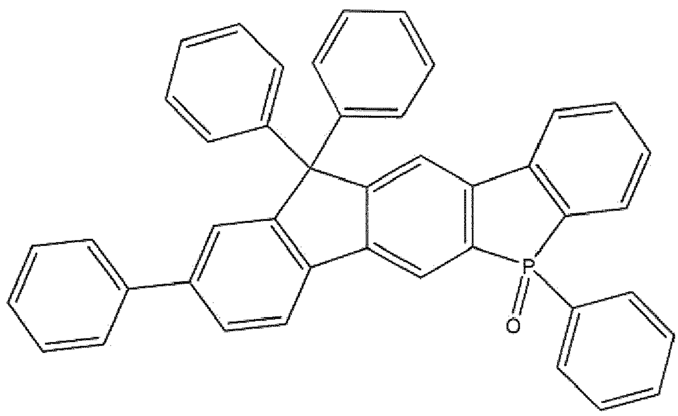

;

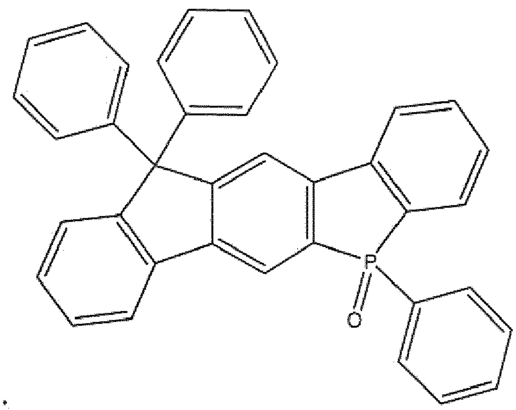

;

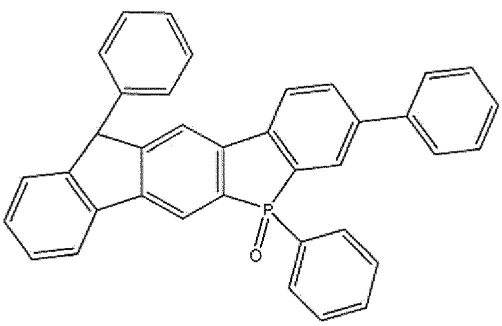

;

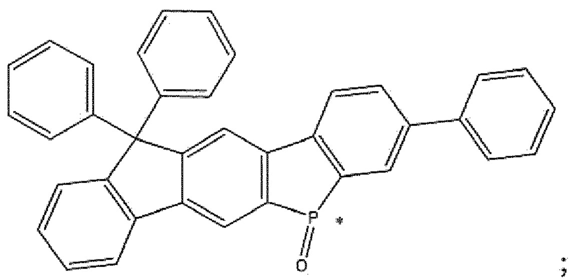

;

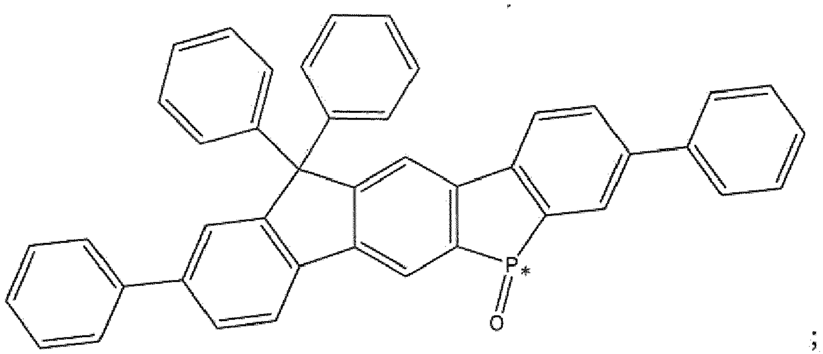

;

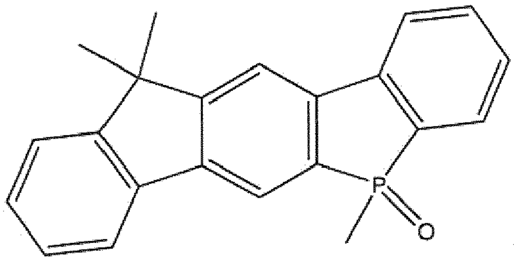

;

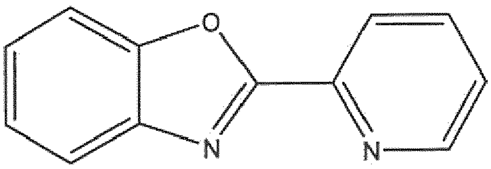 ; 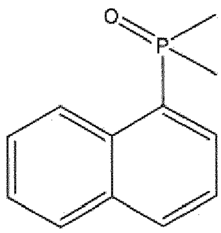 ; 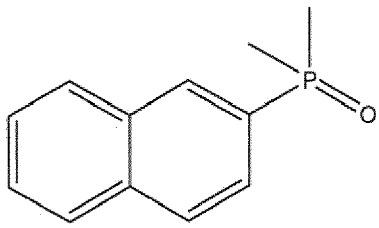 ;

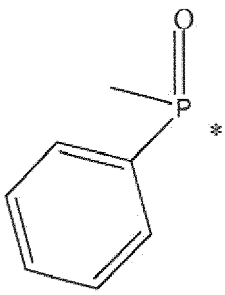 ; 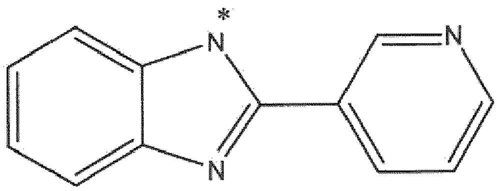 ; 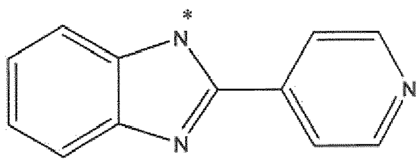

wherein the asterisk symbol "*" represents the binding position.

G may be selected independently from the group consisting of dimethylphosphinyl, diphenylphosphinyl, 2-phenyl-1H-benzo[d]imidazolyl, 2-etlhyl-1H-benzo[d]imidazolyl, 2-phenylbenzo[h]quinolinyl, pyridinyl, 2,2'-bipyridinyl, 5-phenylbenzo[4,5]imidazo[1,2-a]quinolinyl, 9-phenyl-1,10-phenanthrolinyl, 2-quinazolinyl, 4-quinazolinyl, 4-phenyl-2-quinazolinyl and (pyridine-2-yl)imidazo[1,5-a]pyridinyl;

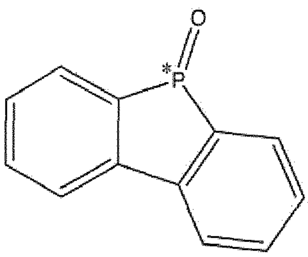 ; 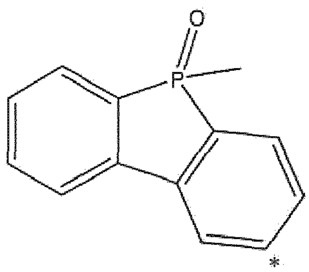 ; 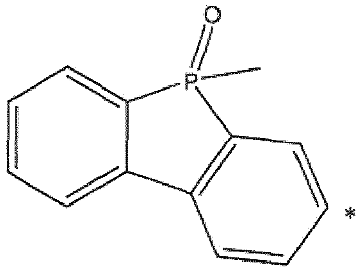 ;

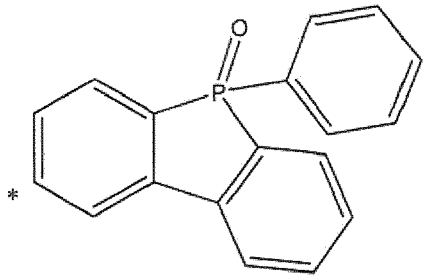 ; 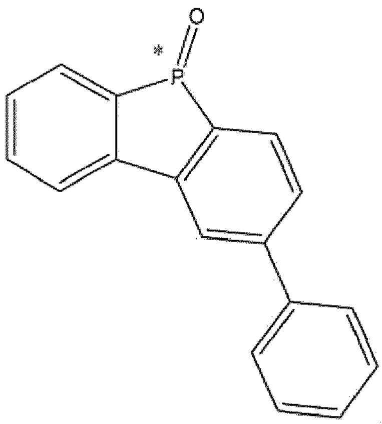 ; 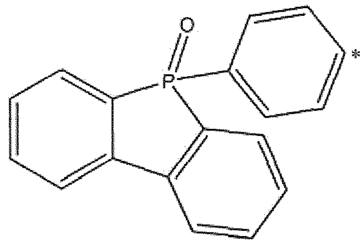

; ;

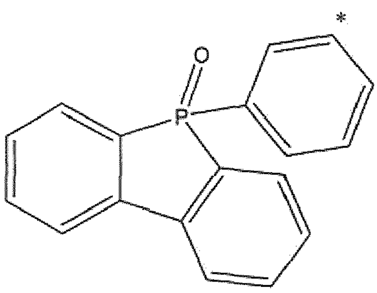 ; 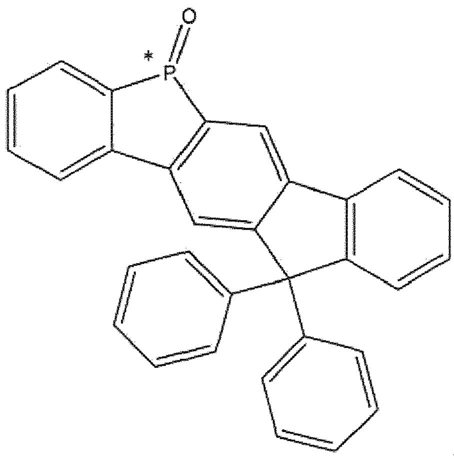 ;

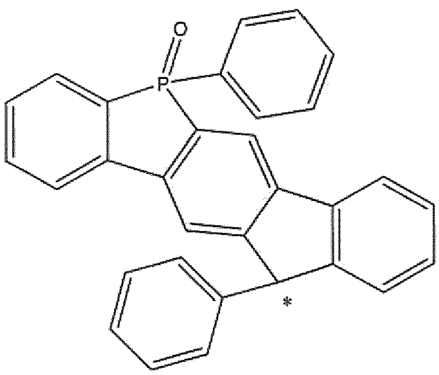 ; 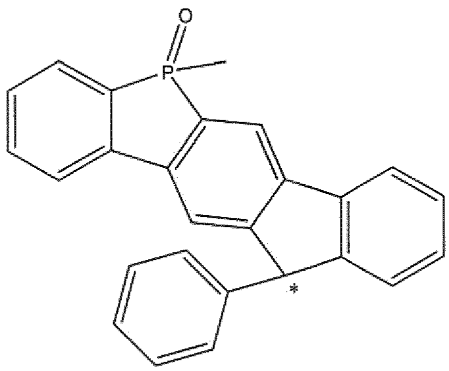 ; 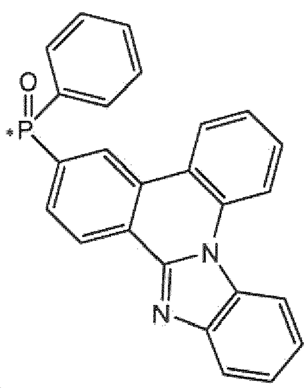

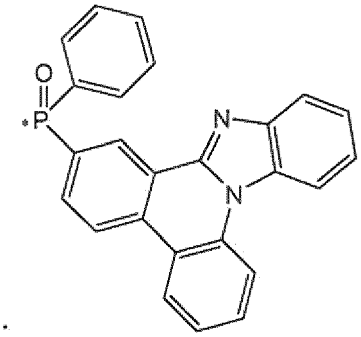

;

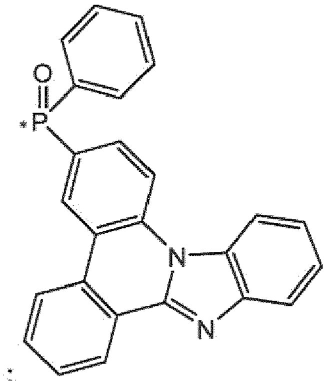

;

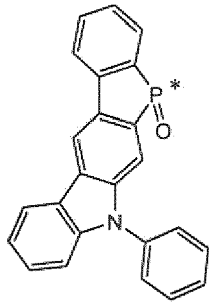

;

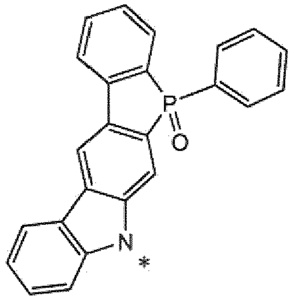

;

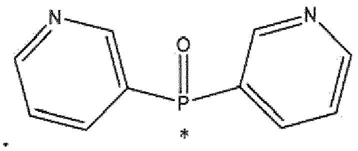

;

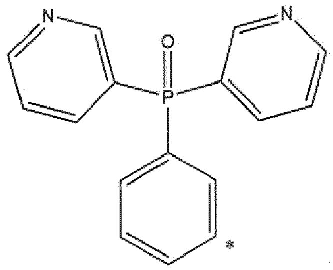

;

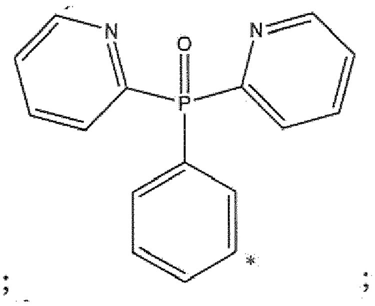

;

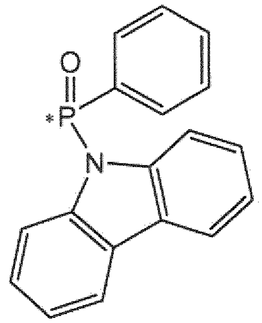

;

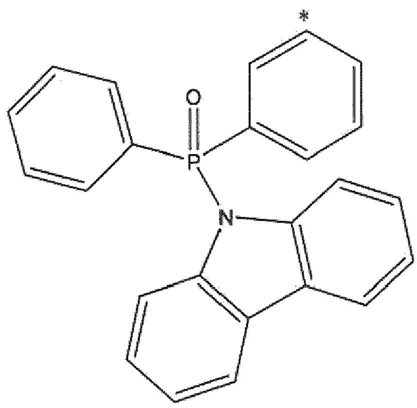

;

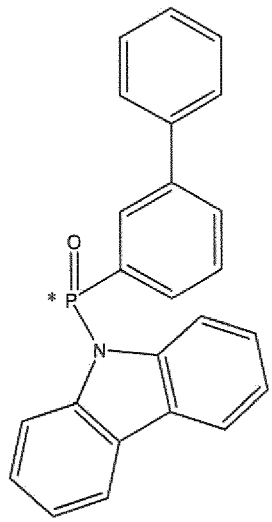

;

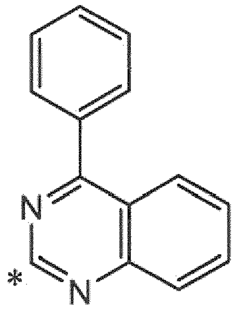

;

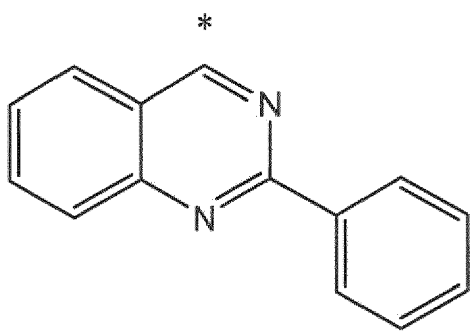

;

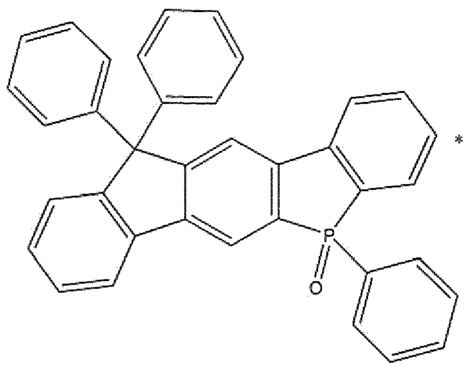

;

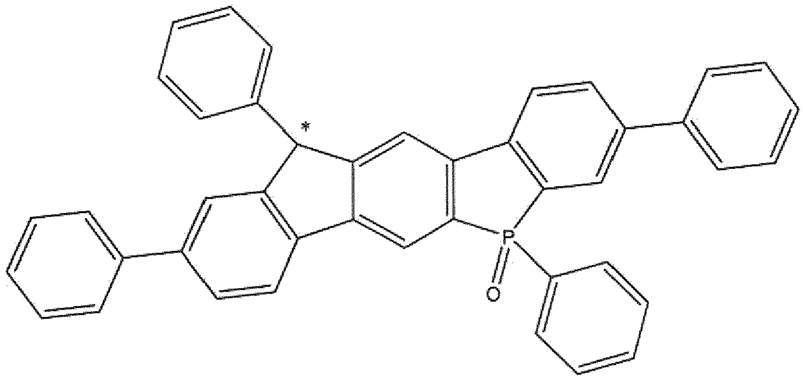

;

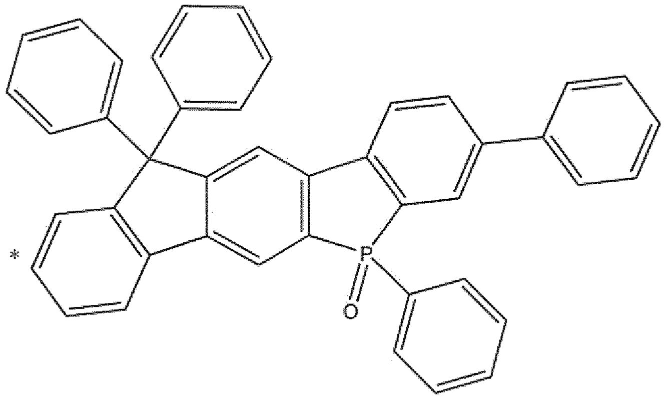

;

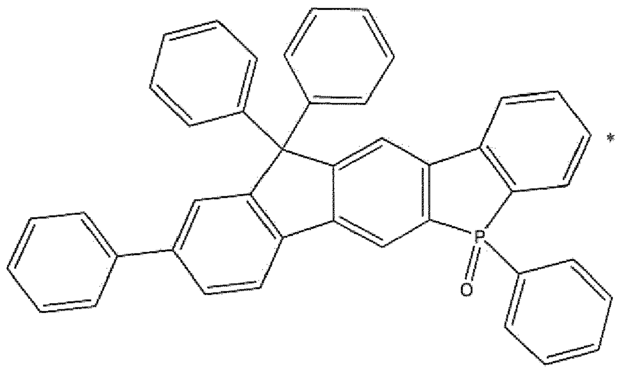

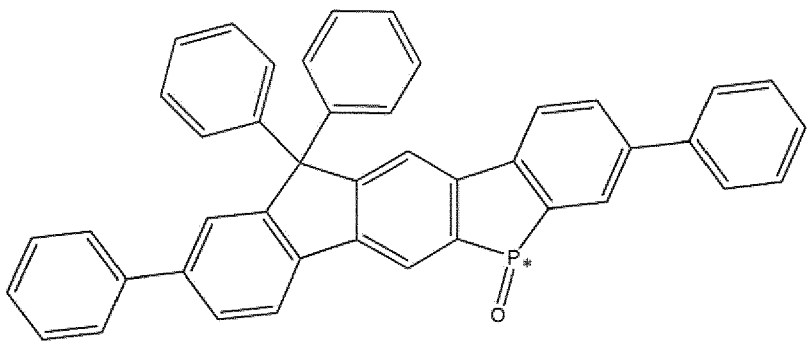

;

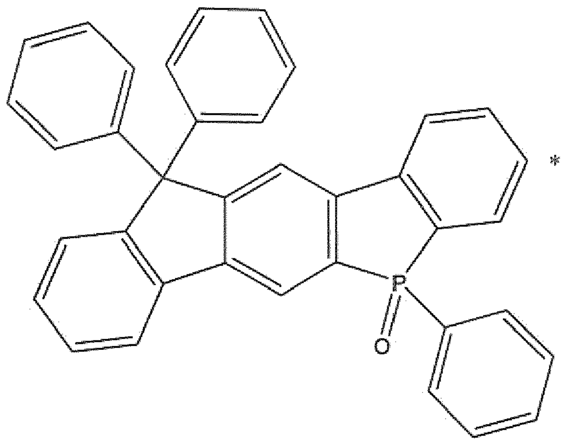

;

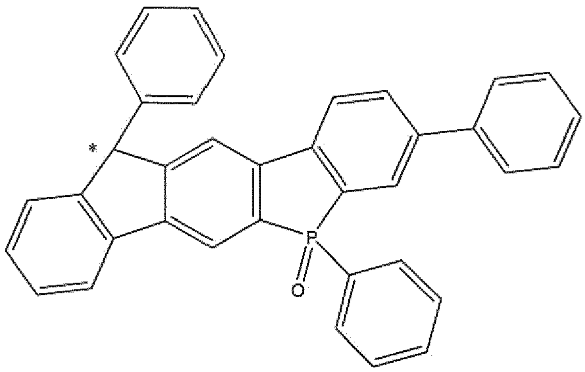

;

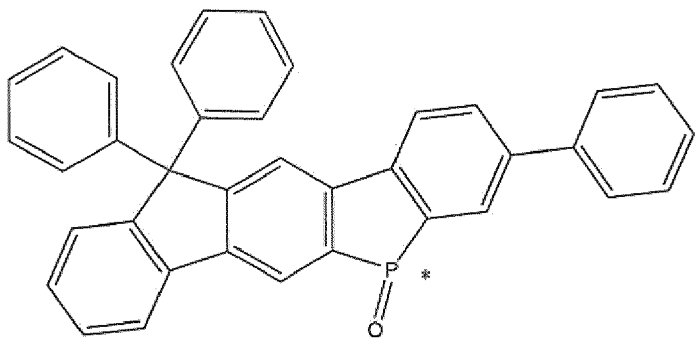

;

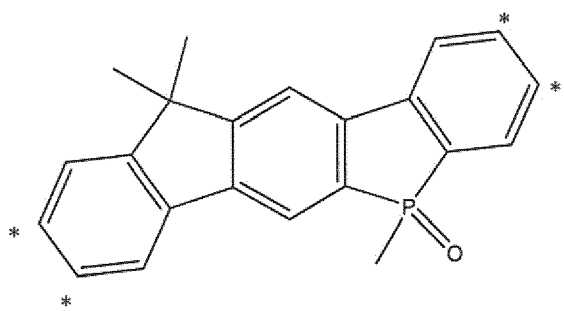

;

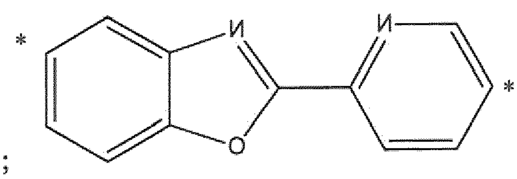

;

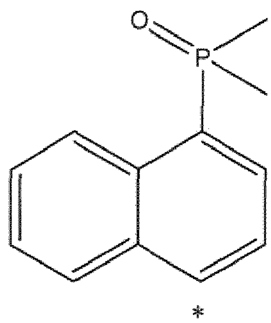

;

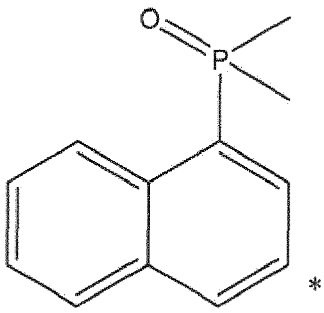

;

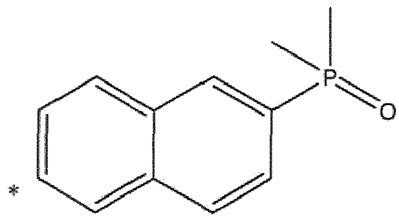

;

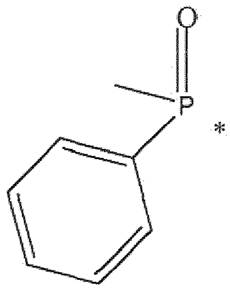;
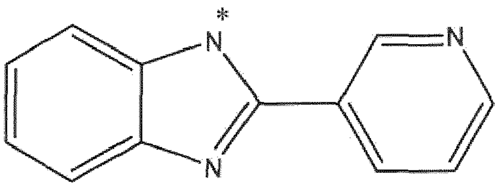;
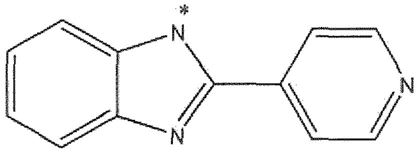

wherein the asterisk symbol "*" represents the binding position.

The compound of formula (I) may be selected from the compounds B-1 to B-26 of the following Table 2.

Table 2:

| Name | Structure | HOMO [eV] | LUMO [eV] | Dipole moment [D] |
|---|---|---|---|---|
| B-1 | 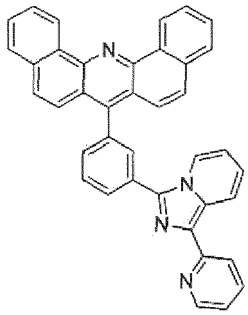 | -5.03 | -1.81 | 0.98 |
| B-2 | 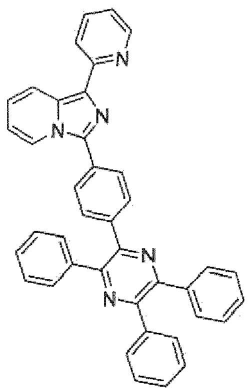 | -4.94 | -1.61 | 1.77 |
| B-3 | 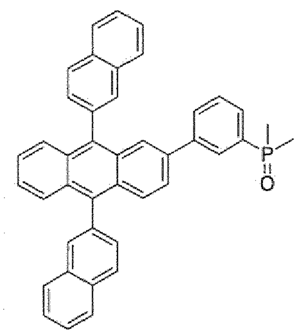 | -5.11 | -1.75 | 3.78 |

(continued)

| Name | Structure | HOMO [eV] | LUMO [eV] | Dipole moment [D] |
|---|---|---|---|---|
| B-4 | 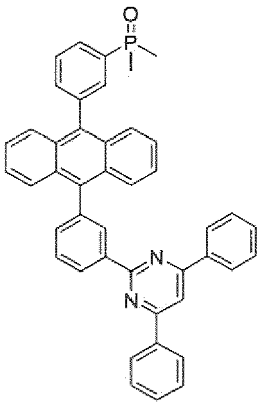 | -5.20 | -1.75 | 6.15 |
| B-5 | 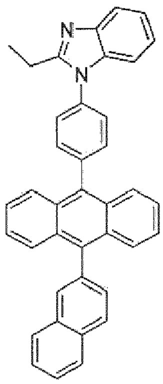 | -5.26 | -1.81 | 4.32 |
| B-6 | 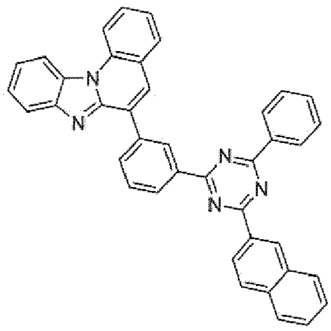 | -5.56 | -1.85 | 3.39 |
| B-7 | 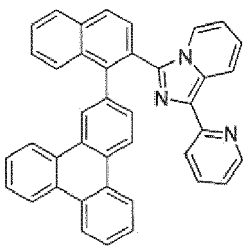 | -5.11 | -1.28 | 3.89 |

(continued)

| Name | Structure | HOMO [eV] | LUMO [eV] | Dipole moment [D] |
|---|---|---|---|---|
| B-8 | 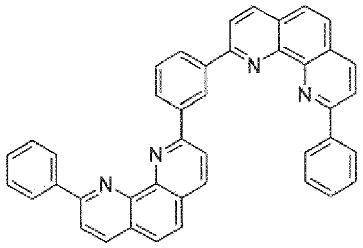 | -5.62 | -1.75 | 2.66 |
| B-9 | 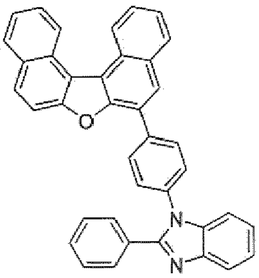 | -5.48 | -1.69 | 4.58 |
| B-10 | 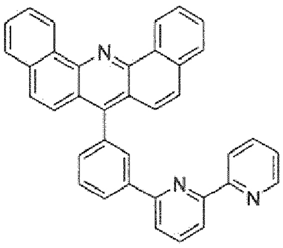 | -5.48 | -1.59 | 4.68 |
| B-11 | 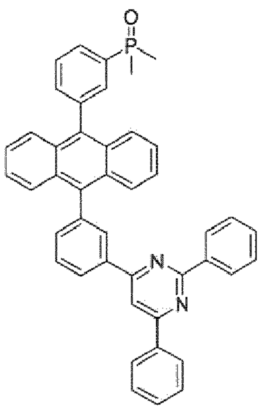 | -5.34 | -1.86 | 2.59 |
| B-12 | 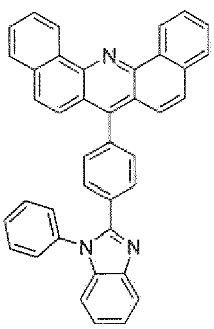 | -5.61 | -1.79 | 3.80 |

(continued)

| Name | Structure | HOMO [eV] | LUMO [eV] | Dipole moment [D] |
|---|---|---|---|---|
| B-13 | 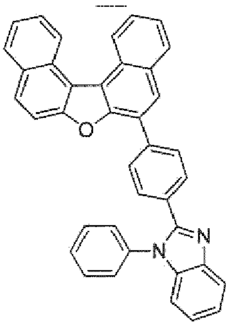 | -5.33 | -1.61 | 3.86 |
| B-14 | 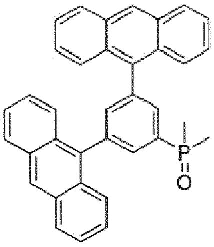 | -5.19 | -1.81 | 4.11 |
| B-15 | 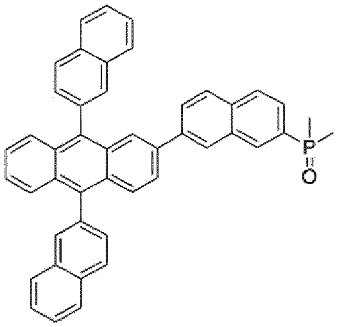 | -5.11 | -1.80 | 3.84 |
| B-16 | 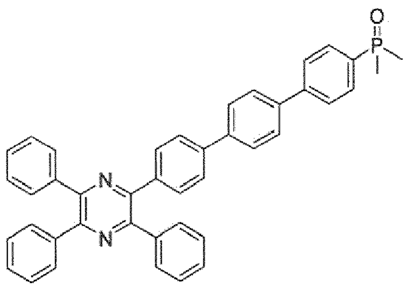 | -5.69 | -1.67 | 4.37 |
| B-17 | 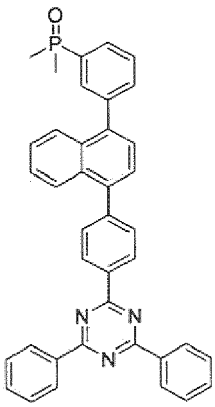 | -5.76 | -1.97 | 4.27 |

(continued)

| Name | Structure | HOMO [eV] | LUMO [eV] | Dipole moment [D] |
|---|---|---|---|---|
| B-18 | 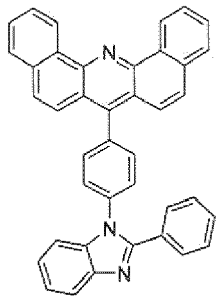 | -5.77 | -1.91 | 2.15 |
| B-19 | 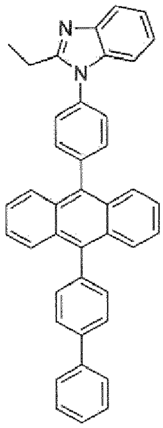 | -5.29 | -1.80 | 4.46 |
| B-20 | 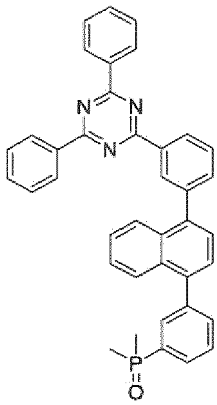 | -5.73 | -1.90 | 4.49 |
| B-21 | 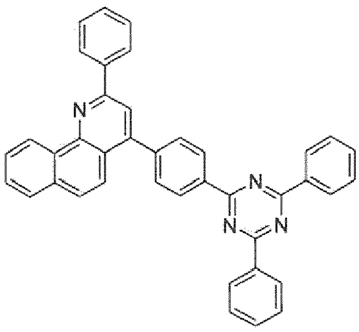 | -5.67 | -2.04 | 1.82 |

(continued)

| Name | Structure | HOMO [eV] | LUMO [eV] | Dipole moment [D] |
|---|---|---|---|---|
| B-22 | 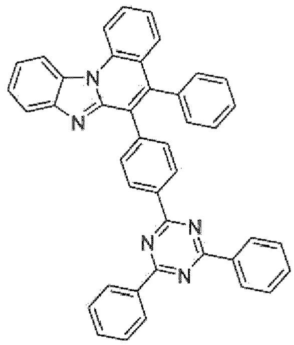 | -5.49 | -1.89 | 3.36 |
| B-23 | 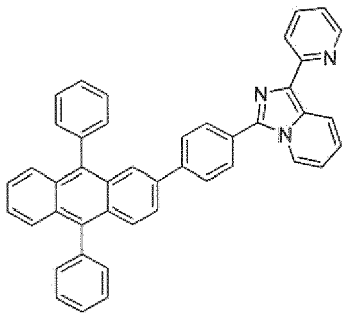 | -4.86 | -1.77 | 2.03 |
| B-24 | 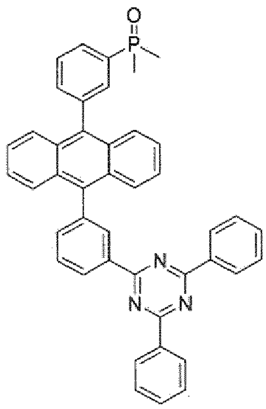 | -5.28 | -1.90 | 4.47 |
| B-25 | 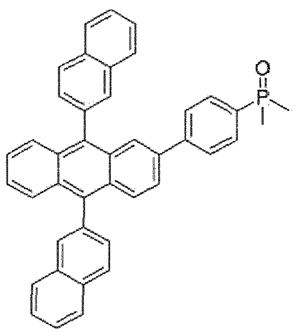 | -5.17 | -1.84 | 4.22 |

(continued)

| Name | Structure | HOMO [eV] | LUMO [eV] | Dipole moment [D] |
|---|---|---|---|---|
| B-26 | 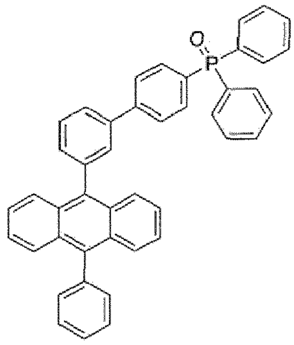 | -5.16 | -1.67 | 4.13 |

In an embodiment the LUMO energy level of the compound of formula (I) in the absolute scale taking vacuum energy level as zero, computed by the TURBOMOLE V6.5 program package using hybrid functional B3LYP and Gaussian 6-31G* basis set, is in the range from -2.30 eV to -1.20 eV, preferably from -2.10 eV to -1.28 eV.

In an embodiment the compound of formula (I) comprises one polar group "G".

It may be provided that the compound of formula (I) does not contain a moiety P=O. It may be provided that the compound of formula (I) does not contain $P(=O)Aryl_2$. It may be provided that the compound of formula (I) does not contain $P(=O)Alkyl_2$. It may be provided that the compound of formula (I) does not contain $P(=O)Ph_2$. It may be provided that the compound of formula (I) does not contain $P(=O)(CH_3)_2$. It may be provided that the compound of formula (I) does not contain R'P(=O)R" wherein R' and R" are connected with each other to form a ring, that is, does not contain ring-phosphine oxide. It may be provided that the compound of formula (I) does not contain R'P(=O)R" wherein R' and R" are connected with each other to form a 7-membered ring.

It may be provided that the compound of formula (I) does not contain two moieties P=O. It may be provided that wherein the compound of formula (I) does not contain two $P(=O)Aryl_2$. It may be provided that wherein the compound of formula (I) does not contain two $P(=O)Alkyl_2$. It may be provided that wherein the compound of formula (I) does not contain two $P(=O)Ph_2$. It may be provided that wherein the compound of formula (I) does not contain two $P(=O)(CH_3)_2$. It may be provided that wherein the compound of formula (I) does not contain CN.

It may be provided that one or more of the following formulas are excluded from the scope of the compound of formula (I)

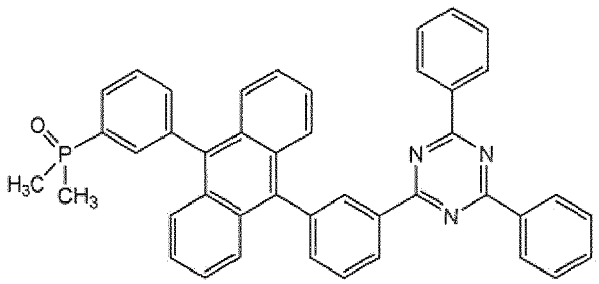 ; 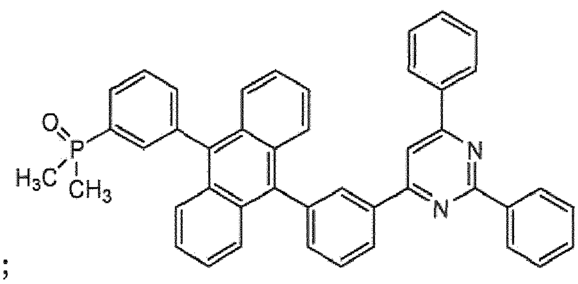 ;

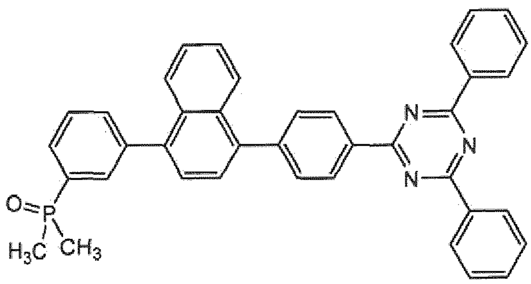 ; 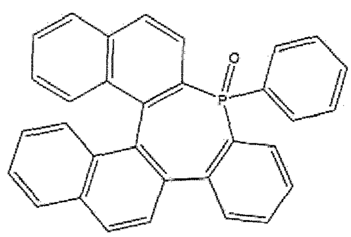 ;

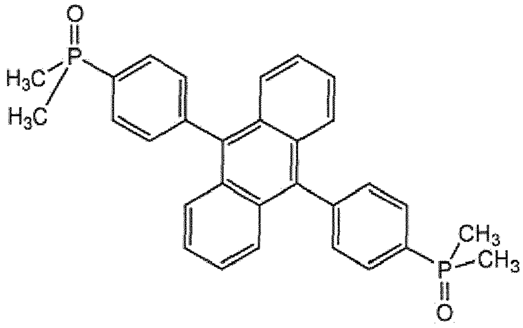
;
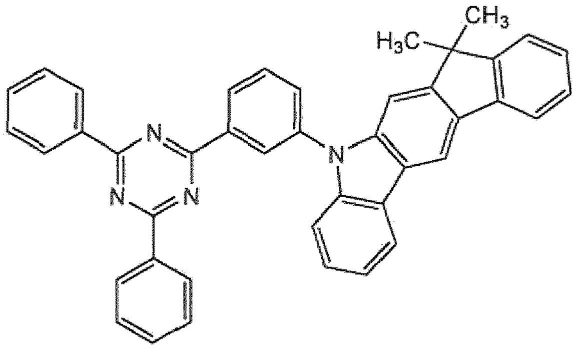
;

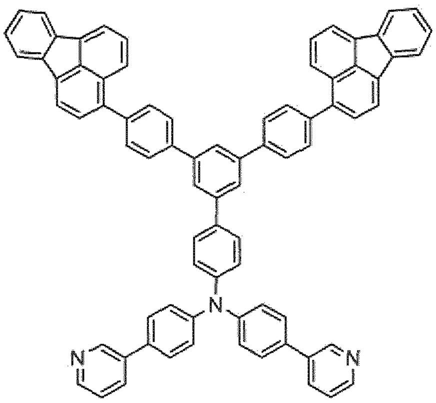
;
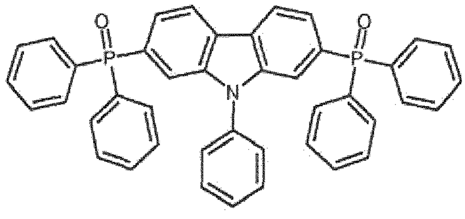
;

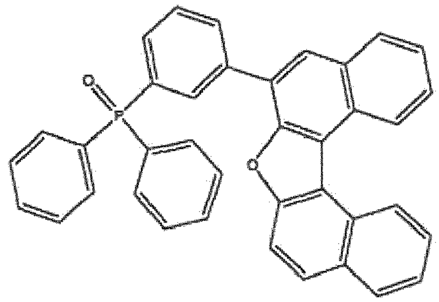
.

Compound of formula (II) and compound of formula (III)

The electron transport layer further comprises a compound of formula (II) and/or a compound of formula (III)

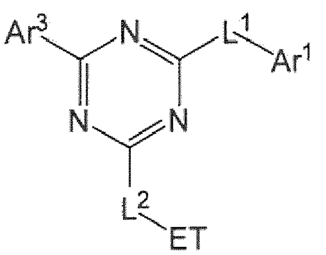
(II);
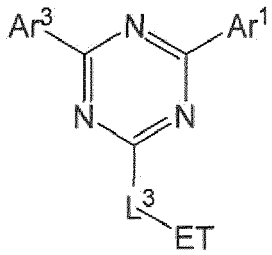
(III)

The electron transport layer may consist of a mixture of the compound of formula (I), the compound of formula (II) and/or (III) and one or more further compounds, provided that none of the further compounds is an electrical dopant. The electron transport layer may comprise more than one compound of formula (II). The electron transport layer may comprise more than one compound of formula (III). The electron transport layer may comprise one or more compounds

of formula (II) and one or more compounds of formula (III). The electron transport layer may consist of a mixture of the compound of formula (I) and the compound of formula (II) and/or (III). Exemplary further electron transport matrix compounds which may be contained are disclosed below.

$Ar^1$ may be independently selected from the group consisting of $C_6$ to $C_{12}$ aryl and $C_3$ to $C_{10}$ heteroaryl. $Ar^1$ may be independently selected from the group consisting of $C_6$ to $C_{10}$ aryl and $C_3$ to $C_5$ heteroaryl. $Ar^1$ may be independently selected from the group consisting of $C_6$ and $C_3$ to $C_5$ heteroaryl.

$Ar^1$ may be independently selected from the group consisting of $C_6$ to $C_{19}$ aryl. $Ar^1$ may be independently selected from the group consisting of $C_6$ to $C_{12}$ aryl. $Ar^1$ may be independently selected from the group consisting of $C_6$ to $C_{10}$ aryl. $Ar^1$ may be phenyl.

Ar3 may be independently selected from the group consisting of $C_6$ to $C_{12}$ aryl and $C_3$ to $C_{10}$ heteroaryl. $Ar^3$ may be independently selected from the group consisting of $C_6$ to $C_{10}$ aryl and $C_3$ to $C_5$ heteroaryl. $Ar^3$ may be independently selected from the group consisting of $C_6$ and $C_3$ to $C_5$ heteroaryl.

$Ar^3$ may be independently selected from the group consisting of $C_6$ to $C_{19}$ aryl. $Ar^3$ may be independently selected from the group consisting of $C_6$ to $C_{12}$ aryl. $Ar^3$ may be independently selected from the group consisting of $C_6$ to $C_{10}$ aryl. $Ar^3$ may be phenyl.

ET may be independently selected from $C_6$ to $C_{54}$ aryl or $C_2$ to $C_{39}$ heteroaryl, alternatively $C_6$ to $C_{48}$ aryl or $C_2$ to $C_{36}$ heteroaryl, alternatively $C_6$ to $C_{42}$ aryl or $C_2$ to $C_{36}$ heteroaryl, alternatively $C_6$ to $C_{36}$ aryl or $C_2$ to $C_{30}$ heteroaryl, alternatively $C_6$ to $C_{30}$ aryl or $C_2$ to $C_{24}$ heteroaryl, alternatively $C_6$ to $C_{20}$ aryl or $C_2$ to $C_{22}$ heteroaryl, alternatively $C_{12}$ to $C_{20}$ aryl or $C_7$ to $C_{22}$ heteroaryl, alternatively $C_{14}$ to $C_{20}$ aryl, such as $C_{20}$ aryl, or $C_{19}$ to $C_{22}$ heteroaryl.

ET may be independently selected from the group consisting of triphenylpyrazinyl; dibenzoacridinyl; pyridyl; anthracenyl; pyridylanthracenyl phenylanthracenyl and a compound of the following formula ET-i

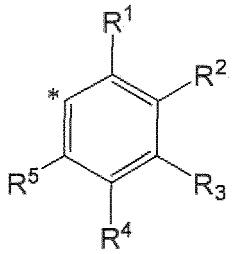
(ET-i)

wherein $R^1$ to $R^5$ are independently H or phenyl, provided that at least two, preferably at least three, most preferred four of $R^1$ to $R^5$ are phenyl with the remaining $R^1$ to $R^5$ being H; and ETi is bonded at * to $L^2$, respectively $L^3$.

ET may be independently selected from moieties of the following formulas

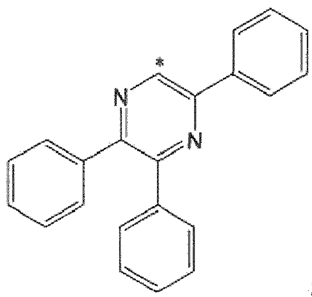
;
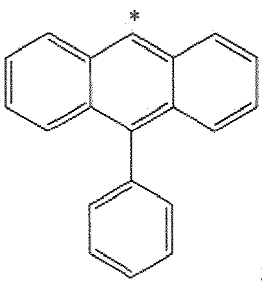
;
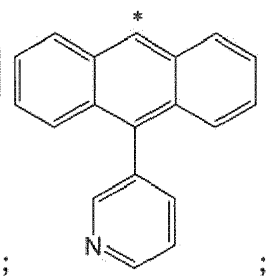
;
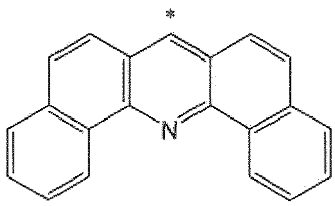
;
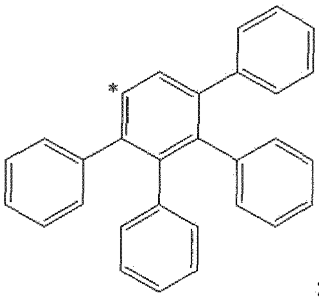
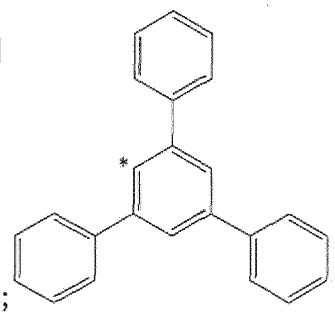
;

wherein the respective group ET is bonded at "*" to $L^2$, respectively to $L^3$.

$L^1$ has the formula (Iia)

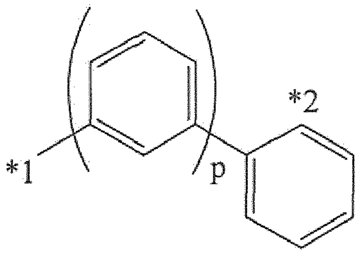

(Iia).

$L^1$ is bonded at *1 to the triazine moiety in formula (II) and at *2 to $Ar^2$, that is, makes the following connection

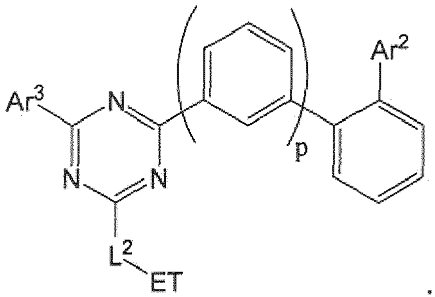

.

P is o or 1.

In case that p is o, $L^1$ has the following formula

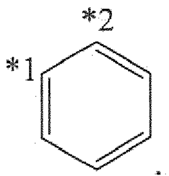

.

In case that p is 1, $L^1$ has the following formula

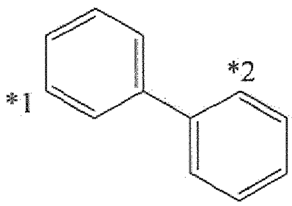

.

P may be o.

$L^3$ has the formula (IIIa.1) or (IIIa.2)

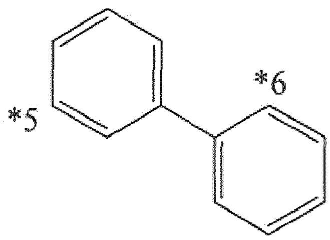

(IIIa.1)

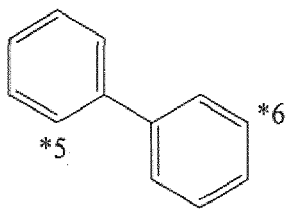

(IIIa.2).

$L^3$ is bonded at *5 to the triazine moiety in formula (III); and $L^3$ is bonded at *6 to ET, that is, makes the following connection (exemplified with IIIa.1)

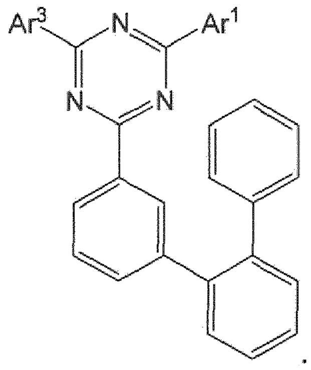

In one embodiment, the electron transport layer comprises the compound of formula (I) and the compound of formula (II) and "p" in formula (IIa) is o.

The compound of formula (II) and/or (III) may comprise 8 to 13 aromatic or heteroaromatic rings, optionally 8 to 11 aromatic or heteroaromatic rings, optionally 9 to 11 aromatic or heteroaromatic rings, and optionally 9 to 10 aromatic or heteroaromatic rings.

The compound of formula (II) and/or (III) may comprise 8 to 13 aromatic or heteroaromatic rings, wherein 1 to 3 of the aromatic or heteroaromatic rings are N-containing rings; optionally 8 to 11 aromatic or heteroaromatic rings, wherein 1 to 3 of the aromatic or heteroaromatic rings are N-containing rings; optionally 9 to 11 aromatic or heteroaromatic rings, wherein 1 or 2 of the aromatic or heteroaromatic rings are N-containing rings; and optionally 9 to 10 aromatic or heteroaromatic rings wherein 1 or 2 of the aromatic or heteroaromatic rings are N-containing rings.

The compound of formula (II) and/or (III) may comprise 8 to 13 aromatic or heteroaromatic rings, wherein 6 to 10 of the aromatic or heteroaromatic rings are aryl rings; optionally 8 to 11 aromatic or heteroaromatic rings, wherein 6 to 10 of the aromatic or heteroaromatic rings are aryl rings; optionally 9 to 11 aromatic or heteroaromatic rings, wherein 7 to 9 of the aromatic or heteroaromatic rings are aryl rings; and optionally 9 to 10 aromatic or heteroaromatic rings wherein 7 to 9 of the aromatic or heteroaromatic rings are N-containing rings.

The compound of formula (II) and/or (III) may have a ratio of aryl rings to N-containing rings may be from 10:1 to 3:1, such as from 9:1 to 3.5:1.

The compound of formula (II) and/or (III) may have a LUMO in the range from -2.0 to -1.5 eV, alternatively -1.9 to -1.6 eV, such as from -1.86 to -1.69 eV.

The compound of formula (II) and/or (III) may have a refractive index at a wavelength of 633 nm in the range from 1.5 to 2.0, alternatively from 1.6 to 1.9, alternatively from 1.7 to 1.8, such as from 1.72 to 1.78.

The compound of formula (II), respectively of formula (III) may be selected from the compounds E-1 to E-8

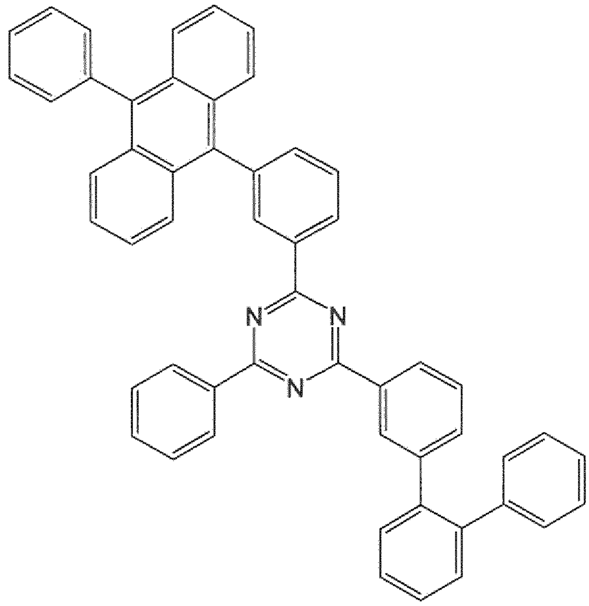

E-1

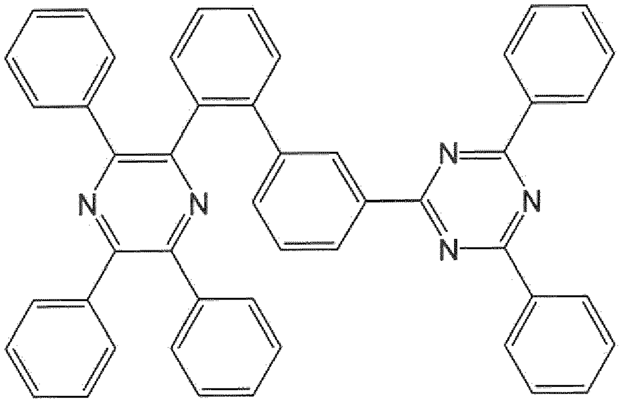

E-2

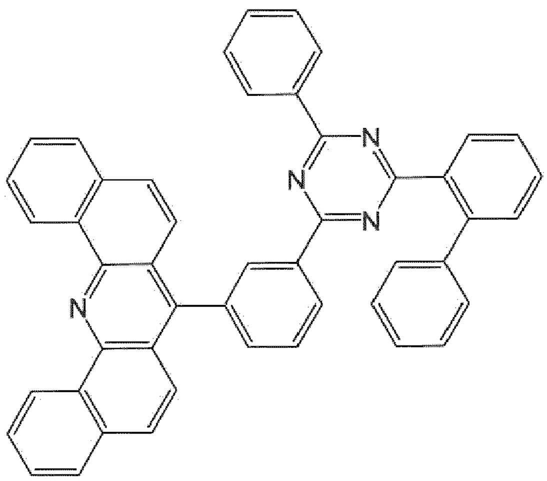

E-3

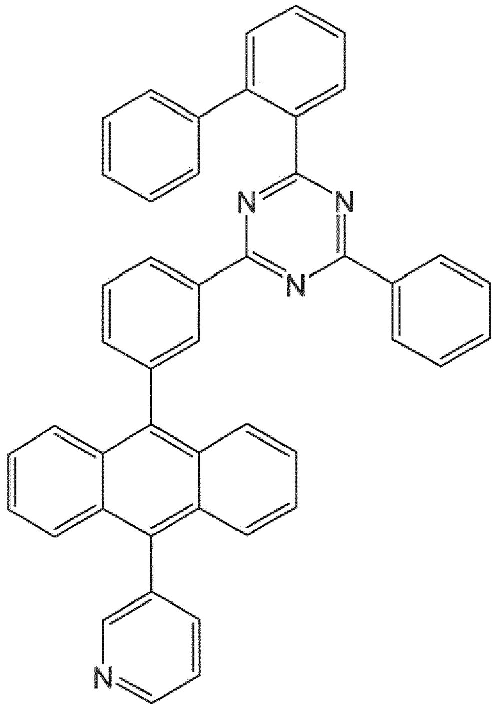

E-4.

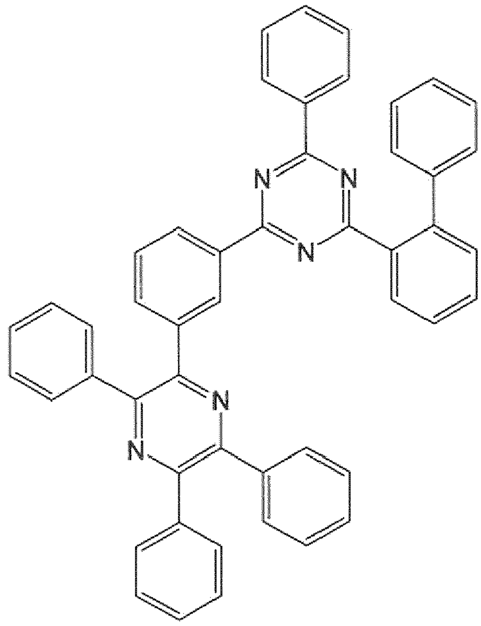

E-5

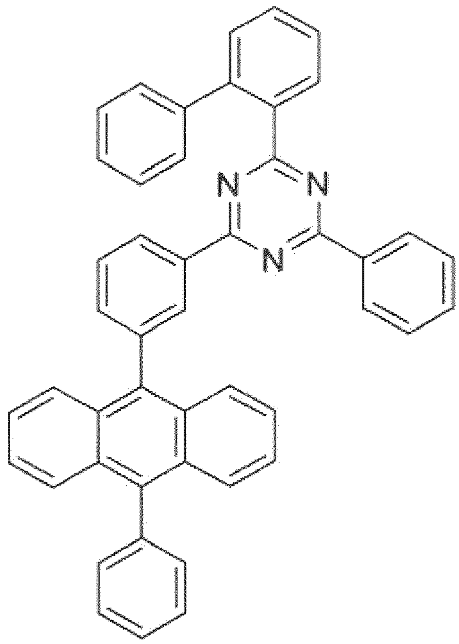

E-6

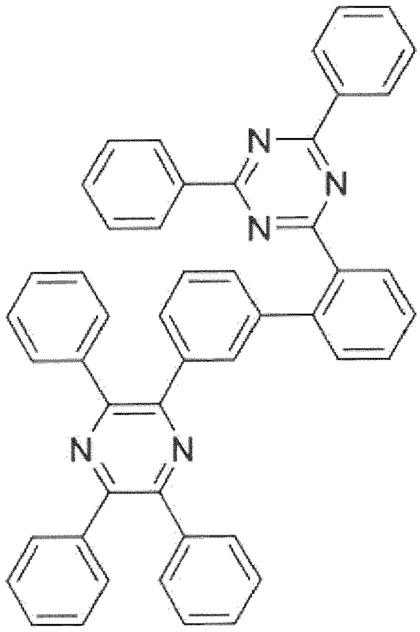

E-7

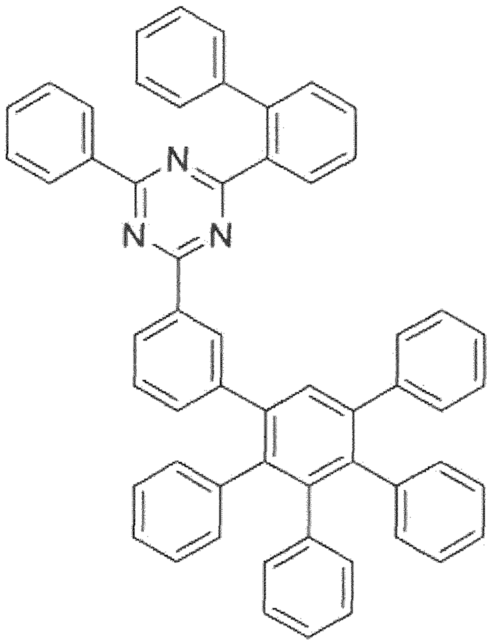

E-8

Exemplary embodiments

According to one embodiment, there is provided an organic light emitting diode, comprising an anode, a cathode, an emission layer, and an electron transport layer,
wherein

- the electron transport layer is arranged between the emission layer and the cathode;
- the electron transport layer is free of an electrical dopant;
- the electron transport layer comprises a compound of formula (I)

$$(Ar^2)_m\text{-}(Z_k\text{-}G)_n \qquad (I);$$

wherein in formula (I)

- m and n are independently 1 or 2;
- k is independently 0, 1 or 2;
- $Ar^2$ is independently selected from the group consisting of $C_3$ to $C_{30}$ heteroaryl and $C_6$ to $C_{42}$ aryl,
  - wherein each $Ar^2$ may be substituted with one or two substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$, to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - wherein each $C_6$ to $C_{12}$ aryl substituent on $Ar^2$ and each $C_3$ to $C_{11}$ heteroaryl substituent on $Ar^2$ may be substituted with $C_1$ to $C_4$ alkyl or halogen;
- Z is independently selected from $C_6$ to $C_{18}$ aryl,

- wherein each Z may be substituted with one or two substituents independently selected from the group consisting of $C_6$ to $C_{12}$ aryl and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- wherein each $C_6$ to $C_{12}$ aryl substituent on Z may be substituted with $C_1$ to $C_4$ alkyl or halogen;

- G is chosen so that the dipole moment of a compound G-phenyl is ≥ 2 D and ≤ 6 D;

- the electron transport layer further comprises a compound of formula (II) and/or a compound of formula (III)

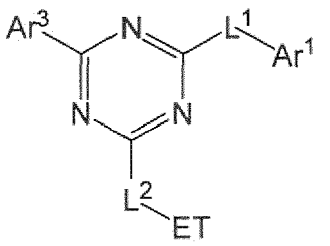
(II);
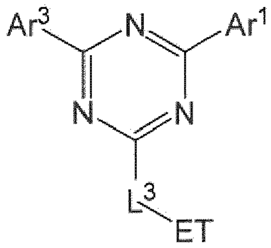
(III)

wherein in formula (II) and in formula (III) respectively

- $Ar^1$ is independently selected from the group consisting of $C_6$ to $C_{12}$ aryl and $C_3$ to $C_{10}$ heteroaryl;

- $Ar_3$ is independently selected from the group consisting of $C_6$ to $C_{12}$ aryl and $C_3$ to $C_{10}$ heteroaryl;

- ET is independently selected from $C_6$ to $C_{30}$ aryl or $C_2$ to $C_{24}$ heteroaryl;

- $L^1$ has the formula (IIa)

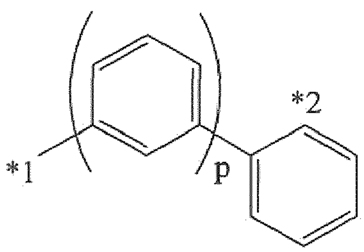
(IIa)

wherein $L^1$ is bonded at *1 to the triazine moiety in formula (II); and $L^1$ is bonded at *2 to $Ar^1$; and p is o or 1;

- $L^2$ has the formula (IIb)

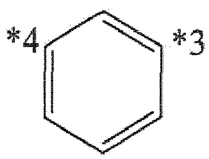
(IIb)

wherein $L^2$ is bonded at *3 to the triazine moiety in formula (II); and $L^2$ is bonded at *4 to ET; and

- $L^3$ has the formula (IIIa.1) or (IIIa.2)

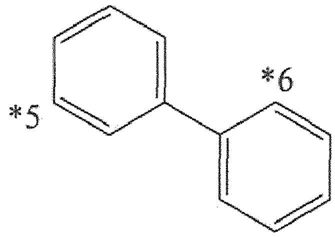

(IIIa.1)

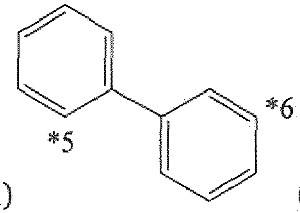

(IIIa.2).

wherein $L^3$ is bonded at *5 to the triazine moiety in formula (III); and $L^3$ is bonded at *6 to ET.

According to one embodiment, there is provided an organic light emitting diode, comprising an anode, a cathode, an emission layer, and an electron transport layer,
wherein

- the electron transport layer is arranged between the emission layer and the cathode;
- the electron transport layer is free of an electrical dopant;
- the electron transport layer comprises a compound of formula (1)

$$(Ar^2)_m\text{-}(Z_k\text{-}G)_n \qquad (I);$$

wherein in formula (I)

- m and n are independently 1 or 2;
- k is independently 1 or 2;
- $Ar^2$ is independently selected from the group consisting of $C_3$ to $C_{21}$ N-containing heteroaryl and $C_6$ to $C_{24}$ aryl,
  - wherein each $Ar^2$ may be substituted with one or two substituents independently selected from the group consisting of phenyl, pyridinyl and biphenyl-yl, optionally para-biphenyl-yl.;
- Z is independently selected from the group consisting of phenylene, naphthylene, phenylene-naphthylene, biphenylene and terphenylene,
  - wherein each Z may be substituted with one or two substituents independently selected from the group consisting of phenyl and $C_1$ to $C_4$ alkyl;
- G is selected from the group consisting of $C_2$ to $C_{25}$ heteroaryl; G may include one or more substituents attached to the group, wherein the one or more substituents are selected from the group consisting of $C_6$ to $C_{12}$ aryl, $C_1$ to $C_6$ alkyl, $C_2$ to $C_{11}$ heteroaryl;
- the electron transport layer further comprises a compound of formula (II) and/or a compound of formula (III)

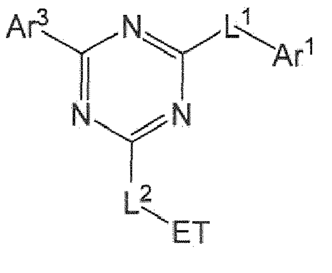

(II);

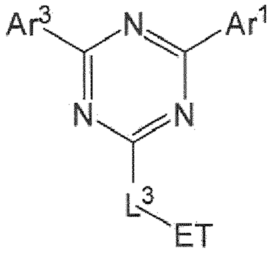

(III)

wherein in formula (II) and in formula (III) respectively

- $Ar^1$ is independently selected from the group consisting of $C_6$ to $C_{19}$ aryl;
- $Ar^3$ is independently selected from the group consisting of $C_6$ to $C_{19}$ aryl;
- ET is independently selected from $C_{14}$ to $C_{30}$ aryl, such as $C_{20}$ to $C_{30}$ aryl, or $C_{19}$ to $C_{22}$ heteroaryl;
- $L^1$ has the formula (IIa)

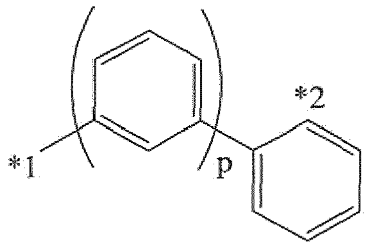

(IIa)

wherein $L^1$ is bonded at *1 to the triazine moiety in formula (II); and $L^1$ is bonded at *2 to $Ar^1$; and p is 0 or 1;

- $L^2$ has the formula (IIb)

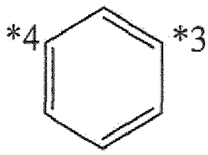

(IIb)

wherein $L^2$ is bonded at *3 to the triazine moiety in formula (II); and $L^2$ is bonded at *4 to ET; and

- $L^3$ has the formula (IIIa.1) or (IIIa.2)

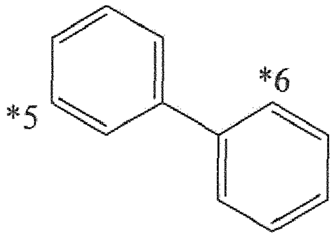

(IIIa.1)

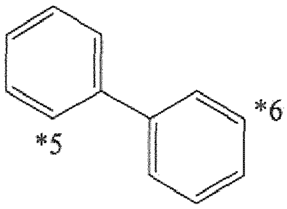

(IIIa.2).

wherein $L^3$ is bonded at *5 to the triazine moiety in formula (III); and $L^3$ is bonded at *6 to ET.

According to one embodiment, there is provided an organic light emitting diode, comprising an anode, a cathode, an emission layer, and an electron transport layer,
wherein

- the electron transport layer is arranged between the emission layer and the cathode;
- the electron transport layer is free of an electrical dopant;
- the electron transport layer comprises a compound of formula (I)

$$(Ar^2)_m\text{-}(Z_k\text{-}G)_n \qquad (I);$$

wherein in formula (I)

- m and n are independently 1 or 2;
- k is independently 1 or 2;
- $Ar^2$ is independently selected from the group consisting of dibenzoacridinyl, 1,3-diazinyl, 1,4-diazinyl, anthracenyl, triazinyl, phenathrolinyl, triphenylenyl, pyridinyl, dinaphthofuranyl,
    - wherein each $Ar^2$ may be substituted with one or two substituents independently selected from the group consisting of phenyl, pyridinyl and biphenyl-yl, optionally para-biphenyl-yl.;
- Z is independently selected from the group consisting of phenylene, naphthylene, phenylene-naphthylene, biphenylene and terphenylene,
    - wherein each Z may be substituted with one or two substituents independently selected from the group consisting of phenyl and $C_1$ to $C_4$ alkyl;
- G is selected from the group consisting of dimethylphosphinyl, diphenylphosphinyl, nitrile, benzonitrile, nicotinonitrile, di-hydro-benzoimidazolone-yl, diphenyl-propane-yl, *N,N*-dimethylacetamid, amide, carbamide, imidazolyl, phenylbenzoimidazolyl, ethylbenzoimidazolyl phenylbenzoquinolinyl, phenylbenzoimidazoquinolinyl, pyridinyl, bipyridinyl, picolinyl, lutidenyl, pyridazinyl, pyrimidinyl, pyrazinyl, triphenyl-pyrazinyl, benzoquinolinyl, phenanthrolinyl, phenylphenanthrolinyl and pyridinyl-imidazopyridinyl;
- the electron transport layer further comprises a compound of formula (II) and/or a compound of formula (III)

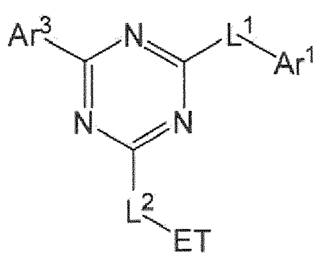
(II);
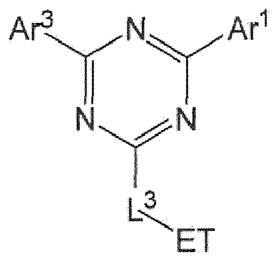
(III)

wherein in formula (II) and in formula (III) respectively

- $Ar^1$ is phenyl;
- $Ar^3$ is phenyl;
- ET is independently selected from triphenylpyrazinyl; dibenzoacridinyl; pyridyl; anthracenyl; pyridylanthracenyl; phenylanthracenyl and a compound of the following formula ET-i

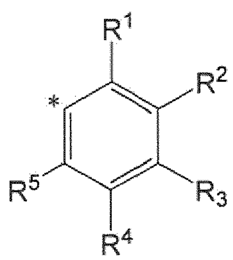
(ET-i)

wherein $R^1$ to $R^5$ are independently H or phenyl, provided that at least two, preferably at least three, most preferred four of $R^1$ to $R^5$ are phenyl with the remaining $R^1$ to $R^5$ being H; and ETi is bonded at * to $L^2$, respectively $L^3$
;

- $L^1$ has the formula (IIa)

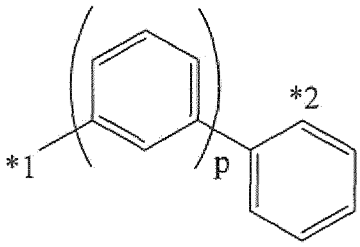

(IIa)

wherein $L^1$ is bonded at *1 to the triazine moiety in formula (II); and $L^1$ is bonded at *2 to $Ar^1$; and p is o or 1;

- $L^2$ has the formula (IIb)

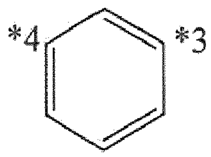

(IIb)

wherein $L^2$ is bonded at *3 to the triazine moiety in formula (II); and $L^2$ is bonded at *4 to ET; and

- $L^3$ has the formula (IIIa.1) or (IIIa.2)

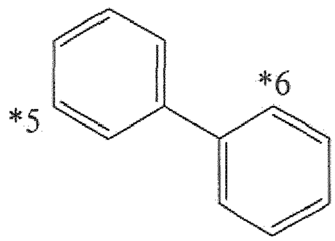

(IIIa.1)

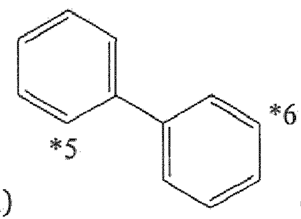

(IIIa.2).

wherein $L^3$ is bonded at *5 to the triazine moiety in formula (III); and $L^3$ is bonded at *6 to ET.

<u>Compound of the formula (IV) or (V)</u>

The invention further relates to a compound having the formula (IV) or the formula (V)

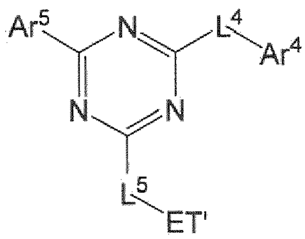

(IV)

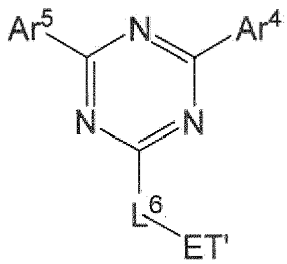

(V).

$Ar^4$ may be independently selected from the group consisting of $C_6$ to $C_{12}$ aryl and $C_3$ to $C_{10}$ heteroaryl. $Ar^4$ may be independently selected from the group consisting of $C_6$ to $C_{10}$ aryl and $C_3$ to $C_5$ heteroaryl. $Ar^4$ may be independently selected from the group consisting of $C_6$ and $C_3$ to $C_5$ heteroaryl.

$Ar^4$ may be independently selected from the group consisting of $C_6$ to $C_{19}$ aryl. $Ar^5$ may be independently

selected from the group consisting of $C_6$ to $C_{12}$ aryl. $Ar^4$ may be independently selected from the group consisting of $C_6$ to $C_{10}$ aryl. $Ar^4$ may be phenyl.

$Ar^5$ may be independently selected from the group consisting of $C_6$ to $C_{12}$ aryl and $C_3$ to $C_{10}$ heteroaryl. $Ar^5$ may be independently selected from the group consisting of $C_6$ to $C_{10}$ aryl and $C_3$ to $C_5$ heteroaryl. $Ar^5$ may be independently selected from the group consisting of $C_6$ and $C_3$ to $C_5$ heteroaryl.

$Ar^5$ may be independently selected from the group consisting of $C_6$ to $C_{19}$ aryl. $Ar^5$ may be independently selected from the group consisting of $C_6$ to $C_{12}$ aryl. $Ar^5$ may be independently selected from the group consisting of $C_6$ to $C_{10}$ aryl. $Ar^5$ may be phenyl.

ET' is selected selected from the group consisting of triphenylpyrazinyl; pyridine-anthracenyl; acridine, benzoacridine, dibenzoacridine, phenylanthracenyl and a compound of the formula ET'-i

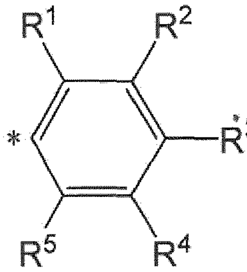

ET'-i

wherein $R^1$ to $R^5$ are independently H or phenyl, provided that at least two, preferably at least three, most preferred four of $R^1$ to $R^5$ are phenyl with the remaining $R^1$ to $R^5$ being H; and ET'-i is bonded at * to $L^9$, respectively to $R^6$.

ET' may be selected selected from the group consisting of triphenylpyrazinyl; pyridine-anthracenyl; acridine, benzoacridine and dibenzoacridine.

ET may be independently from moieties of the following formulas

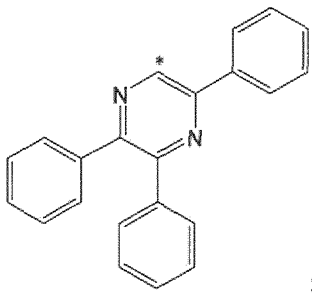

;

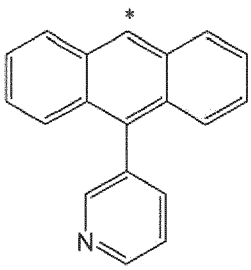

;

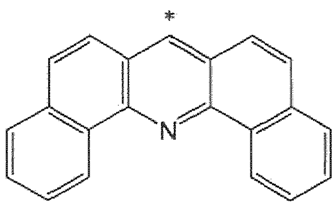

;

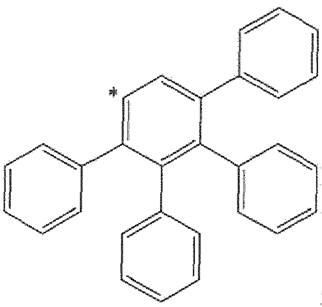

;

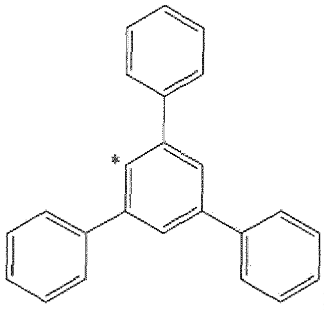

;

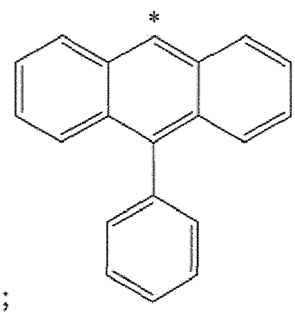

wherein the respective group ET' is bonded at "*" to $L^5$, respectively to $L^6$.

ET may be independently from moieties of the following formulas

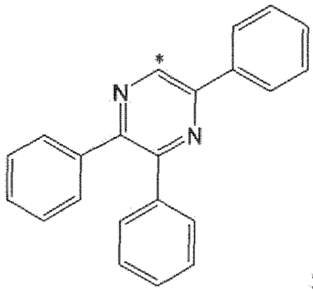 ; 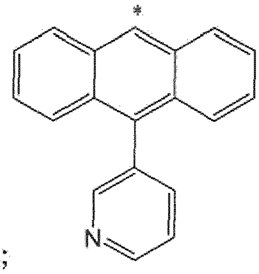 ; 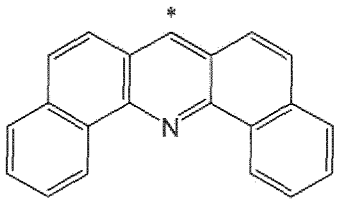 ;

wherein the respective group ET' is bonded at "*" to $L^5$, respectively to $L^6$.

$L^4$ has the formula (IVa)

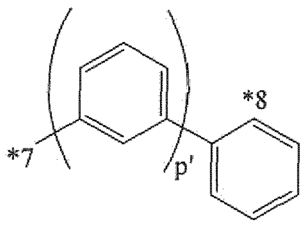

(IVa).

$L^4$ is bonded at *7 to the triazine moiety in formula (IV) and at *8 to $Ar^4$, that is, makes the following connection

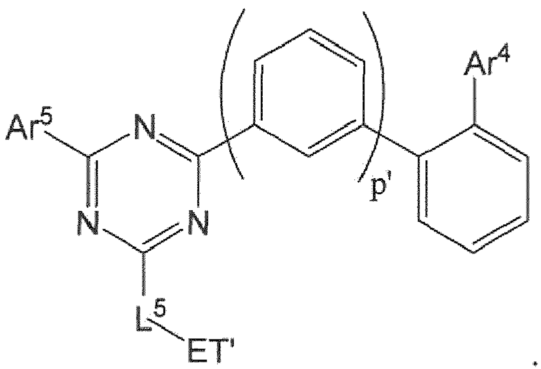

.

p'is 0 or 1.

In case that p' is 0, $L^4$ has the following formula

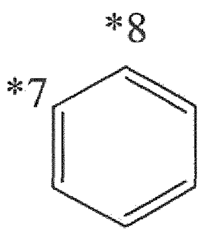

.

In case that p' is 1, $L^4$ has the following formula

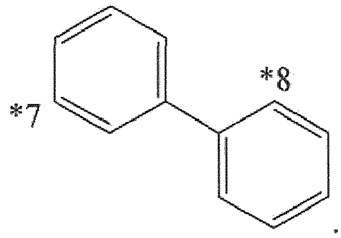

p' may be o.

$L^6$ has the formula (Va.1) or (Va.2)

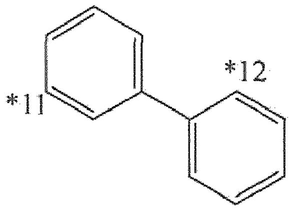

(Va.1)

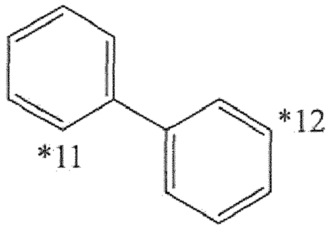

(Va.2).

$L^6$ is bonded at *11 to the triazine moiety in formula (V); and $L^6$ is bonded at *12 to ET', that is, makes the following connection (exemplified with Va.1)

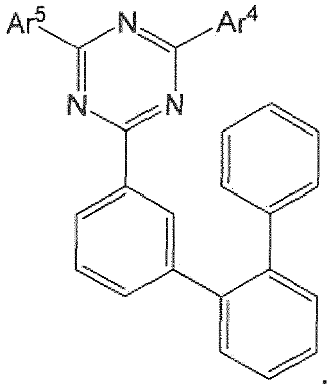

In one embodiment, the electron transport layer comprises the compound of formula (IV) and "p'" in formula (IVa) is o.

The compound of formula (IV) and/or (V) may comprise 8 to 13 aromatic or heteroaromatic rings, optionally 8 to 11 aromatic or heteroaromatic rings, optionally 9 to 11 aromatic or heteroaromatic rings, and optionally 9 to 10 aromatic or heteroaromatic rings.

The compound of formula (IV) and/or (V) may comprise 8 to 13 aromatic or heteroaromatic rings, wherein 1 to 3 of the aromatic or heteroaromatic rings are N-containing rings; optionally 8 to 11 aromatic or heteroaromatic rings, wherein 1 to 3 of the aromatic or heteroaromatic rings are N-containing rings; optionally 9 to 11 aromatic or heteroaromatic rings, wherein 1 or 2 of the aromatic or heteroaromatic rings are N-containing rings; and optionally 9 to 10 aromatic or heteroaromatic rings wherein 1 or 2 of the aromatic or heteroaromatic rings are N-containing rings.

The compound of formula (IV) and/or (V) may comprise 8 to 13 aromatic or heteroaromatic rings, wherein 6 to 10 of the aromatic or heteroaromatic rings are aryl rings; optionally 8 to 11 aromatic or heteroaromatic rings, wherein 6 to 10 of the aromatic or heteroaromatic rings are aryl rings; optionally 9 to 11 aromatic or heteroaromatic rings, wherein 7 to 9 of the aromatic or heteroaromatic rings are aryl rings; and optionally 9 to 10 aromatic or heteroaromatic rings wherein 7 to 9 of the aromatic or heteroaromatic rings are N-containing rings.

The compound of formula (IV) and/or (V) may have a ratio of aryl rings to N-containing rings may be from 10:1 to 3:1, such as from 9:1 to 3.5:1.

The compound of formula (IV) and/or (V) may have a LUMO in the range from -2.0 to -1.5 eV, alternatively -1.9 to -1.6 eV, such as from -1.86 to -1.69 eV.

The compound of formula (IV) and/or (V) may have a refractive index at a wavelength of 633 nm in the range

from 1.5 to 2.0, alternatively from 1.6 to 1.9, alternatively from 1.7 to 1.8, such as from 1.72 to 1.78. when measured as a single layer consisting of the respective compound of formula (IV) and/or (V) as shown in table 1

The electron transport layer comprising a compound of formula (II) and / or formula (III) may have a refractive index at a wavelength of 633 nm in the range from 1.5 to 2.0, alternatively from 1.6 to 1.9, alternatively from 1.7 to 1.8, such as from 1.70 to 1.78. when measured as a single layer comprising the respective compound of formula (II) and/or (III) as shown in table 1.

The compound of formula (IV), respectively of formula (V) may be selected from the compounds E-1, E-2, E-4 and E-7

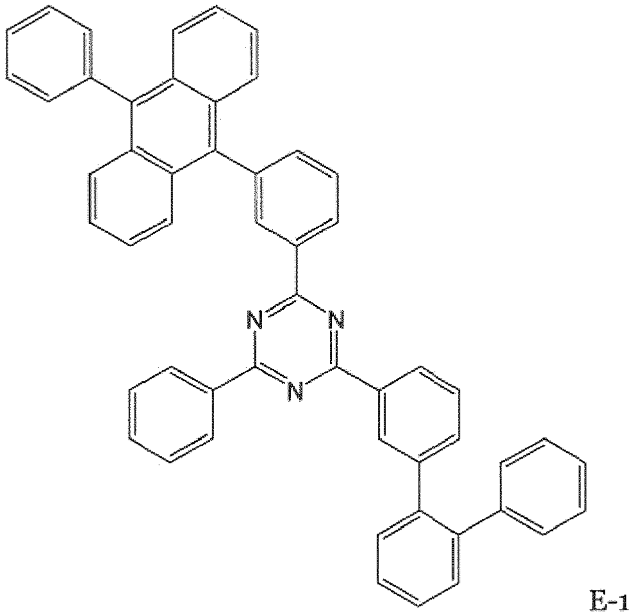

E-1

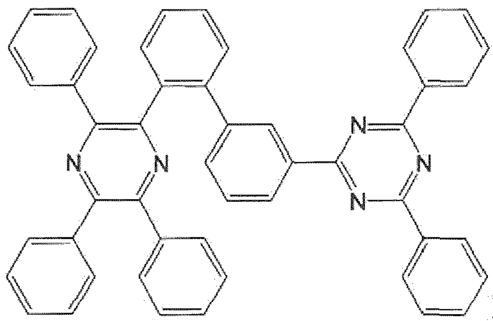

E-2

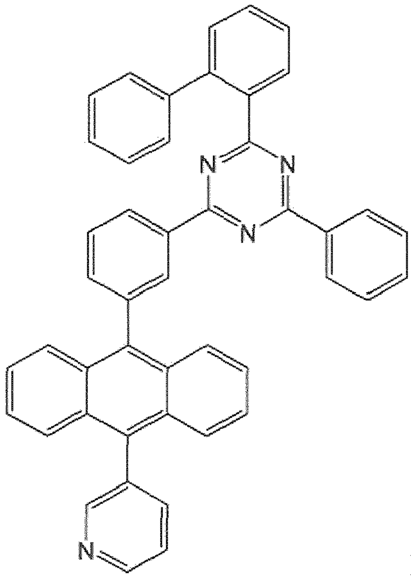

E-4

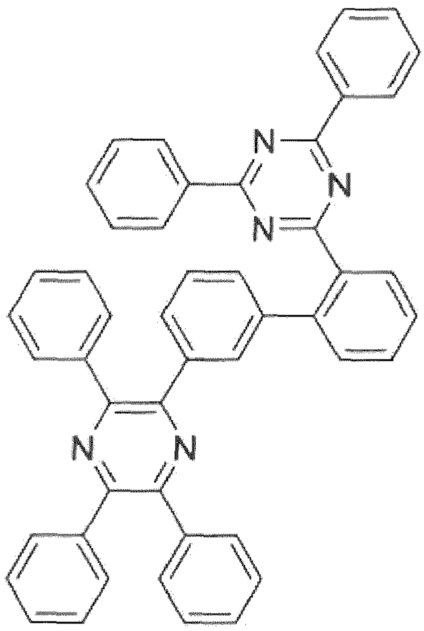

E-7

Electron transport layer

The electron transport layer is free of an electrical dopant, such as an n-type dopant, especially a redox n-type dopant. The term "free of" in this regard does not exclude impurities. Impurities have no technical effect with respect to the object achieved by the present invention. Impurities are not deliberately added to the layer during processing.

The term "free of' a compound means that such compound is not deliberately added to the layer during processing. Under electrical dopant, especially n-type dopant it is understood a compound which, if embedded into an electron transport matrix, improves, in comparison with the neat matrix under the same physical conditions, the electron properties of the formed organic material, particularly in terms of electron injection and/or electron conductivity.

In the context of the present invention "embedded into an electron transport matrix" means homogenously mixed with the electron transport matrix.

The electrical dopant as referred to herein is especially selected from elemental metals, metal salts, metal complexes and organic radicals.

In one embodiment, the electrical dopant is selected from alkali metal salts and alkali metal complexes; preferably from lithium salts and lithium organic complexes; more preferably from lithium halides and lithium organic chelates; even

more preferably from lithium fluoride, a lithium quinolinolate, lithium borate, lithium phenolate, lithium pyridinolate or from a lithium complex with a Schiff base ligand; most preferably,

- the lithium complex has the formula II, III or IV:

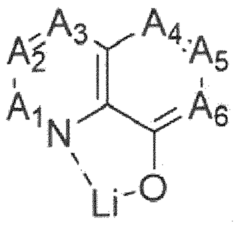 (II), 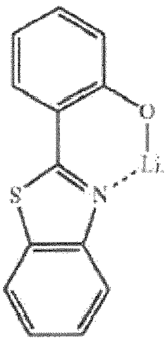 (III), 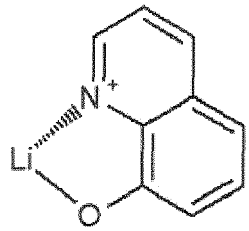 (IV)

  wherein

  $A_1$ to $A_6$ are same or independently selected from CH, CR, N, O;

  R is same or independently selected from hydrogen, halogen, alkyl or aryl or heteroaryl with 1 to 20 carbon atoms; and more preferred $A_1$ to $A_6$ are CH,

  - the borate based organic ligand is a tetra(1H-pyrazol-1-yl)borate,
  - the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
  - the pyridinolate is a 2-(diphenylphosphoryl)pyridin-3-olate,

- the lithium Schiff base has the structure 100, 101, 102 or 103:

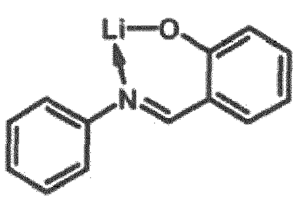 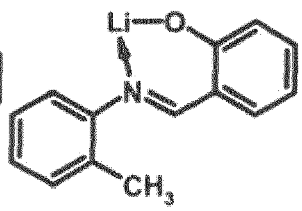 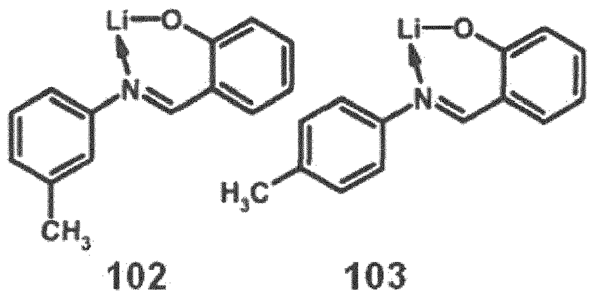

100 101 102 103

According to one embodiment of the invention, the electron transport layer of the present invention is free of a lithium organic complex, alternatively 8-Hydroxyquinolinolato-lithium (= LiQ).

According to one embodiment of the present invention the electron transport layer is free of a metal, preferably selected from alkali metals, alkaline earth metals, rare earth metals and metals of the first transition period Ti, V, Cr and Mn, especially selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

The most practical benchmark for the strength of an n-dopant is the value of its redox potential. There is no particular limitation in terms how negative the value of the redox potential can be.

As reduction potentials of usual electron transport matrices used in organic semiconductors are, if measured by cyclic voltammetry against ferrocene/ferrocenium reference redox couple, roughly in the range from about - 0.8 V to about - 3.1V; the practically applicable range of redox potentials for n-type dopants which can effectively n-dope such matrices is in a slightly broader range, from about - 0.5 to about - 3.3 V.

The measurement of redox potentials is practically performed for a corresponding redox couple consisting of

the reduced and of the oxidized form of the same compound.

In case that the n-type dopant is an electrically neutral metal complex and/or an electrically neutral organic radical, the measurement of its redox potential is actually performed for the redox couple formed by

(i) the electrically neutral metal complex and its cation radical formed by an abstraction of one electron from the electrically neutral metal complex, or

(ii) the electrically neutral organic radical and its cation formed by an abstraction of one electron from the electrically neutral organic radical.

Preferably, the redox potential of the electrically neutral metal complex and/or of the electrically neutral organic radical may have a value which is more negative than - 0.5 V, preferably more negative than - 1.2 V, more preferably more negative than - 1.7 V, even more preferably more negative than - 2.1 V, most preferably more negative than - 2.5 V, if measured by cyclic voltammetry against ferrocene/ferrocenium reference redox couple for a corresponding redox couple consisting of

(i) the electrically neutral metal complex and its cation radical formed by an abstraction of one electron from the electrically neutral metal complex, or

(ii) the electrically neutral organic radical and its cation formed by an abstraction of one electron from the electrically neutral organic radical.

In a preferred embodiment, the redox potential of the n-dopant is between the value which is about 0.5 V more positive and the value which is about 0.5 V more negative than the value of the reduction potential of the chosen electron transport matrix.

Electrically neutral metal complexes suitable as n-type dopants may be e.g. strongly reductive complexes of some transition metals in low oxidation state. Particularly strong n-type dopants may be selected for example from Cr(II), Mo(II) and/or W(II) guanidinate complexes such as $W_2(hpp)_4$, as described in more detail in WO2005/086251.

Electrically neutral organic radicals suitable as n-type dopants may be e.g. organic radicals created by supply of additional energy from their stable dimers, oligomers or polymers, as described in more detail in EP 1 837 926 B1, WO2007/107306, or WO2007/107356. Under an elemental metal, it is understood a metal in a state of a neat metal, of a metal alloy, or in a state of free atoms or metal clusters. It is understood that metals deposited by vacuum thermal evaporation from a metallic phase, e.g. from a neat bulk metal, vaporize in their elemental form. It is further understood that if the vaporized elemental metal is deposited together with a covalent matrix, the metal atoms and/or clusters are embedded in the covalent matrix. In other words, it is understood that any metal doped covalent material prepared by vacuum thermal evaporation contains the metal at least partially in its elemental form.

For the use in consumer electronics, only metals containing stable nuclides or nuclides having very long halftime of radioactive decay might be applicable. As an acceptable level of nuclear stability, the nuclear stability of natural potassium can be taken.

In one embodiment, the electrical may be selected from electropositive metals selected from alkali metals, alkaline earth metals, rare earth metals and metals of the first transition period Ti, V, Cr and Mn. Preferably, the n-dopant may be selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

Organic light emitting diode

The organic light emitting layer may further comprise a hole blocking layer, wherein the hole blocking layer is arranged between the emission layer and the electron transport layer. The hole blocking layer may also be referred to as auxiliary electron transport layer (a-ETL).

The hole blocking layer may be in direct contact with the electron transport layer. The hole blocking layer may be in direct contact with the emission layer. The hole blocking layer may be contacting sandwiched between the emission layer and the electron transport layer.

The organic light emitting diode may further comprise an electron injection layer, wherein the electron injection layer is arranged between the cathode and the electron transport layer, and the electron injection layer may be in direct contact with the electron transport layer. The electron injection layer may be in direct contact with the cathode. The electron injection layer may be contacting sandwiched between the electron transport layer and the cathode.

It may be provided that the electron injection layer does not comprise the compound of formula (I). It may be provided that the electron injection layer does not comprise the compound of formula (II). It may be provided that the

electron injection layer does not comprise the compound of formula (III). It may be provided that the electron injection layer does not comprise any of the compounds of formulas (I) to (III).

The electron injection layer may comprise a first electron injection sub-layer and a second electron injection sub-layer, wherein the first electron injection sub-layer and the second electron injection sub-layer are in direct contact with each other.

The first electron injection sub-layer may be in direct contact with the electron transport layer and the first electron injection sub-layer may comprise a metal salt or a metal complex, preferably a lithium salt or a lithium organic complex; more preferably a compound selected from lithium halides and lithium organic chelates; even more preferably from lithium fluoride, a lithium quinolinolate, lithium borate, lithium phenolate, lithium pyridinolate or from a lithium complex with a Schiff base ligand; most preferably,

- the lithium complex has the formula II, III or IV:

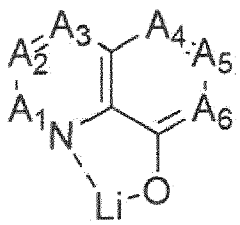 (II), 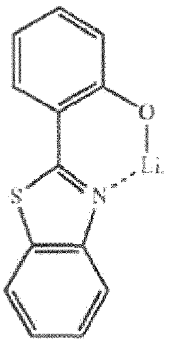 (III), 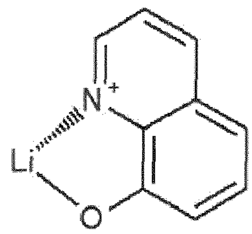 (IV)

wherein

A$_1$ to A$_6$ are same or independently selected from CH, CR, N, O;

R is same or independently selected from hydrogen, halogen, alkyl or aryl or heteroaryl with 1 to 20 carbon atoms; and more preferred A$_1$ to A$_6$ are CH,

- the borate based organic ligand is a tetra(1H-pyrazol-1-yl)borate,
- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
- the pyridinolate is a 2-(diphenylphosphoryl)pyridin-3-olate,

- the lithium Schiff base has the structure 100, 101, 102 or 103:

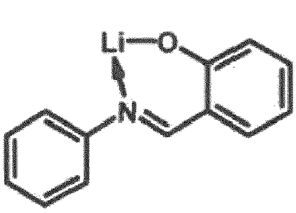 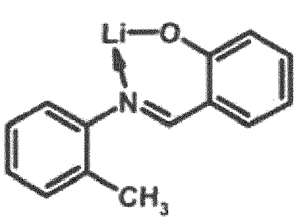 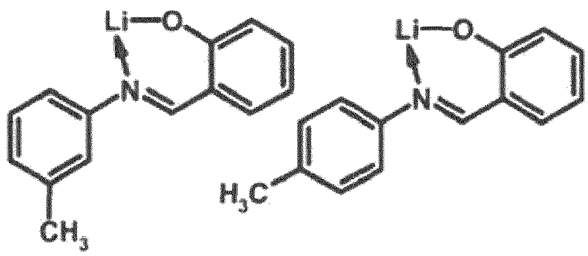

100 101 102 103

Preferably, the first electron injection sub-layer comprises 8-Hydroxyquinolinolato-lithium (= LiQ).

The second electron injection sub-layer comprises a metal selected form the group consisting of alkali metals, alkaline earth metals, and rare earth metals, preferably the metal may be selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb most preferred Yb.

The second electron injection sub-layer may be in direct contact with the cathode.

In accordance with the invention, the organic light emitting diode may comprise, besides the layers already mentioned above, further layers. Exemplary embodiments of respective layers are described in the following:

*Substrate*

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

*Anode electrode*

Either a first electrode or a second electrode comprised in the inventive organic electronic device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide ($SnO_2$), aluminum zinc oxide (AlZO) and zinc oxide (ZnO), may be used to form the anode electrode. The anode electrode may also be formed using metals, typically silver (Ag), gold (Au), or metal alloys.

*Hole injection layer*

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10-8 to 10-3 Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluorotetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluorotetracyanoquinonedimethane (F4TCNQ), which LUMO level is about -5.2 eV; zinc phthalocyanine (ZnPc) (HOMO = -5.2 eV) doped with F4TCNQ; $\alpha$-NPD (N,N'-Bis(naphthalen-i-yl)-N,N'-bis(phenyl)-benzidine) doped with F4TCNQ. $\alpha$-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem, Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Emission layer (EML)*

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of Formula (1).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

*Hole blocking layer (HBL)*

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function. The hole blocking layer may be the inventive organic semiconductor layer comprising or consisting of the inventive compound represented by the general Formula (1) as defined above.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, and phenanthroline derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

The hole blocking layer may also be described as a-ETL or auxiliary ETL.

According to an embodiment, a hole blocking layer is arranged between the at least one emission layer and the electron transport layer comprising the compound of formula (I) and the compound of formula (II) and/or the compound of formula (III).

*Electron transport layer (ETL)*

The OLED according to the present invention comprises one or more electron transport layer(s) (ETL). In accordance with the invention, at least one of the electron transport layers is the inventive electron transport layer comprising the compound of formula (I) and the compound of formula (II) and/or the compound of formula (III) as defined herein.

According to various embodiments the OLED may comprise an electron transport layer or an electron transport layer stack comprising at least a first electron transport layer and at least a second electron transport layer.

By suitably adjusting energy levels of particular layers of the ETL, the injection and transport of the electrons may be controlled, and the holes may be efficiently blocked. Thus, the OLED may have long lifetime.

The electron transport layer may comprise, besides the compound of formula (I) and the compound of formula (II) and/or the compound of formula (III) further ETM materials known in the art. Likewise, the electron transport layer may comprise as the only electron transport matrix material the compound of formula (I) and the compound of formula (II) and/or the compound of formula (III). In case that the inventive organic electronic device comprises more than one electron transport layers, the compound of formula (I) and the compound of formula (II) and/or the compound of formula (III) may be comprised in only one of the electron transport layers, in more than one of the electron transport layers or in all of the electron transport layers.

*Electron injection layer (EIL)*

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, $Li_2O$, BaO, Li, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

*Charge generation layer (CGL)*

The charge generation layer (CGL) may comprise a p- type charge generation layer (p-CGL) and an n-type charge generation layer (n-CGL). An interlayer may be arranged between the p-CGL and the -n-CGL.

Typically, the charge generation layer is a pn junction joining an n-type charge generation layer (electron generating layer) and a hole generating layer. The n-side of the pn junction generates electrons and injects them into the layer which is adjacent in the direction to the anode. Analogously, the p-side of the p-n junction generates holes and injects them into the layer which is adjacent in the direction to the cathode.

Charge generating layers are used in tandem and stacked devices, for example, in tandem or stacked OLEDs comprising, between two electrodes, two or more emission layers. In a tandem or stacked OLED comprising two emission layers, the n-type charge generation layer provides electrons for the first light emission layer arranged near the anode, while the hole generating layer provides holes to the second light emission layer arranged between the first emission layer and the cathode.

Suitable matrix materials for the hole generating layer may be materials conventionally used as hole injection and/or hole transport matrix materials. Also, p-type dopant used for the hole generating layer can employ conventional materials. For example, the p-type dopant can be one selected from a group consisting of tetrafluore-7,7,8,8-tetracyanoquinodimethane (F4-TCNQ), derivatives of tetracyanoquinodimethane, radialene derivatives, iodine, $FeCl_3$, $FeF_3$, and $SbCl_5$. Also, the host can be one selected from a group consisting of N,N'-di(naphthalen-1-yl)-N,N-diphenyl-benzidine (NPB), N,N'-diphenyl-N,N'-bis(3-methylphenyl)-1,1-biphenyl-4,4'-diamine (TPD) and N,N',N'-tetranaphthyl-benzidine (TNB). The p-type charge generation layer may consist of CNHAT.

The n-type charge generating layer may be the layer comprising the compound of Formula (I). The n-type charge generation layer can be layer of a neat n-type dopant, for example of a metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Li, Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds, hydroxyquinoline derivatives like tris(8-hydroxyquinoline)aluminum, benzazole derivatives, and silole derivatives.

The hole generating layer is arranged in direct contact to the n-type charge generation layer.

According to one aspect of the present invention, the electron transport layer is arranged between the first and second emission layer.

According to one aspect of the present invention, the electron transport layer comprising the compound of formula (I) and the compound of formula (II) and/or the compound of formula (III) is arranged between the first and second emission layer and an electron transport layer comprising the compound of formula (I) and the compound of formula (II) and/or the compound of formula (III) is arranged between the second emission layer and the cathode.

According to one aspect of the present invention, there is provided an organic light-emitting diode (OLED) comprising: a substrate; an anode electrode formed on the substrate; a hole injection layer, a hole transport layer, an emission layer, an electron transport layer comprising the compound of formula (I) and the compound of formula (II) and/or the compound of formula (III) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an electron transport layer comprising the compound of formula (I) and the compound of formula (II) and/or the compound of formula (III) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an electron transport layer comprising the compound of formula (I) and the compound of formula (II) and/or the compound of formula (III), an electron injection layer, and a cathode electrode.

According to various embodiments of the present invention, there may be provided OLEDs layers arranged between the above mentioned layers, on the substrate or on the top electrode.

According to one aspect, the OLED can comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

For example, the OLED according to Fig. 2 may be formed by a process, wherein on a substrate (110), an anode (120), a hole injection layer (130), a hole transport layer (140), an electron blocking layer (145), an emission layer (150), a hole blocking layer (155), an electron transport layer (160), an electron injection layer (180) and the cathode electrode (190) are subsequently formed in that order.

According to another aspect of the invention, it is provided an electronic device comprising at least one organic

light emitting device according to any embodiment described throughout this application, preferably, the electronic device comprises the organic light emitting diode in one of embodiments described throughout this application. More preferably, the electronic device is a display device or a lighting device, most preferably a display device.

In one embodiment, the organic light emitting diode according to the invention further comprises a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb):

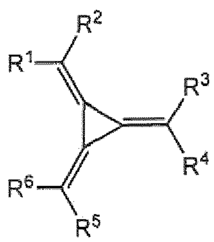 (XX) 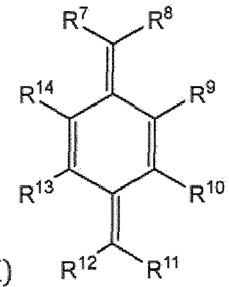 (XXIa) 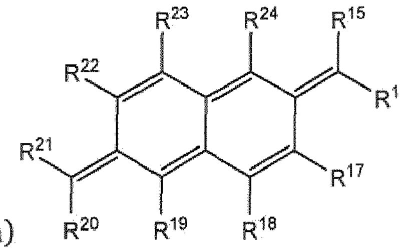 (XXIb),

wherein (as an exception different to the description above) $R^1$, $R^2$, $R^3$, $R^4$, $R^5$, $R^6$, $R^7$, $R^8$, $R^{11}$, $R^{12}$, $R^{15}$, $R^{16}$, $R^{20}$, $R^{21}$ are independently selected from above mentioned electron withdrawing groups and $R^9$, $R^{10}$, $R^{13}$, $R^{14}$, $R^{17}$, $R^{18}$, $R^{19}$, $R^{22}$, $R^{23}$ and $R^{24}$ are independently selected from H, halogen and above mentioned electron withdrawing groups.

According to one embodiment of the present invention, the organic semiconductor layer comprising compound of Formula (1) is adjacent to a layer comprising a compound of formula (XX), (XXIa) or (XXIb).

According to one embodiment of the present invention, the organic semiconductor layer comprising compound of Formula (1) is in direct contact to a layer comprising a compound of formula (XX), (XXIa) or (XXIb).

## Display device

The present invention furthermore relates to a display device comprising an organic electronic device according to the present invention.

## Method for manufacturing the organic electronic device

According to another aspect of the present invention, there is provided a method of manufacturing an organic light emitting diode, the method using:

- at least one deposition source, preferably two deposition sources and more preferred at least three deposition sources.

The methods for deposition that can be suitable comprise:

- deposition via vacuum thermal evaporation;

- deposition via solution processing, preferably the processing is selected from spin-coating, printing, casting; and/or

- slot-die coating.

According to various embodiments of the present invention, there is provided a method using:

- a first deposition source to release the compound of Formula (I) according to the invention, and
- a second deposition source to release the the compound of formula (II) and/or the compound of formula (III);

the method comprising the steps of forming the organic semiconductor layer; whereby for an organic light-emitting diode (OLED):

- the electron transport layer is formed by releasing the compound of formula (I) according to the invention from the first deposition source and the compound of formula (II) and/or the compound of formula (III) from the second deposition source.

According to various embodiments of the present invention, the method may further include forming on the anode electrode, an emission layer and at least one layer selected from the group consisting of forming a hole injection layer, forming a hole transport layer, or forming a hole blocking layer, between the anode electrode and the first electron transport layer.

According to various embodiments of the present invention, the method may further include the steps for forming an organic light-emitting diode (OLED), wherein

- on a substrate a first anode electrode is formed,
- on the first anode electrode an emission layer is formed,
- on the emission layer an electron transport layer stack is formed, optionally a hole blocking layer is formed on the emission layer and an organic semiconductor layer is formed,
- and finally a cathode electrode is formed,
- optional a hole injection layer, a hole transport layer, and a hole blocking layer, formed in that order between the first anode electrode and the emission layer,
- the electron transport layer is formed between the emission layer and the cathode elcectrode;
- optional an electron injection layer is formed between the organic semiconductor layer and the cathode electrode.

According to various embodiments of the present invention, the method may further comprise forming an electron injection layer on the electron transport layer. However, according to various embodiments of the OLED of the present invention, the OLED may not comprise an electron injection layer.

According to various embodiments, the OLED may have the following layer structure, wherein the layers having the following order:

Anode, hole injection layer, first hole transport layer, second hole transport layer, emission layer, optional hole blocking layer, electron transport layer, and cathode.

GENERAL DEFINITIONS

In the present specification, when a definition is not otherwise provided, an "alkyl group" may refer to an aliphatic hydrocarbon group. The alkyl group may refer to "a saturated alkyl group" without any double bond or triple bond. The term "alkyl" as used herein shall encompass linear as well as branched and cyclic alkyl. For example, $C_3$-alkyl may be selected from n-propyl and iso-propyl. Likewise, $C_4$-alkyl encompasses n-butyl, sec-butyl and t-butyl. Likewise, $C_6$-alkyl encompasses n-hexyl and cyclo-hexyl.

As used herein if not explicitly mentioned else, the asterisk symbol "*" represents a binding position at which the moiety labelled accordingly is bond to another moiety.

The subscribed number n in $C_n$ relates to the total number of carbon atoms in the respective alkyl, arylene, heteroarylene or aryl group.

The term "aryl" or "arylene" as used herein shall encompass phenyl ($C_6$-aryl), fused aromatics, such as naphthalene, anthracene, phenanthrene, tetracene etc.. Further encompassed are biphenyl and oligo- or polyphenyls, such as terphenyl, phenyl-substituted biphenyl, phenyl-substituted terphenyl (such as tetraphenyl benzole groups) etc.. "Arylene" respectively "heteroarylene", refers to groups to which two further moieties are attached. In the present specification, the term "aryl group" or "arylene group" may refer to a group comprising at least one hydrocarbon aromatic moiety, and all the elements of the hydrocarbon aromatic moiety may have p-orbitals which form conjugation, for example a phenyl group, a napthyl group, an anthracenyl group, a phenanthrenyl group, a pyrinyl group, a fluorenyl group and the like. Further encompoassed are spiro compounds in which two aromatic moieties are connected with each other via a spiro-atom, such as 9,9'-spirobi[9H-fluorene]yl. The aryl or arylene group may include a monocyclic or fused ring polycyclic (i.e., links sharing adjacent pairs of carbon atoms) functional group.

The term "heteroaryl" as used herein refers to aryl groups in which at least one carbon atom is substituted with a heteroatom. The term "heteroaryl" may refer to aromatic heterocycles with at least one heteroatom, and all the elements of the hydrocarbon heteroaromatic moiety may have p-orbitals which form conjugation. The heteroatom may be selected from N, O, S, B, Si, P, Se, preferably from N, O and S. A heteroarylene ring may comprise at least 1 to 3 heteroatoms. Preferably, a heteroarylene ring may comprise at least 1 to 3 heteroatoms individually selected from N, S and/or O. Just as in case of "aryl"/"arylene", the term "heteroaryl" comprises, for example, spiro compounds in which two aromatic moieties are connected with each other, such as spiro[fluorene-9,9'-xanthene]. Further exemplary heteroaryl groups are diazine, triazine, dibenzofurane, dibenzothiofurane, acridine, benzoacridine, dibenzoacridine etc.

The term "alkenyl" as used herein refers to a group $-CR^1 = CR^2R^3$ comprising a carbon-carbon double bond.

The term "perhalogenated" as used herein refers to a hydrocarbyl group wherein all of the hydrogen atoms of the hydrocarbyl group are replaced by halogen (F, Cl, Br, I) atoms.

The term "alkoxy" as used herein refers to a structural fragment of the Formula -OR with R being hydrocarbyl, preferably alkyl or cycloalkyl.

The term "thioalkyl" as used herein refers to a structural fragment of the Formula -SR with R being hydrocarbyl, preferably alkyl or cycloalkyl.

The subscripted number n in $C_n$-heteroaryl merely refers to the number of carbon atoms excluding the number of heteroatoms. In this context, it is clear that a $C_3$ heteroarylene group is an aromatic compound comprising three carbon atoms, such as pyrazol, imidazole, oxazole, thiazole and the like.

The term "heteroaryl" as used herewith shall encompass pyridine, quinoline, benzoquinoline, quinazoline, benzoquinazoline, pyrimidine, pyrazine, triazine, benzimidazole, benzothiazole, benzo[4,5]thieno[3,2-d]pyrimidine, carbazole, xanthene, phenoxazine, benzoacridine, dibenzoacridine and the like.

In the present specification, the term single bond refers to a direct bond.

The term "fluorinated" as used herein refers to a hydrocarbon group in which at least one of the hydrogen atoms comprised in the hydrocarbon group is substituted by a fluorine atom. Fluorinated groups in which all of the hydrogen atoms thereof are substituted by fluorine atoms are referred to as perfluorinated groups and are particularly addressed by the term "fluorinated".

In terms of the invention, a group is "substituted with" another group if one of the hydrogen atoms comprised in this group is replaced by another group, wherein the other group is the substituent.

In accordance with the present disclosure, in a formula showing the following binding situation,

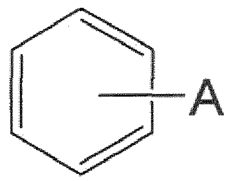

the group A may be bound to any suitable binding position. In a situation were it is shown that the bond of A crosses more than one ring

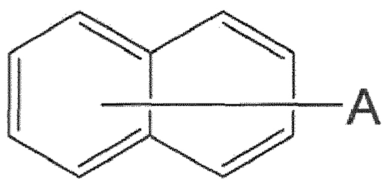

the group A may be bound to any suitable binding position of each ring crossed with the bond.

In terms of the invention, the expression "between" with respect to one layer being between two other layers

does not exclude the presence of further layers which may be arranged between the one layer and one of the two other layers. In terms of the invention, the expression "in direct contact" with respect to two layers being in direct contact with each other means that no further layer is arranged between those two layers. One layer deposited on the top of another layer is deemed to be in direct contact with this layer.

The term "contacting sandwiched" refers to an arrangement of three layers whereby the layer in the middle is in direct contact with the two adjacent layers.

With respect to the inventive electron transport layer stack the compounds mentioned in the experimental part are most preferred.

A lighting device may be any of the devices used for illumination, irradiation, signaling, or projection. They are correspondingly classified as illuminating, irradiating, signaling, and projecting devices. A lighting device usually consists of a source of optical radiation, a device that transmits the radiant flux into space in the desired direction, and a housing that joins the parts into a single device and protects the radiation source and light-transmitting system against damage and the effects of the surroundings.

According to another aspect, the organic electroluminescent device according to the present invention comprises two or three or more emission layers. An OLED comprising more than one emission layer is also described as a tandem OLED or stacked OLED.

The organic electroluminescent device (OLED) may be a bottom- or top-emission device. The organic electroluminescent device (OLED) may emit the light trough a transparent anode or through a transparent cathode.

Another aspect is directed to a device comprising at least one organic electroluminescent device (OLED).

A device comprising organic light-emitting diodes is for example a display or a lighting panel.

In the present invention, the following defined terms, these definitions shall be applied, unless a different definition is given in the claims or elsewhere in this specification.

In the context of the present specification the term "different" or "differs" in connection with the matrix material means that the matrix material differs in their structural Formula.

The terms "OLED" and "organic light-emitting diode" are simultaneously used and have the same meaning. The term "organic electroluminescent device" as used herein may comprise both organic light emitting diodes as well as organic light emitting transistors (OLETs).

As used herein, "weight percent", "wt.-%", "percent by weight", "% by weight", and variations thereof refer to a composition, component, substance or agent as the weight of that component, substance or agent of the respective electron transport layer divided by the total weight of the respective electron transport layer thereof and multiplied by 100. It is under-stood that the total weight percent amount of all components, substances and agents of the respective electron transport layer and electron injection layer are selected such that it does not exceed 100 wt.-%.

As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%,

All numeric values are herein assumed to be modified by the term "about", whether or not explicitly indicated. As used herein, the term "about" refers to variation in the numerical quantity that can occur. Whether or not modified by the term "about" the claims include equivalents to the quantities.

It should be noted that, as used in this specification and the appended claims, the singular forms "a", "an", and "the" include plural referents unless the content clearly dictates otherwise.

The term "free of', "does not contain", "does not comprise" does not exclude impurities. Impurities have no technical effect with respect to the object achieved by the present invention.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

Preferably, the organic semiconducting layer comprising the compound of Formula (1) is essentially non-emissive or non-emitting.

The operating voltage, also named U, is measured in Volt (V) at 10 milliAmpere per square centimeter (mA/cm2).

The candela per Ampere efficiency, also named cd/A efficiency is measured in candela per ampere at 10 milliAmpere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color. Efficiency values are compared at the same CIE-y.

The highest occupied molecular orbital, also named HOMO, and lowest unoccupied molecular orbital, also

named LUMO, are measured in electron volt (eV).

The term "OLED", "organic light emitting diode", "organic light emitting device", "organic optoelectronic device" and "organic light-emitting diode" are simultaneously used and have the same meaning.

The term "life-span" and "lifetime" are simultaneously used and have the same meaning.

The anode and cathode may be described as anode electrode / cathode electrode or anode electrode / cathode electrode or anode electrode layer / cathode electrode layer.

Room temperature, also named ambient temperature, is 23° C.

Hereinafter, the embodiments are illustrated in more detail with reference to examples. However, the present disclosure is not limited to the following examples. Reference will now be made in detail to the exemplary aspects.

DESCRIPTION OF THE DRAWINGS

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

FIG. 1 is a schematic sectional view of an organic electronic device, according to an exemplary embodiment of the present invention;

FIG. 2 is a schematic sectional view of an organic light-emitting diode (OLED), according to an exemplary embodiment of the present invention;

FIG. 3 is a schematic sectional view of an OLED, according to an exemplary embodiment of the present invention.

FIG. 4 is a schematic sectional view of an OLED comprising a charge generation layer and two emission layers, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic electronic device 100, according to an exemplary embodiment of the present invention. The organic electronic device 100 includes a substrate 110, an anode 120, an emission layer (EML) 125, an electron transport layer comprising the compound of formula (I) and the compound of formula (II) and/or the compound of formula (III) 160. The electron transport layer comprising the compound of formula (I) and the compound of formula (II) and/or the compound of formula (III) 160 is formed on the EML 125. Onto the electron transport layer comprising the compound of formula (I) and the compound of formula (II) and/or the compound of formula (III) 160, a cathode 190 is disposed.

FIG. 2 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an emission layer (EML) 150, an electron transport layer (ETL) 160. The electron transport layer (ETL) 160 is formed on the EML 150. Onto the electron transport layer (ETL) 160, an electron injection layer (EIL) 180 is disposed. The cathode 190 is disposed directly onto the electron injection layer (EIL) 180.

Fig. 3 is a schematic sectional view of an OLED 100, according to another exemplary embodiment of the present invention. Fig. 3 differs from Fig. 2 in that the OLED 100 of Fig. 3 comprises an electron blocking layer (EBL) 145 and a hole blocking layer (HBL) 155.

Referring to Fig. 3, the OLED 100 includes a substrate 110, an anode 120, a hole injection layer (HIL) 130, a hole transport layer (HTL) 140, an electron blocking layer (EBL) 145, an emission layer (EML) 150, a hole blocking layer (HBL) 155, electron transport layer comprising the compound of formula (I) and the compound of formula (II) and/or the

compound of formula (III) 160, an electron injection layer (EIL) 180 and a cathode electrode 190.

Fig. 4 is a schematic sectional view of an OLED 100, according to another exemplary embodiment of the present invention. Fig. 4 differs from Fig. 3 in that the OLED 100 of Fig. 4 further comprises a charge generation layer (CGL) and a second emission layer (151).

Referring to Fig. 4, the OLED 100 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer comprising the compound of formula (I) and the compound of formula (II) and/or the compound of formula (III)160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a second hole blocking layer (EBL) 156, a second electron transport layer comprising the compound of formula (I) and the compound of formula (II) and/or the compound of formula (III) 161, a second electron injection layer (EIL) 181 and a cathode 190.

While not shown in Fig. 1, Fig. 2, Fig. 3 and Fig. 4, a sealing layer may further be formed on the cathode electrodes 190, in order to seal the OLEDs 100. In addition, various other modifications may be applied thereto.

Hereinafter, the embodiments are illustrated in more detail with reference to examples. However, the present disclosure is not limited to the following examples.

DETAILED DESCRIPTION

The invention is furthermore illustrated by the following examples which are illustrative only and non-binding.

EXPERIMENTAL PART

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

Rate onset temperature

The rate onset temperature ($T_{RO}$) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than $10^{-5}$ mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

Reduction potential

The reduction potential is determined by cyclic voltammetry with potenioststic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard $Fc^+/Fc$ redox couple, afforded finally the values reported above. All studied compounds as well as the reported comparative compounds showed well-defined reversible electrochemical behavior.

Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment is determined by a semi-empirical molecular orbital method.

The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

Refractive index measurement of thin films

Neat films of host materials with a thickness of 70 nm on silicon substrates (0.5 nm native $SiO_2$, 675 µm thickness, Siegert Wafer GmbH) are prepared by thermal evaporation in a vacuum system (Cluster Tool, Sunic System Ltd.) at a deposition rate of 1 Å/s and a pressure of approximately 3e-7 mbar. The samples are stored in glovebox with pure nitrogen atmosphere until the measurement takes place (maximum 1 hour of air exposure). Reflectance is measured using a Filmetrics F10-RT Spectrometer with a spectral range of 380 nm to 1050 nm. A silicon reference sample from Filmetrics is used for reflectance standard. Measured reflectance data is then modelled using the Cauchy model in the FILMeasure software, which results in the refractive index in the range of 420 nm to 1020 nm.

Table 1.

| Material | Ref. Index at 633 nm (neat layer, Reflectometer) | Ref. Index at 633 nm (co-deposited with 20% ET3, Reflectometer) | Tg / °C | Tm / °C | Rate Onset Temperature / °C | DFT LUMO / eV | DFT HOMO / eV | DFT dipole / Debye |
|---|---|---|---|---|---|---|---|---|
| E-1 | 1.76 | 1.76 | 137 | - | 248 | -1.79 | -5.1 | 0.31 |
| E-2 | 1.73 | 1.74 | 116 | 233 | 209 | -1.76 | -5.64 | 0.26 |
| E-3 | 1.78 | 1.78 | 133 | 284 | 246 | -1.86 | -5.65 | 1.61 |
| E-4 | 1.76 | 1.76 | 124 | - | 228 | -1.82 | -5.23 | 2.28 |
| E-5 | 1.76 | 1.76 | 113 | - | 226 | -1.78 | -5.79 | 0.25 |
| E-6 | 1.76 | 1.76 | 121 | 263 | 219 | -1.77 | -5.11 | 0.33 |
| E-7 | 1.74 | 1.75 | 114 | 254 | 218 | -1.79 | -5.54 | 0.39 |
| E-8 | 1.72 | 1.70 | 127 | - | 223 | -1.69 | -5.82 | 0.50 |

## Synthesis Procedures

Compound E-1

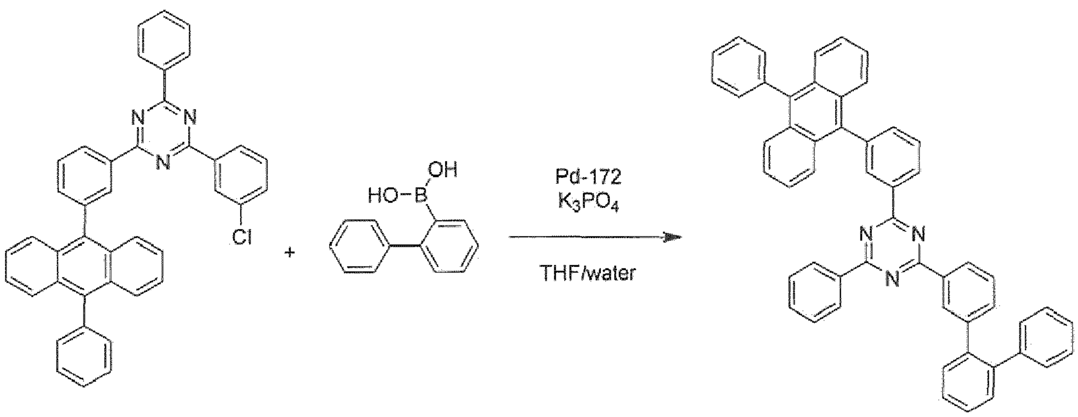


2-(3-chlorophenyl)-4-phenyl-6-(3-(10-phenylanthracen-9-yl)phenyl)-1,3,5-triazine (30.0 g, 50.3 mmol) and biphenyl-2-boronic acid (12.0 g, 60.4 mmol) were suspended in tetrahydrofuran (200 ml) in a Schlenk flask. Potassium phosphate (21.2 g, 100 mmol) was added as a solution in water (50 ml) and the mixture was purged with N2. Chloro(crotyl)(2-dicyclohexylphosphino-2',6'-dimethoxybiphenyl)palladium(II) (611 mg, 1.00 mmol) was added under a stream of $N_2$ and the mixture was heated to 52 °C for 60 h. Further portions of biphenyl-2-boronic acid (10 g, 50.5 mmol)

and chloro(crotyl)(2-dicyclohexylphosphino-2',6'-dimethoxybiphenyl)palladium(II) (600 mg, 988 mg) were added and the mixture heated at 52 °C for a further 16 h. The solvent was subsequently removed under reduced pressure and the residue taken up in dichloromethane and water. After filtration, the phases were separated. The organic phase was washed again with water, dried with $MgSO_4$ and filtered through a pad of silica gel. The filtrate was concentrated to 100 ml under reduced pressure and 500 ml cyclohexane was added. A further 200 ml of solvent was removed under reduced pressure, leading to precipitation of a pale yellow solid. The solid was recrystallized from hot toluene, separated by filtration, dissolved in dichloromethane and precipitated again with n-hexane, and washed with n-hexane to provide the pure product (17.6 g, 49%).

$$\text{ESI-MS} - m/z = 714\ ([M]^+)$$

Compound E-2

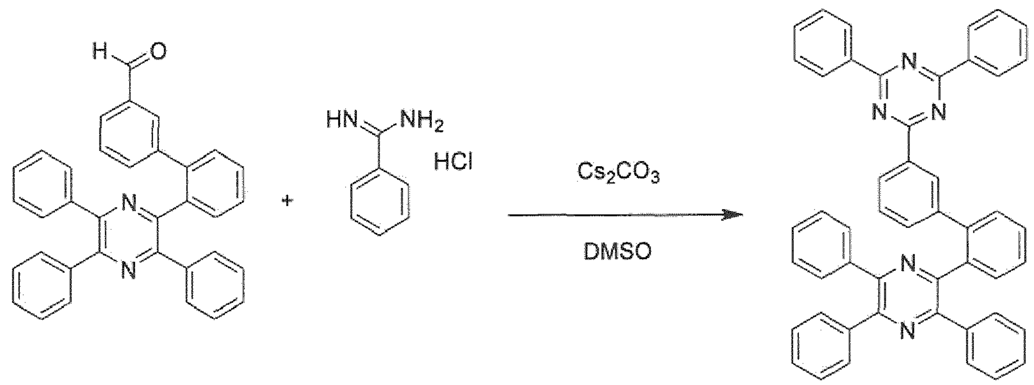


2'-(3,5,6-triphenylpyrazin-2-yl)-[1,1'-biphenyl]-3-carbaldehyde (46.0 g, 94.2 mmol), benzimidamide hydrochloride (29.5 g, 118.3 mmol) and caesium carbonate (61.4 g, 118.3 mmol) were placed in a Schlenk flask under N2 and suspended in dimethyl sulfoxide (300 ml). The mixture was heated to 90 °C for 20 h. After cooling to room temperature, the off-white precipitate was collected by filtration, washed with water and ethanol. The solid was then dissolved in dichloromethane (200 ml) and precipitated by the addition of methanol (300 ml). Filtration and drying at 100 °C under reduced pressure yielded the desired product (24.5 g, 38%).

$$\text{ESI-MS} - m/z = 692\ ([M]^+)$$

Compound E-4

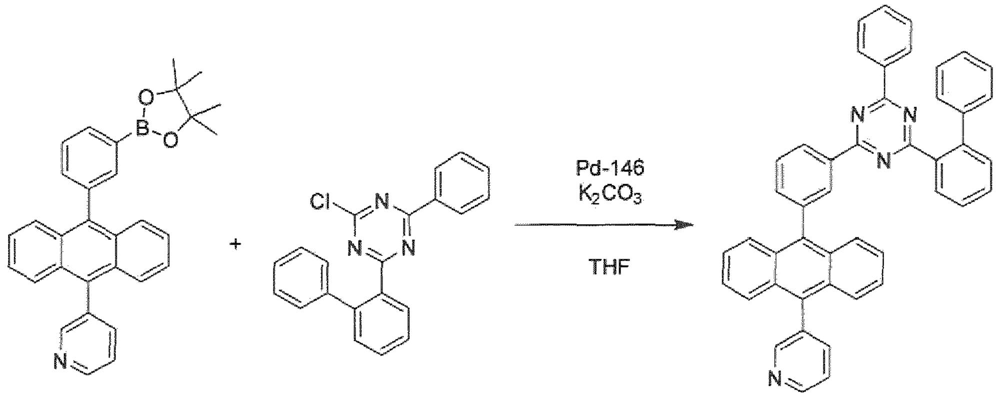

3-(10-bromoanthracen-9-yl)pyridine (14.6 g, 43.7 mmol), 2-([1,1'-biphenyl]-2-yl)-4-phenyl-6-(3-(4,4,5,5-tetramethyl-1,3,2-dioxaborolan-2-yl)phenyl)-1,3,5-triazine (29.1 g, 56.8 mmol) and [1,1'-Bis(diphenylphosphino)ferrocene]-dichloropalladium(II) (159 mg, 218 mmol) were dissolved in tetrahydrofuran (180 ml) in a Schlenk flask and the solution was purged with N2. Potassium carbonate (12.1 g, 87.4 mmol) was then dissolved in water (43 ml) and added. The mixture was heated to 55 C for 16 h. Water (250 ml) was subsequently added to the reaction mixture, the phases separated, and the organic phase evaporated to dryness under reduced pressure. The residue was dissolved in chlorobenzene at 60 °C and filtered through a pad of silica gel. Concentration of the solution to 150 ml and addition of n-hexane led to precipitation of a solid, which was washed with n-hexane and methanol. Recrystallisation from ethyl acetate/hexane afforded the pure product (12.0 g, 43%).

ESI-MS – m/z = 639 ([M]$^+$)

Compound E-7

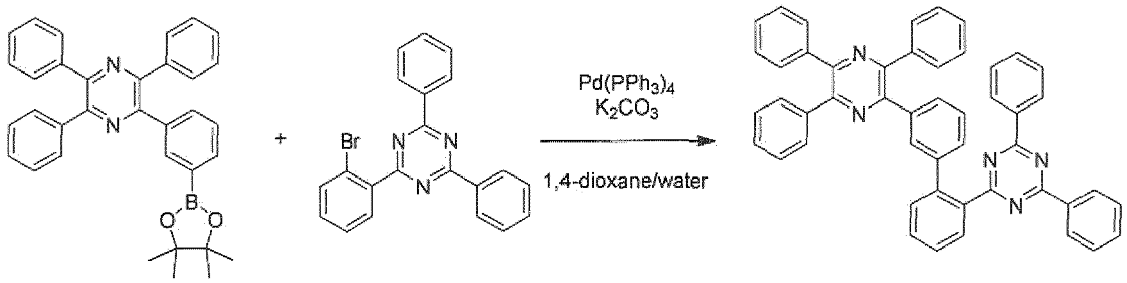


Potassium carbonate (16.0 g, 115 mmol) was placed in a Schlenk flask with 1,4-dioxane (230 ml) and water (58 ml), and the resulting mixture was purged with $N_2$. Subsequently, 2,3,5-triphenyl-6-(3-(4,4,5,5-tetrametbyl-1,3,2-dioxaborolan-2-yl)phenyl)pyrazine (21.3 g, 41.7 mmol), 2-(2-bromophenyl)-4,6-diphenyl-1,3,5-triazine (18.0 g, 46.4 mmol) and tetrakis(triphenylphosphine)-palladium(o) (1.07 g, 0.93 mmol) were added under a flow of $N_2$ and the mixture was heated to reflux for 60 h. Further portions of 2,3,5-triphenyl-6-(3-(4,4,5,5-tetramethyl-1,3,2-dioxaborolan-2-yl)phenyl)pyrazine (2.40 g, 4.17 mmol) and tetrakis(triphenylphosphine)palladium(o) (300 mg, 0.259 mmol) were added, and the mixture refluxed for a further 16 h. The resulting precipitate was separated by precipitation and washed with 1,4-dioxane and water. The solid was then dissolved in $CHCl_3$, dried over $MgSO_4$ and filtered through a pad of silica. The solution was concentrated to 500 ml under reduced pressure and acetonitrile was added (100 ml) to precipitate the product as a colourless solid, which was washed with acetonitrile and hexane, and dried under vacuum (25.4 g, 78%).

ESI-MS - m/z = 692 ([M]$^+$)

## General procedure for fabrication of OLEDs

For the top emission OLED devices a substrate with dimensions of 150 mm x 150 mm x 0.7 mm was ultrasonically cleaned with a 2% aqueous solution of Deconex FPD 211 for 7 minutes and then with pure water for 5 minutes, and dried for 15 minutes in a spin rinse dryer. Subsequently, Ag was deposited as anode at a pressure of $10^{-5}$ to $10^{-7}$ mbar.

Then, HT-1 and D-1 were vacuum co-deposited on the anode to form a HIL. Then, HT-1 was vacuum deposited on the HIL to form an HTL. Then, HT-2 was vacuum deposited on the HTL to form an electron blocking layer (EBL).

Afterwards the emission layer was formed on the EBL by co-deposition of HOST-1 and EMITTER-1.

Then, ET-1 was vacuum deposited onto the emission layer to form the HBL.

Then, the electron transport layer was formed on the HBL by co-depositing a mix of ET-3 with ET-5 for the comparative examples -1 and -2. The electron transport layer was formed on the HBL by co-depositing a mix of ET-3 with a compound of Formula (II) or formula (III) for inventive OLED examples OLED-1 to OLED-5. The electron transport layer was formed on the HBL by co-depositing a mix of ET-4 with a compound of Formula (II) or formula (III) for inventive OLED examples OLED-12 and OLED-13. The mixing ratios are given in table 3.

Then, the electron injection layer is formed as a double layer on the electron transport layer by depositing as the first EIL LiQ (for the comparative example-i and for OLED-1 to OLED-5) or a mix of ET-2:Li [99:1 vol%] (for OLED-6 to OLED-13) and subsequently Yb as the second EIL.

Ag:Mg is then evaporated at a rate of 0.01 to 1 Å/s at $10^{-7}$ mbar to form a cathode.

A cap layer of HT-3 is formed on the cathode.

The details of the layer stack in the top emission OLED devices are given below. A slash "/" separates individual layers. Layer thicknesses are given in squared brackets [...], mixing ratios in wt% given in round brackets (...):

*Layer stack details used in the OLED device examples of Table 3*

Ag [100nm] / HT-1:D-1 (vol% 92:8) [10nm] / HT-1 [130nm] / HT-2 [5nm] / H09:BD200 (vol% 97:3) [20 nm] / ET-1 [5nm] / ET-3:ET-5 (vol% 20:80) or ET-3:Compound of Formula (II) or Formula (III) (wt% 20:80) or ET-4: Compound of Formula (II) or Formula (III) (vol% 30:70) [30nm] / LiQ [mm] or ET-2:Li (vol% 99:1) [15 nm] / Yb [2nm] / Ag:Mg (vol% 90:10) [13 nm] / HT-3 [75nm]

Table 2: List of compounds used

| | IUPAC name | Reference |
|---|---|---|
| HT-1 | N-([1,1'-biphenyl]-2-yl)-N-(9,9-dimethyl-9H-fluoren-2-yl)-9,9'-spirobi[fluoren]-2-amine [CAS 1364603-07-5] | WO2012034627 |
| HT-2 | N-([1,1'-biphenyl]-4-yl)-9,9-diphenyl-N-(4-(triphenylsilyl) phenyl)-9H-fluoren-2-amine [CAS 1613079-70-11 | WO2014088047 |
| D-1 | 4,4',4"-((1E,1'E,1"E)-cyclopropane-1,2,3-triylidenetris (cyanomethanylylidene))tris(2,3,5,6-tetrafluorobenzonitrile) [CAS 1224447-88-4] | US2008265216 |
| HOST-1 | H09 (Fluorescent-blue host material) | Commercially available from Sun Fine Chemicals, Inc, S. Korea |
| EMITTER-1 | BD200 (Fluorescent-blue emitter material) | Commercially available from Sun Fine Chemicals, Inc, S. Korea |
| ET-1 | 2,4-diphenyl-6-(4',5',6'-triphenyl-[1,1':2',1":3",1‴,:3‴,1⁗-quinquephenyl]-3⁗-yl)-1,3,5-triazine [CAS 2032364-64-8] | WO2016171358 |
| ET-2 | 2,2'-(1,3-Phenylene)bis[9-phenyl-1,10-phenanthroline] [CAS 721969-94-4] | JP2004281390 |
| ET-3 | (3-(4-(4-(4,6-diphenyl-1,3,5-triazin-2-yl)phenyl)naphthalen-1-yl)phenyl)dimethylphosphine oxide | WO20212S0276 |
| | [CAS 2253724-56-8] | |
| ET-4 | 7-(3-(1-(pyridin-2-yl)imidazo[1,5-a]pyridin-3-yl)phenyl) dibenzo[c,h]acridine [CAS 2646631-34-5] | WO2021105518 |
| ET-5 | 2-([1,1'-biphenyl]-3-yl)-4-phenyl-6-(3-(3,5,6-triphenylpyrazin-2-yl)phenyl)-1,3,5-triazine [CAS 2437303-45-0] | WO19238858 |
| LiQ | 8-Hydroxyquinolinolato-lithium [CAS 850918-68-2] | WO2013079217 |
| HT-3 | N-([1,1'-biphenyl]-4-yl)-9,9-dimethyl-N-(4-(9-phenyl-9H-carbazol-3-y])phenyl)-9H-fluoren-2-amine CAS 1242056-42-3 | US2016322581 |

Table 3. Performance data of an organic electroluminescent device comprising in the electron transport layer a mixture of a compound of Formula (I) with a compound of Formula (II) and / or compound of Formula (III).

| OLED device example | Compound Formula (I) | Compound Formula (II) or Formula (III) or coparative compound ET-5 | Vol% Mix ratio Compound Formula (I) : Formula (II) or (III) or ET-5 | EIL1 | EIL 2 | Relative Operating voltage at 10 mA/cm2 (% vs Comparative) | Relative cd/A efficiency at 10 mA/cm2 (% vs Comparative) |
|---|---|---|---|---|---|---|---|
| Comparative Example-1 | ET-3 | ET-5 | 20:80 | LiQ | Yb | 100 | 100 |
| OLED-1 | ET-3 | E-1 | 20:80 | LiQ | Yb | **100** | **103** |

| OLED device example | Compound Formula (I) | Compound Formula (II) or Formula (III) or coparative compound ET-5 | Vol% Mix ratio Compound Formula (I) : Formula (II) or (III) or ET-5 | EIL1 | EIL 2 | Relative Operating voltage at 10 mA/cm2 (% vs Comparative) | Relative cd/A efficiency at 10 mA/cm2 (% vs Comparative) |
|---|---|---|---|---|---|---|---|
| OLED-2 | ET-3 | E-2 | 20:80 | LiQ | Yb | **99** | **105** |
| OLED-3 | ET-3 | E-5 | 20:80 | LiQ | Yb | **99** | **103** |
| OLED-4 | ET-3 | E-6 | 20:80 | LiQ | Yb | **99** | **104** |
| OLED-5 | ET-3 | E-7 | 20:80 | LiQ | Yb | **100** | **105** |
| Comparative example-2 | ET-3 | ET-5 | 20:80 | ET-2:Li | Yb | 100 | 100 |
| OLED-6 | ET-3 | E-1 | 20:80 | ET-2:Li | Yb | **99** | **104** |
| OLED-7 | ET-3 | E-2 | 20:80 | ET-2:Li | Yb | **100** | **106** |
| OLED-8 | ET-3 | E-5 | 20:80 | ET-2:Li | Yb | **98** | **104** |
| OLED-9 | ET-3 | E-6 | 20:80 | ET-2:Li | Yb | **99** | **104** |
| OLED-10 | ET-3 | E-7 | 20:80 | ET-2:Li | Yb | **100** | **105** |
| OLED-11 | ET-3 | E-8 | 20:80 | ET-2:Li | Yb | **102** | **107** |
| OLED-12 | ET-4 | E-2 | 30:70 | ET-2:Li | Yb | **97** | **103** |
| OLED-13 | ET-4 | E-4 | 30:70 | ET-2:Li | Yb | **99** | **101** |

Examples OLED-1 to OLED-13 show that the cd/A efficiency is increased at comparable or voltage if Compound Formula (I) : Formula (II) or (III) is used instead of $ET\text{-}_5$ is used.

The features disclosed in the foregoing description and in the dependent claims may, both separately and in any combination thereof, be material for realizing the aspects of the disclosure made in the independent claims, in diverse forms thereof.

## Claims

1. Organic light emitting diode, comprising an anode, a cathode, an emission layer, and an electron transport layer, wherein

- the electron transport layer is arranged between the emission layer and the cathode;

- the electron transport layer is free of an electrical dopant;
- the electron transport layer comprises a compound of formula (I)

$$(Ar^2)_m\text{-}(Z_k\text{-}G)_n \qquad (I);$$

wherein in formula (I)
- m and n are independently 1 or 2;
- k is independently 0, 1 or 2;
- $Ar^2$ is independently selected from the group consisting of $C_2$ to $C_{42}$ heteroaryl and $C_6$ to $C_{60}$ aryl,

  - wherein each $Ar^2$ may be substituted with one or two substituents independently selected from the group consisting of $C_6$ to $C_{12}$ aryl, $C_3$ to $C_{11}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - wherein each $C_6$ to $C_{12}$ aryl substituent on $Ar^2$ and each $C_3$ to $C_{11}$ heteroaryl substituent on $Ar^2$ may be substituted with $C_1$ to $C_4$ alkyl or halogen;

- Z is independently selected from $C_6$ to $C_{30}$ aryl,

  - wherein each Z may be substituted with one or two substituents independently selected from the group consisting of $C_6$ to $C_{12}$ aryl and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - wherein each $C_6$ to $C_{12}$ aryl substituent on Z may be substituted with $C_1$ to $C_4$ alkyl or halogen;

- G is chosen so that the dipole moment of a compound G-phenyl is $\geq 1$ D and $\leq 7$ D;
- the electron transport layer further comprises a compound of formula (II) and/or a compound of formula (III)

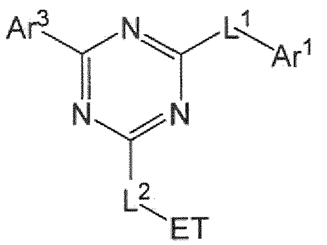
(II);
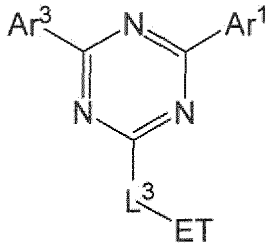
(III)

wherein in formula (II) and in formula (III) respectively
- $Ar^1$ is selected independently from the group consisting of $C_6$ to $C_{19}$ aryl and $C_2$ to $C_{19}$ heteroaryl;

  - wherein $Ar^1$ may be substituted with one or two substituents independently selected from the group consisting of $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- $Ar^3$ is selected independently from the group consisting of $C_6$ to $C_{19}$ aryl;

  - wherein $Ar^3$ may be substituted with one or two substituents independently selected from the group consisting of $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- ET is selected independently from the group consisting of $C_6$ to $C_{60}$ aryl and $C_2$ to $C_{60}$ heteroaryl;

  - wherein ET may be substituted with one or two substituents independently selected from the group consisting of $C_6$ to $C_{20}$ aryl, $C_3$ to $C_{20}$ heteroaryl, and $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;
  - wherein each $C_6$ to $C_{20}$ aryl substituent on ET and each $C_3$ to $C_{20}$ heteroaryl substituent on ET may be substituted with $C_1$ to $C_4$ alkyl or halogen;

- $L^1$ has the formula (IIa)

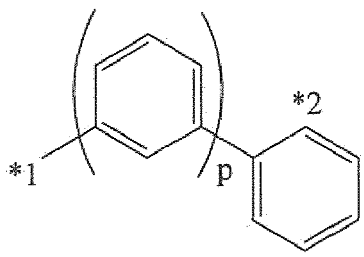

(IIa)

wherein $L^1$ is bonded at *1 to the triazine moiety in formula (II); and $L^1$ is bonded at *2 to $Ar^1$; and p is 0 or 1;
- $L^2$ has the formula (IIb)

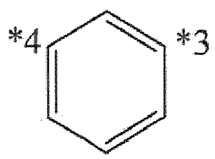

(IIb)

wherein $L^2$ is bonded at *3 to the triazine moiety in formula (II); and $L^2$ is bonded at *4 to ET; and
- $L^3$ has the formula (IIIa.1) or (IIIa.2)

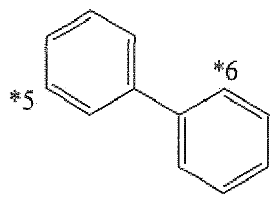

(IIIa.1)

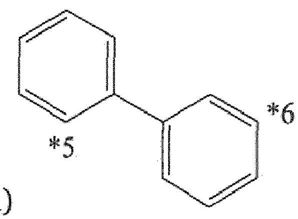

(IIIa.2)

wherein $L^3$ is bonded at *5 to the triazine moiety in formula (III); and $L^3$ is bonded at *6 to ET.

**2.** Organic light emitting diode according to claim 1, wherein $Ar^2$ is independently selected from the group consisting of pyridinyl, triazinyl, 1,2-diazinyl, 1,3-diazinyl, 1,4-diazinyl, quinazolinyl, benzoquinazolinyl, benzimidazolyl, quinolinyl, benzoquinolinyl benzoacridinyl, dibenzoacridinyl, fluoranthenyl, anthracenyl, naphthyl, triphenylenyl, phenathrolinyl, and dinaphthofuranyl which may be substituted or unsubstituted, respectively.

**3.** Organic light emitting diode according to claim 1 or 2, wherein

- G is selected from the group consisting of dialkylphosphinyl, diarylphosphinyl, alkylarylphosphinyl, nitrile, benzonitrile, nicotinonitrile, amide-yl, carbamide-yl and $C_2$ to $C_{17}$ heteroaryl;
- the respective G may include one or more substituents attached to the group, wherein the one or more substituents are selected from the group consisting of phenyl, methyl, ethyl, and pyridyl.

**4.** Organic light emitting diode according to any of the preceding claims, wherein $Ar^1$ is substituted or unsubstituted $C_6$ to $C_{19}$ aryl.

**5.** Organic light emitting diode according to any of the preceding claims, wherein $Ar^3$ is substituted or unsubstituted phenyl.

**6.** Organic light emitting diode according to any of the preceding claims, wherein ET is selected from the group consisting of triphenylpyrazinyl; dibenzoacridinyl; pyridyl; anthracenyl; pyridylanthracenyl phenylanthracenyl and a group of the following formula ET-i

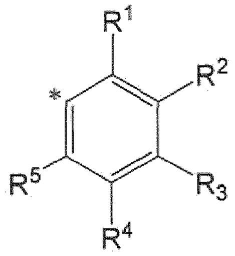

(ET-i)

wherein $R^1$ to $R^5$ are independently H or phenyl, provided that at least two of $R^1$ to $R^5$ are phenyl with the remaining $R^1$ to $R^5$ being H; and ET-i is bonded at * to $L^2$, respectively $L^3$.

**7.** Organic light emitting diode according to any of the preceding claims, wherein the electron transport layer comprises the compound of formula (I) and the compound of formula (II) and "p" in formula (IIa) is o.

**8.** Organic light emitting diode according to any of the preceding claims, wherein the organic light emitting layer further comprises a hole blocking layer and the hole blocking layer is arranged between the emission layer and the electron transport layer.

**9.** Organic light emitting diode according to any of the preceding claims, wherein the organic light emitting diode further comprises an electron injection layer and the electron injection layer is arranged between the cathode and the electron transport layer and the electron injection layer is in direct contact with the electron transport layer.

**10.** Organic light emitting diode according to claim 9, wherein the electron injection layer comprises a first electron injection sub-layer and a second electron injection sub-layer, wherein the first electron injection sub-layer and the second electron injection sub-layer are in direct contact with each other.

**11.** Organic light emitting diode according to claim 10, wherein the first electron injection sub-layer is in direct contact with the electron transport layer and the first electron injection sub-layer comprises a metal salt or a metal complex, preferably comprises LiQ.

**12.** Organic light emitting diode according to claim 10 or 11, wherein the second electron injection sub-layer comprises a metal selected form the group consisting of alkali metals, alkaline earth metals, and rare earth metals, preferably consists of Yb.

**13.** Device comprising the organic light emitting diode according to any of the preceding claims, wherein the device is a display device or a lighting device.

**14.** Compound of the formula (IV) or of the formula (V)

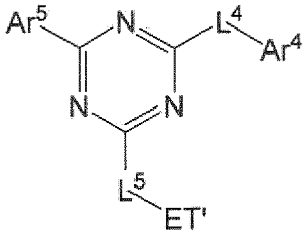
(IV)

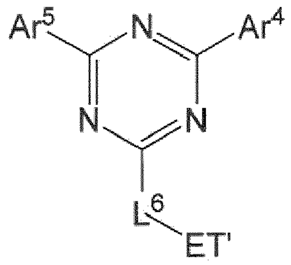
(V)

wherein in formula (IV) and in formula (V) respectively

- $Ar^4$ is selected from the group consisting of $C_6$ to $C_{19}$ aryl and $C_2$ to $C_{19}$ heteroaryl;

  - wherein $Ar^4$ may be substituted with one or two substituents independently selected from the group consisting of $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- $Ar^5$ is selected from the group consisting of $C_6$ to $C_{19}$ aryl;

  - wherein $Ar^5$ may be substituted with one or two substituents independently selected from the group consisting of $C_1$ to $C_6$ alkyl, D, $C_1$ to $C_6$ alkoxy, $C_3$ to $C_6$ branched alkyl, $C_3$ to $C_6$ cyclic alkyl, $C_3$ to $C_6$ branched alkoxy, $C_3$ to $C_6$ cyclic alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy, halogen, CN or $PY(R^{10})_2$, wherein Y is selected from O, S or Se, preferably O, and $R^{10}$ is independently selected from $C_6$ to $C_{12}$ aryl, $C_3$ to $C_{12}$ heteroaryl, $C_1$ to $C_6$ alkyl, $C_1$ to $C_6$ alkoxy, partially or perfluorinated $C_1$ to $C_6$ alkyl, partially or perfluorinated $C_1$ to $C_6$ alkoxy, partially or perdeuterated $C_1$ to $C_6$ alkyl, partially or perdeuterated $C_1$ to $C_6$ alkoxy;

- ET' is selected from the group consisting of pyrazinyl; triphenylpyrazinyl; pyridine-anthracenyl; acridine, benzoacridine, dibenzoacridine, phenylanthracenyl and a compound of the formula ET'-i

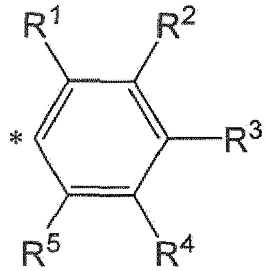
ET'-i

wherein $R^1$ to $R^5$ are independently H or phenyl, provided that at least two, preferably at least three, most preferred four of $R^1$ to $R^5$ are phenyl with the remaining $R^1$ to $R^5$ being H; and ET-i is bonded at * to $L^5$, respectively to $R^6$

;

- $L^4$ has the formula (IVa)

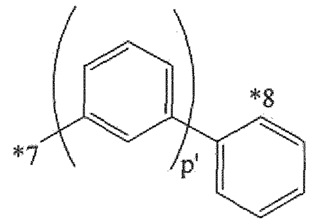

(IVa)

wherein $L^4$ is bonded at *7 to the triazine moiety in formula (IV); and $L^4$ is bonded at *8 to $Ar^4$; and p' is 0 or 1;
- $L^5$ has the formula (IVb)

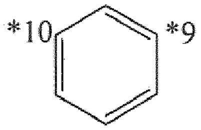

(IVb)

wherein $L^5$ is bonded at *9 to the triazine moiety in formula (IV); and $L^5$ is bonded at *10 to ET'; and
- $L^6$ has the formula (Va.1) or (Va.2)

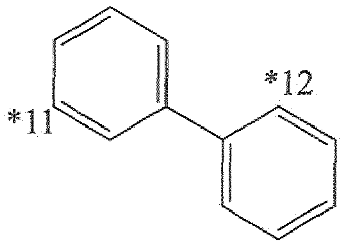

(Va.1)

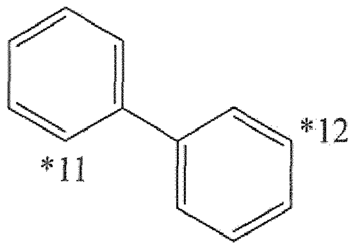

(Va.2)

wherein $L^6$ is bonded at *11 to the triazine moiety in formula (V); and $L^6$ is bonded at *12 to ET'.

**15.** Compound of the following formulas:

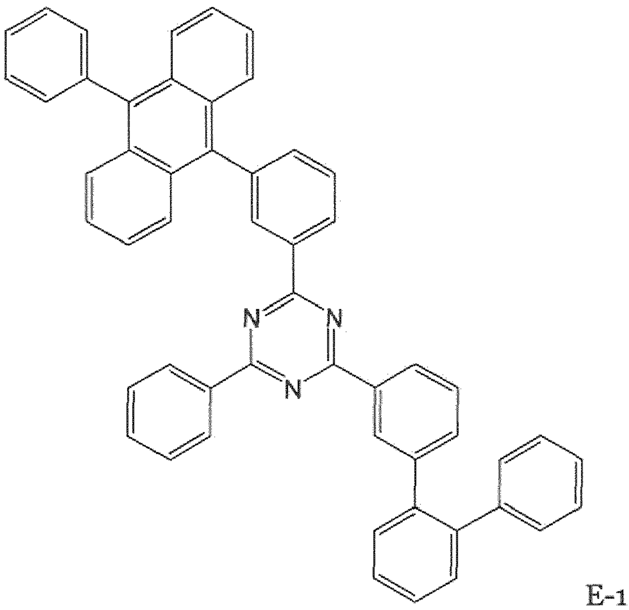

E-1

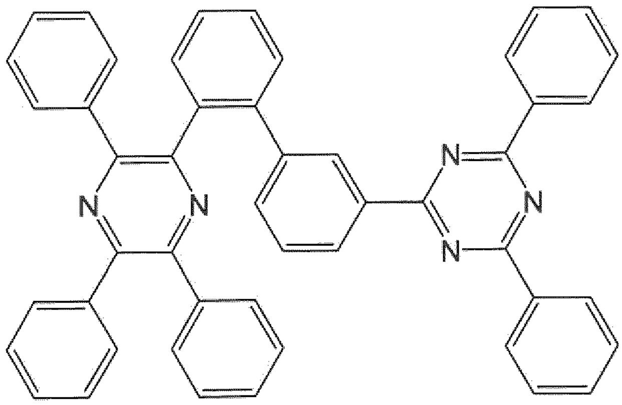

E-2

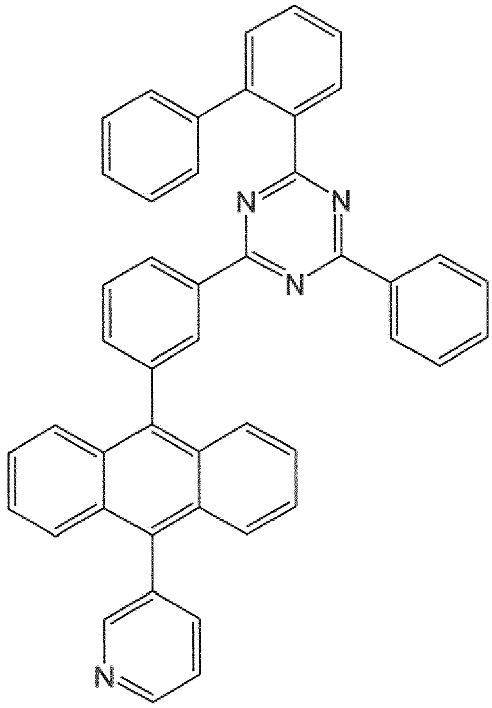

E-4

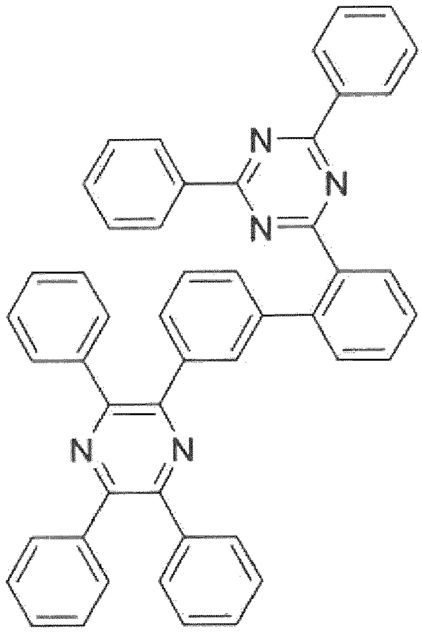

E-7

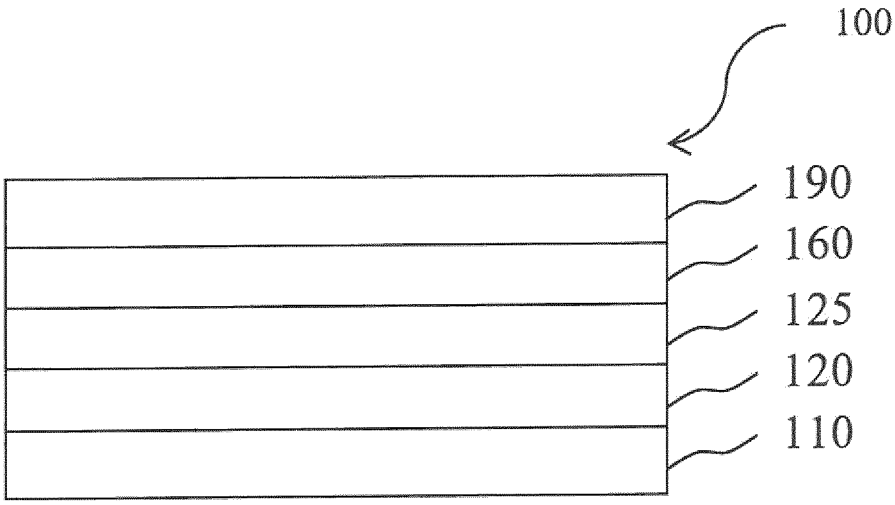


Fig.1

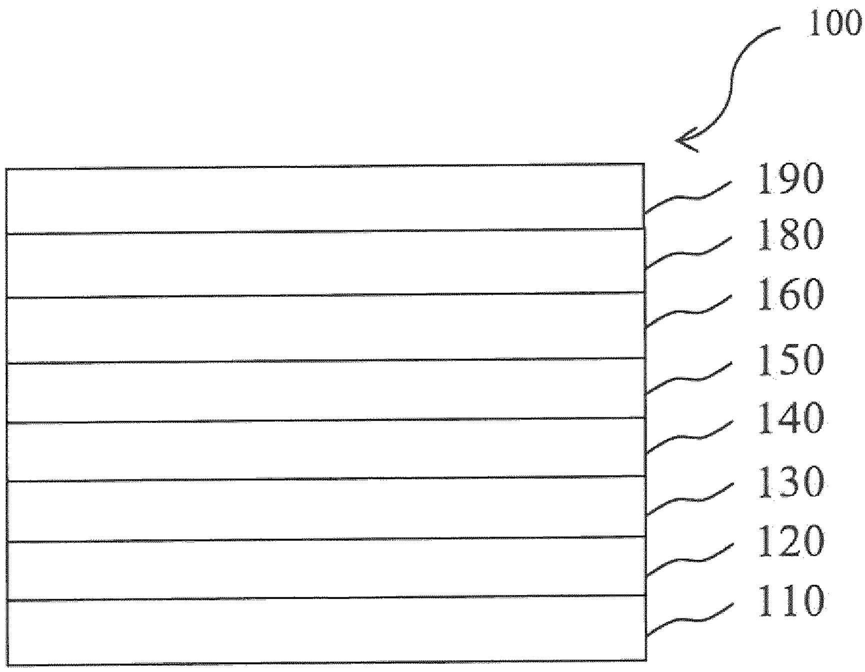


Fig.2

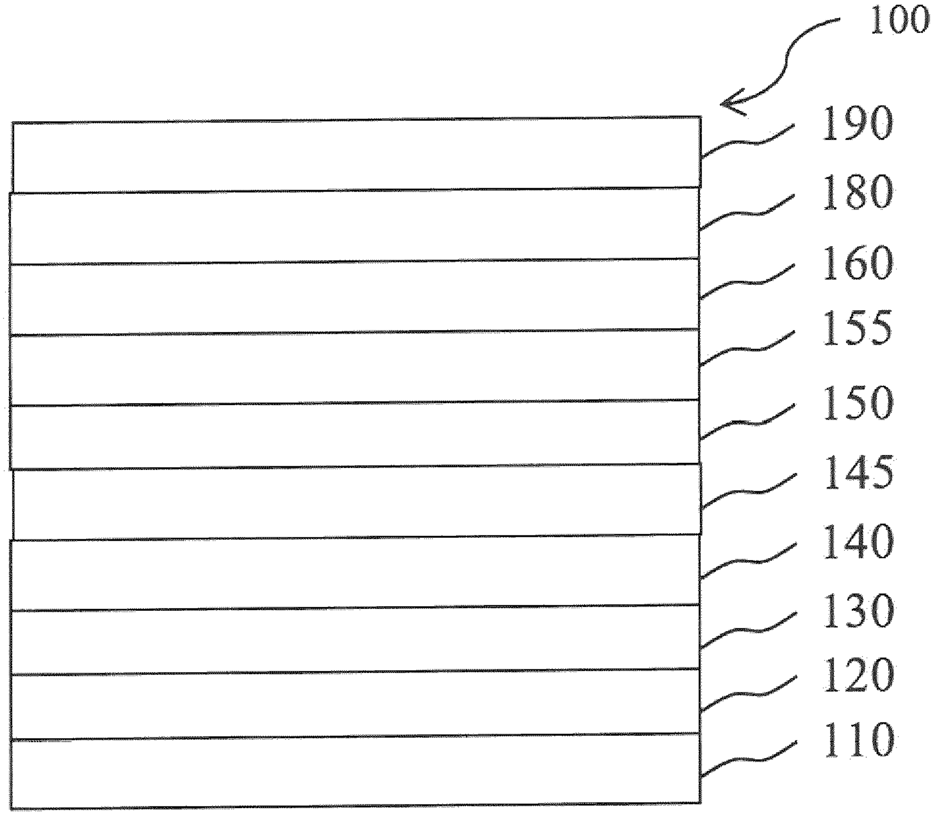


Fig.3

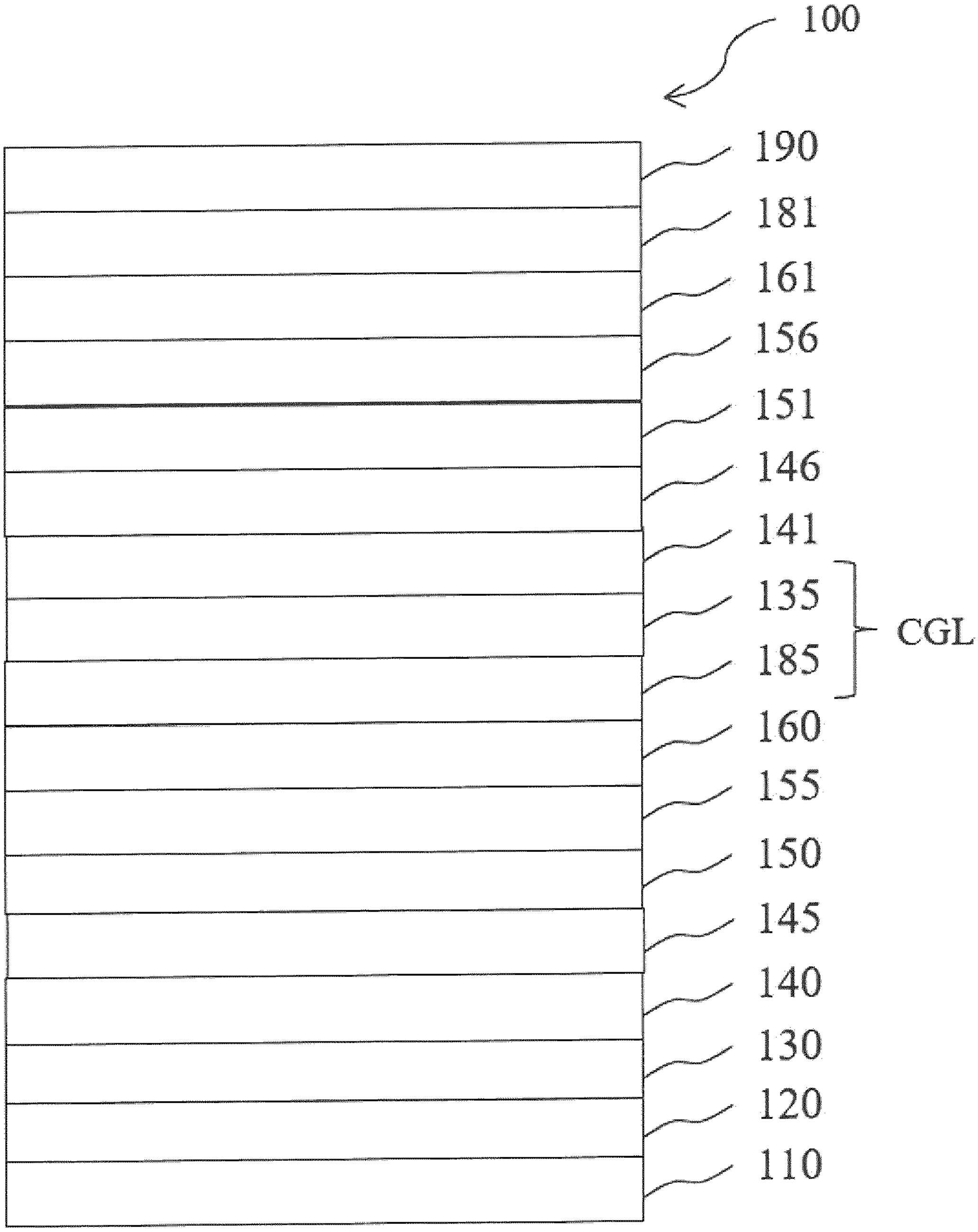
100
190
181
161
156
151
146
141
135
185
CGL
160
155
150
145
140
130
120
110

Fig.4

Europäisches Patentamt
European Patent Office
Office européen des brevets

# EUROPEAN SEARCH REPORT

Application Number
EP 22 21 1740

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 3 208 861 A1 (NOVALED GMBH [DE]) 23 August 2017 (2017-08-23) * paragraphs [0323] - [0327]; example 2; table 7; compounds A18, ETM 1-32 * ----- | 1-13 | INV. H10K85/60 ADD. H10K50/16 |
| Y | US 2017/309830 A1 (KIM HYUNG SUN [KR] ET AL) 26 October 2017 (2017-10-26) * paragraph [0085]; compounds [1],[46] * ----- | 1-13 | |
| X | EP 3 766 875 A1 (NOVALED GMBH [DE]) 20 January 2021 (2021-01-20) * compounds 120,126 * ----- | 14,15 | |
| X | EP 3 412 751 A1 (SAMSUNG SDI CO LTD [KR]) 12 December 2018 (2018-12-12) * compounds 4,8,40 * ----- | 14 | |

TECHNICAL FIELDS SEARCHED (IPC)

H10K

The present search report has been drawn up for all claims

1

| Place of search | Date of completion of the search | Examiner |
|---|---|---|
| The Hague | 31 May 2023 | Fratiloiu, Silvia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 21 1740

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2023

| Patent document cited in search report | Publication date | Patent family member(s) | Publication date |
|---|---|---|---|
| EP 3208861 A1 | 23-08-2017 | CN 108701773 A | 23-10-2018 |
| | | EP 3208861 A1 | 23-08-2017 |
| | | EP 3417495 A1 | 26-12-2018 |
| | | KR 20180110681 A | 10-10-2018 |
| | | US 2021210708 A1 | 08-07-2021 |
| | | US 2021376259 A1 | 02-12-2021 |
| | | WO 2017140780 A1 | 24-08-2017 |
| US 2017309830 A1 | 26-10-2017 | CN 107001292 A | 01-08-2017 |
| | | EP 3287505 A1 | 28-02-2018 |
| | | JP 6714002 B2 | 24-06-2020 |
| | | JP 2018518036 A | 05-07-2018 |
| | | KR 20160126698 A | 02-11-2016 |
| | | TW 201710243 A | 16-03-2017 |
| | | US 2017309830 A1 | 26-10-2017 |
| | | WO 2016171358 A1 | 27-10-2016 |
| EP 3766875 A1 | 20-01-2021 | CN 114222736 A | 22-03-2022 |
| | | EP 3766875 A1 | 20-01-2021 |
| | | EP 3999497 A1 | 25-05-2022 |
| | | KR 20220035181 A | 21-03-2022 |
| | | US 2022165958 A1 | 26-05-2022 |
| | | WO 2021009206 A1 | 21-01-2021 |
| EP 3412751 A1 | 12-12-2018 | CN 108713051 A | 26-10-2018 |
| | | EP 3412751 A1 | 12-12-2018 |
| | | JP 6579725 B2 | 25-09-2019 |
| | | JP 2019511464 A | 25-04-2019 |
| | | KR 20170093023 A | 14-08-2017 |
| | | TW 201728574 A | 16-08-2017 |
| | | US 2018339967 A1 | 29-11-2018 |
| | | WO 2017135510 A1 | 10-08-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005086251 A
- EP 1837926 B1
- WO 2007107306 A
- WO 2007107356 A
- EP 2722908 A1
- WO 2012034627 A
- WO 2014088047 A
- US 2008265216 A
- WO 2016171358 A
- JP 2004281390 B
- WO 20212S0276 A
- WO 2021105518 A
- WO 19238858 A
- WO 2013079217 A
- US 2016322581 A

### Non-patent literature cited in the description

- **YASUHIKO SHIROTA ; HIROSHI KAGEYAMA.** *Chem, Rev.,* 2007, vol. 107, 953-1010
- *DIN EN ISO 11357,* March 2010
- *CHEMICAL ABSTRACTS,* 1364603-07-5
- *CHEMICAL ABSTRACTS,* 1613079-70-11
- *CHEMICAL ABSTRACTS,* 1224447-88-4
- *CHEMICAL ABSTRACTS,* 2032364-64-8
- *CHEMICAL ABSTRACTS,* 721969-94-4
- *CHEMICAL ABSTRACTS,* 2253724-56-8
- *CHEMICAL ABSTRACTS,* 2646631-34-5
- *CHEMICAL ABSTRACTS,* 2437303-45-0
- *CHEMICAL ABSTRACTS,* 1242056-42-3